# NEW EUROPEAN PATENT SPECIFICATION

(11) **EP 0 972 041 B2**
(45) Date of publication and mention of the opposition decision: **18.01.2017**
(45) Mention of the grant of the patent: 11.10.2006
(21) Application number: 98915305.1
(22) Date of filing: 06.04.1998
(51) Int. Cl.: C12N 15/12, C07K 14/47, C12N 15/62, C07K 16/18, A61K 38/16

(54) **NOVEL HUMAN DELTA3 COMPOSITIONS AND THERAPEUTIC AND DIAGNOSTIC USES THEREFOR**
NEUE MENSCHLICHE DELTA3-ZUSAMMENSETZUNG UND DEREN THERAPEUTISCHE UND DIAGNOSTISCHE VERWENDUNGEN
NOUVELLES COMPOSITIONS CONTENANT DES PROTEINES D'ORIGINE HUMAINE DELTA3

(30) Priority: 04.04.1997 US 832633; 11.06.1997 US 872855
(43) Date of publication of application: 19.01.2000
(73) Proprietor: MILLENNIUM PHARMACEUTICALS, INC., Cambridge, MA 02139 (US)
(72) Inventor: MCCARTHY, Sean, A., Boston, MA 02114 (US); GEARING, David, P., Wellesley, MA 02181 (US)
(74) Representative: Goodfellow, Hugh Robin
(86) International application number: PCT/US1998/006775
(87) International publication number: WO 1998/045434

(56) References cited:
- WO-A-97/01571
- WO-A-97/19172
- HILLIER L ET AL: "Homo sapiens cDNA clone 687729 3' (accession number AA235839)" EMBL SEQUENCE DATABASE,6 March 1997, XP002076961 Heidelberg, Germany
- DORNSEIFER P ET AL: "Overexpression of a zebrafish homologue of the Drosophila neurogenic gene Delta perturbs differentiation of primary neurons and somite development" MECHANISMS OF DEVELOPMENT, vol. 63, May 1997, pages 159-171, XP002076962 & CAMPOS-ORTEGA J A : "D. rerio mRNA for deltaD protein" EMBL SEQUENCE DATABASE,12 March 1997, Heidelberg, Germany cited in the application

### SEQUENCE LISTING

(1) GENERAL INFORMATION:
   (i) APPLICANT: McCarthy, Sean A.
      Gearing, David P.
   (ii) TITLE OF INVENTION: NOVEL HUMAN DELTA3 COMPOSITIONS AND THERAPEUTIC USES THEREFOR
   (iii) NUMBER OF SEQUENCES: 23
   (iv) CORRESPONDENCE ADDRESS:
      (A) ADDRESSEE: FOLEY, HOAG & ELIOT LLP
      (B) STREET: One Post Office Square
      (C) CITY: Boston
      (D) STATE: MA
      (E) COUNTRY: USA
      (F) ZIP: 02109-2170
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentln Release #1.0, Version #1.30
   (vi) CURRENT APPLICATION DATA:
      (A) APPLICATION NUMBER: PCT/US
      (B) FILING DATE: 6-April-1998
      (C) CLASSIFICATION:
   (vii) PRIOR APPLICATION DATA:
      (A) APPLICATION NUMBER: US 08/872,855
      (B) FILING DATE: 11-June-1997
   (vii) PRIOR APPLICATION DATA:
      (A) APPLICATION NUMBER: US 08/832,633
      (B) FILING DATE: 4-April-1997
   (viii) ATTORNEY/AGENT INFORMATION:
      (A) NAME: Arnold, Beth E.
      (B) REGISTRATION NUMBER: 35,430
      (C) REFERENCE/DOCKET NUMBER: MAA-003.25
   (ix) TELECOMMUNICATION INFORMATION:
      (A) TELEPHONE: 617-832-1000
      (B) TELEFAX: 617-832-7000
(2) INFORMATION FOR SEQ ID NO:1:
   (i) SEQUENCE CHARACTERISTICS:
      (A) LENGTH: 2800 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 338..2392
   (xi) SEQUENCE DESCRIPTION: SEQ ID NO:1:
   GTCGACCCAC GCGTCCGGCT GCGCGCAGGC CGGGAACACG AGGCCAAGAG CCGCAGCCCC     60
   AGCCGCCTTG GTGCAGCGTA CACCGGCACT AGCCCGCTTG CAGCCCCAGG ATTAGACAGA    120
   AGACGCGTCC TCGGCGCGGT CGCCGCCCAG CCGTAGTCAC CTGGATTACC TACAGCGGCA    180
   GCTGCAGCGG AGCCAGCGAG AAGGCCAAAG GGGAGCAGCG TCCCGAGAGG AGCGCCTCTT    240
   TTCAGGGACC CCGCCGGCTG GCGGACGCGC GGGAAAGCGG CGTCGCGAAC AGAGCCAGAT    300
   TGAGGGCCCG CGGGTGGAGA GAGCGACGCC CGAGGGG ATG GCG GCA GCG TCC CGG   355
                                           Met Ala Ala Ala Ser Arg
                                             1               5
   AGC GCC TCT GGC TGG GCG CTA CTG CTG CTG GTG GCA CTT TGG CAG CAG      403
   Ser Ala Ser Gly Trp Ala Leu Leu Leu Leu Val Ala Leu Trp Gln Gln
               10                  15                  20
   CGC GCG GCC GGC TCC GGC GTC TTC CAG CTG CAG CTG CAG GAG TTC ATC 451
   Arg Ala Ala Gly Ser Gly Val Phe Gln Leu Gln Leu Gln Glu Phe Ile
   25 30 35
   AAC GAG CGC GGC GTA CTG GCC AGT GGG CGG CCT TGC GAG CCC GGC TGC 499
   Asn Glu Arg Gly Val Leu Ala Ser Gly Arg Pro Cys Glu Pro Gly Cys
   40 45 50
   CGG ACT TTC TTC CGC GTC TGC CTT AAG CAC TTC CAG GCG GTC GTC TCG 547
   Arg Thr Phe Phe Arg Val Cys Leu Lys His Phe Gln Ala Val Val Ser
   55 60 65 70
   CCC GGA CCC TGC ACC TTC GGG ACC GTC TCC ACG CCG GTA TTG GGC ACC 595
   Pro Gly Pro Cys Thr Phe Gly Thr Val Ser Thr Pro Val Leu Gly Thr
   75 80 85
   AAC TCC TTC GCT GTC CGG GAC GAC AGT AGC GGC GGG GGG CGC AAC CCT 643
   Asn Ser Phe Ala Val Arg Asp Asp Ser Ser Gly Gly Gly Arg Asn Pro
   90 95 100
   CTC CAA CTG CCC TTC AAT TTC ACC TGG CCG GGT ACC TTC TCG CTC ATC 691
   Leu Gln Leu Pro Phe Asn Phe Thr Trp Pro Gly Thr Phe Ser Leu Ile
   105 110 115
   ATC GAA GCT TGG CAC GCG CCA GGA GAC GAC CTG CGG CCA GAG GCC TTG 739
   Ile Glu Ala Trp His Ala Pro Gly Asp Asp Leu Arg Pro Glu Ala Leu
   120 125 130
   CCA CCA GAT GCA CTC ATC AGC AAG ATC GCC ATC CAG GGC TCC CTA GCT 787
   Pro Pro Asp Ala Leu Ile Ser Lys Ile Ala Ile Gln Gly Ser Leu Ala
   135 140 145 150
   GTG GGT CAG AAC TGG TTA TTG GAT GAG CAA ACC AGC ACC CTC ACA AGG 835
   Val Gly Gln Asn Trp Leu Leu Asp Glu Gln Thr Ser Thr Leu Thr Arg
   155 160 165
   CTG CGC TAC TCT TAC CGG GTC ATC TGC AGT GAC AAC TAC TAT GGA GAC 883
   Leu Arg Tyr Ser Tyr Arg Val Ile Cys Ser Asp Asn Tyr Tyr Gly Asp
   170 175 180
   AAC TGC TCC CGC CTG TGC AAG AAG CGC AAT GAC CAC TTC GGC CAC TAT 931
   Asn Cys Ser Arg Leu Cys Lys Lys Arg Asn Asp His Phe Gly His Tyr
   185 190 195
   GTG TGC CAG CCA GAT GGC AAC TTG TCC TGC CTG CCC GGT TGG ACT GGG 979
   Val Cys Gln Pro Asp Gly Asn Leu Ser Cys Leu Pro Gly Trp Thr Gly
   200 205 210
   GAA TAT TGC CAA CAG CCT ATC TGT CTT TCG GGC TGT CAT GAA CAG AAT 1027
   Glu Tyr Cys Gln Gln Pro Ile Cys Leu Ser Gly Cys His Glu Gln Asn
   215 220 225 230
   GGC TAC TGC AGC AAG CCA GCA GAG TGC CTC TGC CGC CCA GGC TGG CAG 1075
   Gly Tyr Cys Ser Lys Pro Ala Glu Cys Leu Cys Arg Pro Gly Trp Gln
   235 240 245
   GGC CGG CTG TGT AAC GAA TGC ATC CCC CAC AAT GGC TGT CGC CAC GGC 1123
   Gly Arg Leu Cys Asn Glu Cys Ile Pro His Asn Gly Cys Arg His Gly
   250 255 260
   ACC TGC AGC ACT CCC TGG CAA TGT ACT TGT GAT GAG GGC TGG GGA GGC 1171
   Thr Cys Ser Thr Pro Trp Gln Cys Thr Cys Asp Glu Gly Trp Gly Gly
   265 270 275
   CTG TTT TGT GAC CAA GAT CTC AAC TAC TGC ACC CAC CAC TCC CCA TGC 1219
   Leu Phe Cys Asp Gln Asp Leu Asn Tyr Cys Thr His His Ser Pro Cys
   280 285 290
   AAG AAT GGG GCA ACG TGC TCC AAC AGT GGG CAG CGA AGC TAC ACC TGC 1267
   Lys Asn Gly Ala Thr Cys Ser Asn Ser Gly Gln Arg Ser Tyr Thr Cys
   295 300 305 310
   ACC TGT CGC CCA GGC TAC ACT GGT GTG GAC TGT GAG CTG GAG CTC AGC 1315
   Thr Cys Arg Pro Gly Tyr Thr Gly Val Asp Cys Glu Leu Glu Leu Ser
   315 320 325
   GAG TGT GAC AGC AAC CCC TGT CGC AAT GGA GGC AGC TGT AAG GAC CAG 1363
   Glu Cys Asp Ser Asn Pro Cys Arg Asn Gly Gly Ser Cys Lys Asp Gln
   330 335 340
   GAG GAT GGC TAC CAC TGC CTG TGT CCT CCG GGC TAC TAT GGC CTG CAT 1411
   Glu Asp Gly Tyr His Cys Leu Cys Pro Pro Gly Tyr Tyr Gly Leu His
   345 350 355
   TGT GAA CAC AGC ACC TTG AGC TGC GCC GAC TCC CCC TGC TTC AAT GGG     1459
   Cys Glu His Ser Thr Leu Ser Cys Ala Asp Ser Pro Cys Phe Asn Gly
       360                 365                 370
   GGC TCC TGC CGG GAG CGC AAC CAG GGG GCC AAC TAT GCT TGT GAA TGT     1507
   Gly Ser Cys Arg Glu Arg Asn Gln Gly Ala Asn Tyr Ala Cys Glu Cys
   375                 380                 385                 390
   CCC CCC AAC TTC ACC GGC TCC AAC TGC GAG AAG AAA GTG GAC AGG TGC     1555
   Pro Pro Asn Phe Thr Gly Ser Asn Cys Glu Lys Lys Val Asp Arg Cys
                   395                 400                 405
   ACC AGC AAC CCC TGT GCC AAC GGG GGA CAG TGC CTG AAC CGA GGT CCA     1603
   Thr Ser Asn Pro Cys Ala Asn Gly Gly Gln Cys Leu Asn Arg Gly Pro
               410                 415                 420
   AGC CGC ATG TGC CGC TGC CGT CCT GGA TTC ACG GGC ACC TAC TGT GAA     1651
   Ser Arg Met Cys Arg Cys Arg Pro Gly Phe Thr Gly Thr Tyr Cys Glu
           425                 430                 435
   CTC CAC GTC AGC GAC TGT GCC CGT AAC CCT TGC GCC CAC GGT GGC ACT     1699
   Leu His Val Ser Asp Cys Ala Arg Asn Pro Cys Ala His Gly Gly Thr
       440                 445                 450
   TGC CAT GAC CTG GAG AAT GGG CTC ATG TGC ACC TGC CCT GCC GGC TTC     1747
   Cys His Asp Leu Glu Asn Gly Leu Met Cys Thr Cys Pro Ala Gly Phe
   455                 460                 465                 470
   TCT GGC CGA CGC TGT GAG GTG CGG ACA TCC ATC GAT GCC TGT GCC TCG     1795
   Ser Gly Arg Arg Cys Glu Val Arg Thr Ser Ile Asp Ala Cys Ala Ser
                   475                 480                 485
   AGT CCC TGC TTC AAC AGG GCC ACC TGC TAC ACC GAC CTC TCC ACA GAC     1843
   Ser Pro Cys Phe Asn Arg Ala Thr Cys Tyr Thr Asp Leu Ser Thr Asp
               490                 495                 500
   ACC TTT GTG TGC AAC TGC CCT TAT GGC TTT GTG GGC AGC CGC TGC GAG     1891
   Thr Phe Val Cys Asn Cys Pro Tyr Gly Phe Val Gly Ser Arg Cys Glu
           505                 510                 515
   TTC CCC GTG GGC TTG CCG CCC AGC TTC CCC TGG GTG GCC GTC TCG CTG     1939
   Phe Pro Val Gly Leu Pro Pro Ser Phe Pro Trp Val Ala Val Ser Leu
   520 525 530
   GGT GTG GGG CTG GCA GTG CTG CTG GTA CTG CTG GGC ATG GTG GCA GTG 1987
   Gly Val Gly Leu Ala Val Leu Leu Val Leu Leu Gly Met Val Ala Val
   535 540 545 550
   GCT GTG CGG CAG CTG CGG CTT CGA CGG CCG GAC GAC GGC AGC AGG GAA 2035
   Ala Val Arg Gln Leu Arg Leu Arg Arg Pro Asp Asp Gly Ser Arg Glu
   555 560 565
   GCC ATG AAC AAC TTG TCG GAC TTC CAG AAG GAC AAC CTG ATT CCT GCC 2083
   Ala Met Asn Asn Leu Ser Asp Phe Gln Lys Asp Asn Leu Ile Pro Ala
   570 575 580
   GCC CAG CTT AAA AAC ACA AAC CAG AAG AAG GAG CTG GAA GTG GAC TGT 2131
   Ala Gln Leu Lys Asn Thr Asn Gln Lys Lys Glu Leu Glu Val Asp Cys
   585 590 595
   GGC CTG GAC AAG TCC AAC TGT GGC AAA CAG CAA AAC CAC ACA TTG GAC 2179
   Gly Leu Asp Lys Ser Asn Cys Gly Lys Gln Gln Asn His Thr Leu Asp
   600 605 610
   TAT AAT CTG GCC CCA GGG CCC CTG GGG CGG GGG ACC ATG CCA GGA AAG 2227
   Tyr Asn Leu Ala Pro Gly Pro Leu Gly Arg Gly Thr Met Pro Gly Lys
   615 620 625 630
   TTT CCC CAC AGT GAC AAG AGC TTA GGA GAG AAG GCG CCA CTG CGG TTA 2275
   Phe Pro His Ser Asp Lys Ser Leu Gly Glu Lys Ala Pro Leu Arg Leu
   635 640 645
   CAC AGT GAA AAG CCA GAG TGT CGG ATA TCA GCG ATG TGC TCC CCC AGG 2323
   His Ser Glu Lys Pro Glu Cys Arg Ile Ser Ala Met Cys Ser Pro Arg
   650 655 660
   GAC TCC ATG TAC CAG TCT GTG TGT TTG ATA TCA GAG GAG AGG AAT GAA 2371
   Asp Ser Met Tyr Gln Ser Val Cys Leu Ile Ser Glu Glu Arg Asn Glu
   665 670 675
   TGT GTC ATT GCC ACG GAG GTA TAAGGCAGGA GCCTACCTGG ACATCCCTGC 2422
   Cys Val Ile Ala Thr Glu Val
   680 685
   TCAGCCCCGC GGCTGGACCT TCCTTCTGCA TTGTTTACAT TGCATCCTGG ATGGGACGTT 2482
   TTTCATATGC AACGTGCTGC TCTCAGGAGG AGGAGGGAAT GGCAGGAACC GGACAGACTG 2542
   TGAACTTGCC AAGAGATGCA ATACCCTTCC ACACCTTTGG GTGTCTGTCT GGCATCAGAT 2602
   TGGCAGCTGC ACCAACCAGA GGAACAGAAG AGAAGAGAGT GGCAGTAGCC CCATGGGGCC 2662
   CGGAGCTGCT GTGGCCTCCA CTGGCATCCG TGTTTCCAAA AGTGCCTTTG GCCCAGCCAA 2722
   GGGTGCCAGG CCTAACTGGG GCACTCAGGG CAGTGTGTTG GAAATTCCAC TGAGGGGGAA 2782
   ATCAGGTGCT GCGGCCGC 2800
(2) INFORMATION FOR SEQ ID NO:2:
   (i) SEQUENCE CHARACTERISTICS:
      (A) LENGTH: 685 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Ala Ala Ala Ser Arg Ser Ala Ser Gly Trp Ala Leu Leu Leu Leu
   1 5 10 15
   Val Ala Leu Trp Gln Gln Arg Ala Ala Gly Ser Gly Val Phe Gln Leu
   20 25 30
   Gln Leu Gln Glu Phe Ile Asn Glu Arg Gly Val Leu Ala Ser Gly Arg
   35 40 45
   Pro Cys Glu Pro Gly Cys Arg Thr Phe Phe Arg Val Cys Leu Lys His
   50 55 60
   Phe Gln Ala Val Val Ser Pro Gly Pro Cys Thr Phe Gly Thr Val Ser
   65 70 75 80
   Thr Pro Val Leu Gly Thr Asn Ser Phe Ala Val Arg Asp Asp Ser Ser
   85 90 95
   Gly Gly Gly Arg Asn Pro Leu Gln Leu Pro Phe Asn Phe Thr Trp Pro
   100 105 110
   Gly Thr Phe Ser Leu Ile Ile Glu Ala Trp His Ala Pro Gly Asp Asp
   115 120 125
   Leu Arg Pro Glu Ala Leu Pro Pro Asp Ala Leu Ile Ser Lys Ile Ala
   130 135 140
   Ile Gln Gly Ser Leu Ala Val Gly Gln Asn Trp Leu Leu Asp Glu Gln
   145 150 155 160
   Thr Ser Thr Leu Thr Arg Leu Arg Tyr Ser Tyr Arg Val Ile Cys Ser
   165 170 175
   Asp Asn Tyr Tyr Gly Asp Asn Cys Ser Arg Leu Cys Lys Lys Arg Asn
   180 185 190
   Asp His Phe Gly His Tyr Val Cys Gln Pro Asp Gly Asn Leu Ser Cys
   195 200 205
   Leu Pro Gly Trp Thr Gly Glu Tyr Cys Gln Gln Pro Ile Cys Leu Ser
   210 215 220
   Gly Cys His Glu Gln Asn Gly Tyr Cys Ser Lys Pro Ala Glu Cys Leu
   225 230 235 240
   Cys Arg Pro Gly Trp Gln Gly Arg Leu Cys Asn Glu Cys Ile Pro His
   245 250 255
   Asn Gly Cys Arg His Gly Thr Cys Ser Thr Pro Trp Gln Cys Thr Cys
   260 265 270
   Asp Glu Gly Trp Gly Gly Leu Phe Cys Asp Gln Asp Leu Asn Tyr Cys
   275 280 285
   Thr His His Ser Pro Cys Lys Asn Gly Ala Thr Cys Ser Asn Ser Gly
   290 295 300
   Gln Arg Ser Tyr Thr Cys Thr Cys Arg Pro Gly Tyr Thr Gly Val Asp
   305 310 315 320
   Cys Glu Leu Glu Leu Ser Glu Cys Asp Ser Asn Pro Cys Arg Asn Gly
   325 330 335
   Gly Ser Cys Lys Asp Gln Glu Asp Gly Tyr His Cys Leu Cys Pro Pro
   340 345 350
   Gly Tyr Tyr Gly Leu His Cys Glu His Ser Thr Leu Ser Cys Ala Asp
   355 360 365
   Ser Pro Cys Phe Asn Gly Gly Ser Cys Arg Glu Arg Asn Gln Gly Ala
   370 375 380
   Asn Tyr Ala Cys Glu Cys Pro Pro Asn Phe Thr Gly Ser Asn Cys Glu
   385 390 395 400
   Lys Lys Val Asp Arg Cys Thr Ser Asn Pro Cys Ala Asn Gly Gly Gln
   405 410 415
   Cys Leu Asn Arg Gly Pro Ser Arg Met Cys Arg Cys Arg Pro Gly Phe
   420 425 430
   Thr Gly Thr Tyr Cys Glu Leu His Val Ser Asp Cys Ala Arg Asn Pro
   435 440 445
   Cys Ala His Gly Gly Thr Cys His Asp Leu Glu Asn Gly Leu Met Cys
   450 455 460
   Thr Cys Pro Ala Gly Phe Ser Gly Arg Arg Cys Glu Val Arg Thr Ser
   465 470 475 480
   Ile Asp Ala Cys Ala Ser Ser Pro Cys Phe Asn Arg Ala Thr Cys Tyr
   485 490 495
   Thr Asp Leu Ser Thr Asp Thr Phe Val Cys Asn Cys Pro Tyr Gly Phe
   500 505 510
   Val Gly Ser Arg Cys Glu Phe Pro Val Gly Leu Pro Pro Ser Phe Pro
   515 520 525
   Trp Val Ala Val Ser Leu Gly Val Gly Leu Ala Val Leu Leu Val Leu
   530 535 540
   Leu Gly Met Val Ala Val Ala Val Arg Gln Leu Arg Leu Arg Arg Pro
   545 550 555 560
   Asp Asp Gly Ser Arg Glu Ala Met Asn Asn Leu Ser Asp Phe Gln Lys
   565 570 575
   Asp Asn Leu Ile Pro Ala Ala Gln Leu Lys Asn Thr Asn Gln Lys Lys
   580 585 590
   Glu Leu Glu Val Asp Cys Gly Leu Asp Lys Ser Asn Cys Gly Lys Gln
   595 600 605
   Gln Asn His Thr Leu Asp Tyr Asn Leu Ala Pro Gly Pro Leu Gly Arg
   610 615 620
   Gly Thr Met Pro Gly Lys Phe Pro His Ser Asp Lys Ser Leu Gly Glu
   625 630 635 640
   Lys Ala Pro Leu Arg Leu His Ser Glu Lys Pro Glu Cys Arg Ile Ser
   645 650 655
   Ala Met Cys Ser Pro Arg Asp Ser Met Tyr Gln Ser Val Cys Leu Ile
   660 665 670
   Ser Glu Glu Arg Asn Glu Cys Val Ile Ala Thr Glu Val
   675 680 685
(2) INFORMATION FOR SEQ ID NO:3:
   (i) SEQUENCE CHARACTERISTICS:
      (A) LENGTH: 2055 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:3:
   ATGGCGGCAG CGTCCCGGAG CGCCTCTGGC TGGGCGCTAC TGCTGCTGGT GGCACTTTGG 60
   CAGCAGCGCG CGGCCGGCTC CGGCGTCTTC CAGCTGCAGC TGCAGGAGTT CATCAACGAG 120
   CGCGGCGTAC TGGCCAGTGG GCGGCCTTGC GAGCCCGGCT GCCGGACTTT CTTCCGCGTC 180
   TGCCTTAAGC ACTTCCAGGC GGTCGTCTCG CCCGGACCCT GCACCTTCGG GACCGTCTCC 240
   ACGCCGGTAT TGGGCACCAA CTCCTTCGCT GTCCGGGACG ACAGTAGCGG CGGGGGGCGC 300
   AACCCTCTCC AACTGCCCTT CAATTTCACC TGGCCGGGTA CCTTCTCGCT CATCATCGAA 360
   GCTTGGCACG CGCCAGGAGA CGACCTGCGG CCAGAGGCCT TGCCACCAGA TGCACTCATC 420
   AGCAAGATCG CCATCCAGGG CTCCCTAGCT GTGGGTCAGA ACTGGTTATT GGATGAGCAA 480
   ACCAGCACCC TCACAAGGCT GCGCTACTCT TACCGGGTCA TCTGCAGTGA CAACTACTAT 540
   GGAGACAACT GCTCCCGCCT GTGCAAGAAG CGCAATGACC ACTTCGGCCA CTATGTGTGC 600
   CAGCCAGATG GCAACTTGTC CTGCCTGCCC GGTTGGACTG GGGAATATTG CCAACAGCCT 660
   ATCTGTCTTT CGGGCTGTCA TGAACAGAAT GGCTACTGCA GCAAGCCAGC AGAGTGCCTC 720
   TGCCGCCCAG GCTGGCAGGG CCGGCTGTGT AACGAATGCA TCCCCCACAA TGGCTGTCGC 780
   CACGGCACCT GCAGCACTCC CTGGCAATGT ACTTGTGATG AGGGCTGGGG AGGCCTGTTT 840
   TGTGACCAAG ATCTCAACTA CTGCACCCAC CACTCCCCAT GCAAGAATGG GGCAACGTGC 900
   TCCAACAGTG GGCAGCGAAG CTACACCTGC ACCTGTCGCC CAGGCTACAC TGGTGTGGAC 960
   TGTGAGCTGG AGCTCAGCGA GTGTGACAGC AACCCCTGTC GCAATGGAGG CAGCTGTAAG 1020
   GACCAGGAGG ATGGCTACCA CTGCCTGTGT CCTCCGGGCT ACTATGGCCT GCATTGTGAA 1080
   CACAGCACCT TGAGCTGCGC CGACTCCCCC TGCTTCAATG GGGGCTCCTG CCGGGAGCGC 1140
   AACCAGGGGG CCAACTATGC TTGTGAATGT CCCCCCAACT TCACCGGCTC CAACTGCGAG 1200
   AAGAAAGTGG ACAGGTGCAC CAGCAACCCC TGTGCCAACG GGGGACAGTG CCTGAACCGA 1260
   GGTCCAAGCC GCATGTGCCG CTGCCGTCCT GGATTCACGG GCACCTACTG TGAACTCCAC 1320
   GTCAGCGACT GTGCCCGTAA CCCTTGCGCC CACGGTGGCA CTTGCCATGA CCTGGAGAAT 1380
   GGGCTCATGT GCACCTGCCC TGCCGGCTTC TCTGGCCGAC GCTGTGAGGT GCGGACATCC 1440
   ATCGATGCCT GTGCCTCGAG TCCCTGCTTC AACAGGGCCA CCTGCTACAC CGACCTCTCC 1500
   ACAGACACCT TTGTGTGCAA CTGCCCTTAT GGCTTTGTGG GCAGCCGCTG CGAGTTCCCC 1560
   GTGGGCTTGC CGCCCAGCTT CCCCTGGGTG GCCGTCTCGC TGGGTGTGGG GCTGGCAGTG 1620
   CTGCTGGTAC TGCTGGGCAT GGTGGCAGTG GCTGTGCGGC AGCTGCGGCT TCGACGGCCG 1680
   GACGACGGCA GCAGGGAAGC CATGAACAAC TTGTCGGACT TCCAGAAGGA CAACCTGATT 1740
   CCTGCCGCCC AGCTTAAAAA CACAAACCAG AAGAAGGAGC TGGAAGTGGA CTGTGGCCTG 1800
   GACAAGTCCA ACTGTGGCAA ACAGCAAAAC CACACATTGG ACTATAATCT GGCCCCAGGG 1860
   CCCCTGGGGC GGGGGACCAT GCCAGGAAAG TTTCCCCACA GTGACAAGAG CTTAGGAGAG 1920
   AAGGCGCCAC TGCGGTTACA CAGTGAAAAG CCAGAGTGTC GGATATCAGC GATGTGCTCC 1980
   CCCAGGGACT CCATGTACCA GTCTGTGTGT TTGATATCAG AGGAGAGGAA TGAATGTGTC 2040
   ATTGCCACGG AGGTA 2055
(2) INFORMATION FOR SEQ ID NO:4:
   (i) SEQUENCE CHARACTERISTICS:
      (A) LENGTH: 720 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO:4:
   Met Gly Arg Arg Ser Ala Leu Ala Leu Ala Val Val Ser Ala Leu Leu
   1 5 10 15
   Cys Gln Val Trp Ser Ser Gly Val Phe Glu Leu Lys Leu Gln Glu Phe
   20 25 30
   Val Asn Lys Lys Gly Leu Leu Gly Asn Arg Asn Cys Cys Arg Gly Gly
   35 40 45
   Ser Gly Pro Pro Cys Ala Cys Arg Thr Phe Phe Arg Val Cys Leu Lys
   50 55 60
   His Gln Ala Ser Val Ser Pro Glu Pro Pro Cys Thr Tyr Gly Ser Ala
   65 70 75 80
   Val Thr Pro Val Leu Gly Val Asp Ser Phe Ser Leu Pro Asp Gly Ala
   85 90 95
   Gly Ile Asp Pro Ala Phe Ser Asn Pro Ile Arg Phe Pro Phe Gly Phe
   100 105 110
   Thr Trp Pro Gly Thr Phe Ser Leu Ile Ile Glu Ala Leu His Thr Asp
   115 120 125
   Ser Pro Asp Asp Leu Ala Thr Glu Asn Pro Glu Arg Leu Ile Ser Arg
   130 135 140
   Leu Thr Thr Gln Arg His Thr Val Gly Glu Glu Trp Ser Gln Asp Leu
   145 150 155 160
   His Ser Ser Gly Arg Thr Asp Leu Arg Tyr Ser Tyr Arg Phe Val Cys
   165 170 175
   Asp Glu His Tyr Tyr Gly Glu Gly Cys Ser Val Phe Cys Arg Pro Arg
   180 185 190
   Asp Asp Ala Phe Gly His Phe Thr Cys Gly Asp Arg Gly Glu Lys Met
   195 200 205
   Cys Asp Pro Gly Trp Lys Gly Gln Tyr Cys Thr Asp Pro Ile Cys Leu
   210 215 220
   Pro Gly Cys Asp Asp Gln His Gly Tyr Cys Asp Lys Pro Gly Glu Cys
   225 230 235 240
   Lys Cys Arg Val Gly Trp Gln Gly Arg Tyr Cys Asp Glu Cys Ile Arg
   245 250 255
   Tyr Pro Gly Cys Leu His Gly Thr Cys Gln Gln Pro Trp Gln Cys Asn
   260 265 270
   Cys Gln Glu Gly Trp Gly Gly Leu Phe Cys Asn Gln Asp Leu Asn Tyr
   275 280 285
   Cys Thr His His Lys Pro Cys Arg Asn Gly Ala Thr Cys Thr Asn Thr
   290 295 300
   Gly Gln Gly Ser Tyr Thr Cys Ser Cys Arg Pro Gly Tyr Thr Gly Ala
   305 310 315 320
   Asn Cys Glu Leu Glu Val Asp Glu Cys Ala Pro Ser Pro Cys Lys Asn
   325 330 335
   Gly Ala Ser Cys Thr Asp Leu Glu Asp Ser Phe Ser Cys Thr Cys Pro
   340 345 350
   Pro Gly Phe Tyr Gly Lys Val Cys Glu Leu Ser Ala Met Thr Cys Ala
   355 360 365
   Asp Gly Pro Cys Phe Asn Gly Gly Arg Cys Ser Asp Asn Pro Asp Gly
   370 375 380
   Gly Tyr Thr Cys His Cys Pro Leu Gly Phe Ser Gly Phe Asn Cys Glu
   385 390 395 400
   Lys Lys Met Asp Leu Cys Gly Ser Ser Pro Cys Ser Asn Gly Ala Lys
   405 410 415
   Cys Val Asp Leu Gly Asn Ser Tyr Leu Cys Arg Cys Gln Ala Gly Phe
   420 425 430
   Ser Gly Arg Tyr Cys Glu Asp Asn Val Asp Asp Cys Ala Ser Ser Pro
   435 440 445
   Cys Ala Asn Gly Gly Thr Cys Arg Asp Ser Val Asn Asp Phe Ser Cys
   450 455 460
   Thr Cys Pro Pro Gly Tyr Thr Gly Lys Asn Cys Ser Ala Pro Val Ser
   465 470 475 480
   Arg Cys Glu His Ala Pro Cys His Asn Gly Ala Thr Cys His Gln Arg
   485 490 495
   Gly Gln Arg Tyr Met Cys Glu Cys Ala Gln Gly Tyr Gly Gly Pro Asn
   500 505 510
   Cys Gln Phe Leu Leu Pro Glu Pro Pro Pro Gly Pro Met Val Val Asp
   515 520 525
   Leu Ser Glu Arg His Met Glu Ser Gln Gly Gly Pro Phe Pro Trp Val
   530 535 540
   Ala Val Cys Ala Gly Val Val Leu Val Leu Leu Leu Leu Leu Gly Cys
   545 550 555 560
   Ala Ala Val Val Val Cys Val Arg Leu Lys Leu Gln Lys His Gln Pro
   565 570 575
   Pro Pro Glu Pro Cys Gly Gly Glu Thr Glu Thr Met Asn Asn Leu Ala
   580 585 590
   Asn Cys Gln Arg Glu Lys Asp Val Ser Val Ser Ile Ile Gly Ala Thr
   595 600 605
   Gln Ile Lys Asn Thr Asn Lys Lys Ala Asp Phe His Gly Asp His Gly
   610 615 620
   Ala Lys Lys Ser Ser Phe Lys Val Arg Tyr Pro Thr Val Asp Tyr Asn
   625 630 635 640
   Leu Val Arg Asp Leu Lys Gly Asp Glu Ala Thr Val Arg Asp Thr His
   645 650 655
   Ser Lys Arg Asp Thr Lys Cys Gln Ser Gln Ser Ser Ala Gly Glu Glu
   660 665 670
   Lys Ile Ala Pro Thr Leu Arg Gly Gly Glu Ile Pro Asp Arg Lys Arg
   675 680 685
   Pro Glu Ser Val Tyr Ser Thr Ser Lys Asp Thr Lys Tyr Gln Ser Val
   690 695 700
   Tyr Val Leu Ser Ala Glu Lys Asp Glu Cys Val Ile Ala Thr Glu Val
   705 710 715 720
(2) INFORMATION FOR SEQ ID NO:5:
   (i) SEQUENCE CHARACTERISTICS:
      (A) LENGTH: 713 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO:5:
   Met Gly Arg Arg Ser Ala Leu Ala Leu Ala Val Val Ser Ala Leu Leu
   1 5 10 15
   Cys Gln Val Trp Ser Ser Gly Val Phe Glu Leu Lys Leu Gln Glu Phe
   20 25 30
   Val Asn Lys Lys Gly Leu Leu Gly Asn Arg Asn Cys Cys Arg Gly Gly
   35 40 45
   Ser Gly Pro Pro Cys Ala Cys Arg Thr Phe Phe Arg Val Cys Leu Lys
   50 55 60
   His Tyr Gln Ala Ser Val Ser Pro Glu Pro Pro Cys Thr Tyr Gly Ser
   65 70 75 80
   Ala Val Thr Ala Val Leu Gly Val Asp Ser Phe Ser Leu Pro Asp Gly
   85 90 95
   Ala Gly Ile Asp Pro Ala Phe Ser Asn Pro Ile Arg Phe Pro Phe Gly
   100 105 110
   Phe Thr Trp Pro Gly Thr Phe Ser Leu Ile Ile Glu Ala Leu His Thr
   115 120 125
   Asp Ser Pro Asp Asp Leu Ala Thr Glu Asn Pro Glu Arg Leu Ile Ser
   130 135 140
   Arg Leu Thr Thr Gln Arg His Thr Val Gly Glu Glu Trp Ser Gln Asp
   145 150 155 160
   Leu His Ser Ser Gly Arg Thr Asp Leu Arg Tyr Ser Tyr Arg Phe Val
   165 170 175
   Cys Asp Glu His Tyr Tyr Gly Glu Gly Cys Ser Val Phe Cys Arg Pro
   180 185 190
   Arg Asp Asp Ala Phe Gly His Phe Thr Cys Gly Glu Arg Gly Glu Lys
   195 200 205
   Met Cys Asp Pro Gly Trp Lys Gly Gln Tyr Cys Thr Asp Pro Ile Cys
   210 215 220
   Leu Pro Gly Cys Asp Asp Gln His Gly Tyr Cys Asp Lys Pro Gly Glu
   225 230 235 240
   Cys Lys Cys Arg Val Gly Trp Gln Gly Arg Tyr Cys Asp Glu Cys Ile
   245 250 255
   Arg Tyr Pro Gly Cys Leu His Gly Thr Cys Gln Gln Pro Trp Gln Cys
   260 265 270
   Asn Cys Gln Glu Gly Trp Gly Gly Leu Phe Cys Asn Gln Asp Leu Asn
   275 280 285
   Tyr Cys Thr His His Lys Pro Cys Arg Asn Gly Ala Thr Cys Thr Asn
   290 295 300
   Thr Gly Gln Gly Ser Tyr Thr Cys Ser Cys Arg Pro Gly Tyr Thr Gly
   305 310 315 320
   Ala Asn Cys Glu Leu Glu Val Asp Glu Cys Ala Pro Ser Pro Cys Lys
   325 330 335
   Asn Gly Gly Ser Cys Thr Asp Leu Glu Asp Ser Tyr Ser Cys Thr Cys
   340 345 350
   Pro Pro Gly Phe Tyr Gly Lys Val Cys Glu Leu Ser Ala Met Thr Cys
   355 360 365
   Ala Asp Gly Pro Cys Phe Asn Gly Gly Arg Cys Ser Asp Asn Pro Asp
   370 375 380
   Gly Gly Tyr Thr Cys His Cys Pro Ala Gly Phe Ser Gly Phe Asn Cys
   385 390 395 400
   Glu Lys Lys Ile Asp Leu Cys Ser Ser Ser Pro Cys Ser Asn Gly Ala
   405 410 415
   Lys Cys Val Asp Leu Gly Asn Ser Tyr Leu Cys Arg Cys Gln Thr Gly
   420 425 430
   Phe Ser Gly Arg Tyr Cys Glu Asp Asn Val Asp Asp Cys Ala Ser Ser
   435 440 445
   Pro Cys Ala Asn Gly Gly Thr Cys Arg Asp Ser Val Asn Asp Phe Ser
   450 455 460
   Cys Thr Cys Pro Pro Gly Tyr Thr Gly Lys Asn Cys Ser Ala Pro Val
   465 470 475 480
   Ser Arg Cys Glu His Ala Pro Cys His Asn Gly Ala Thr Cys His Gln
   485 490 495
   Arg Gly Gln Arg Tyr Met Cys Glu Cys Ala Gln Gly Tyr Gly Gly Ala
   500 505 510
   Asn Cys Gln Phe Leu Leu Pro Glu Pro Pro Pro Asp Leu Ile Val Ala
   515 520 525
   Ala Gln Gly Gly Ser Phe Pro Trp Val Ala Val Cys Ala Gly Val Val
   530 535 540
   Leu Val Leu Leu Leu Leu Leu Gly Cys Ala Ala Val Val Val Cys Val
   545 550 555 560
   Arg Leu Lys Leu Gln Lys His Gln Pro Pro Pro Asp Pro Cys Gly Gly
   565 570 575
   Glu Thr Glu Thr Met Asn Asn Leu Ala Asn Cys Gln Arg Glu Lys Asp
   580 585 590
   Val Ser Val Ser Ile Ile Gly Ala Thr Gln Ile Lys Asn Thr Asn Lys
   595 600 605
   Lys Ala Asp Phe His Gly Asp His Gly Ala Asp Lys Ser Ser Phe Lys
   610 615 620
   Ala Arg Tyr Pro Thr Val Asp Tyr Asn Leu Ile Arg Asp Leu Lys Gly
   625 630 635 640
   Asp Glu Ala Thr Val Arg Asp Ala His Ser Lys Arg Asp Thr Lys Cys
   645 650 655
   Gln Ser Gln Gly Ser Ala Gly Glu Glu Lys Ser Thr Ser Thr Leu Arg
   660 665 670
   Gly Gly Glu Val Pro Asp Arg Lys Arg Pro Glu Ser Val Tyr Ser Thr
   675 680 685
   Ser Lys Asp Thr Lys Tyr Gln Ser Val Tyr Val Leu Ser Ala Glu Lys
   690 695 700
   Asp Glu Cys Val Ile Ala Thr Glu Val
   705 710
(2) INFORMATION FOR SEQ ID NO:6:
   (i) SEQUENCE CHARACTERISTICS:
      (A) LENGTH: 157 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO:6:
   Glu Asn Ser Tyr Ser Cys Thr Cys Pro Pro Gly Phe Tyr Gly Lys Ile
   1 5 10 15
   Cys Glu Leu Ser Ala Met Thr Cys Ala Asp Gly Pro Cys Phe Asn Gly
   20 25 30
   Gly Arg Cys Ser Asp Ser Pro Asp Gly Gly Tyr Ser Cys Arg Cys Pro
   35 40 45
   Val Cys Tyr Ser Gly Phe Asn Cys Glu Lys Lys Ile Asp Tyr Cys Ser
   50 55 60
   Ser Ser Pro Cys Ser Asn Gly Ala Lys Cys Val Asp Leu Gly Asp Ala
   65 70 75 80
   Tyr Leu Cys Arg Cys Gln Ala Gly Phe Ser Cys Arg His Cys Asp Asp
   85 90 95
   Asn Val Asp Asp Cys Ala Ser Ser Pro Cys Ala Asn Gly Gly Thr Cys
   100 105 110
   Arg Asp Gly Val Asn Asp Phe Ser Cys Thr Cys Pro Pro Gly Tyr Thr
   115 120 125
   Gly Arg Asn Cys Ser Ala Pro Ala Ser Arg Cys Glu His Ala Pro Cys
   130 135 140
   His Asn Gly Ala Thr Cys His Glu Arg Gly His Arg Tyr
   145 150 155
(2) INFORMATION FOR SEQ ID NO:7:
   (i) SEQUENCE CHARACTERISTICS:
      (A) LENGTH: 721 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO:7:
   Met Gly Gln Gln Arg Met Leu Thr Leu Leu Val Leu Ser Ala Val Leu
   1 5 10 15
   Cys Gln Ile Ser Cys Ser Gly Leu Phe Glu Leu Arg Leu Gln Glu Phe
   20 25 30
   Val Asn Lys Lys Gly Leu Leu Gly Asn Met Asn Cys Cys Arg Pro Gly
   35 40 45
   Ser Leu Ala Ser Leu Gln Arg Cys Glu Cys Lys Thr Phe Phe Arg Ile
   50 55 60
   Cys Leu Lys His Tyr Gln Ser Asn Val Ser Pro Glu Pro Pro Cys Thr
   65 70 75 80
   Tyr Gly Gly Ala Val Thr Pro Val Leu Gly Thr Asn Ser Phe Val Val
   85 90 95
   Pro Glu Ser Ser Asn Ala Asp Pro Thr Phe Ser Asn Pro Ile Arg Phe
   100 105 110
   Pro Phe Gly Phe Thr Trp Pro Gly Thr Phe Ser Leu Ile Ile Glu Ala
   115 120 125
   Ile His Ala Asp Ser Ala Asp Asp Leu Asn Thr Glu Asn Pro Glu Arg
   130 135 140
   Leu Ile Ser Arg Leu Ala Thr Gln Arg His Leu Thr Val Gly Glu Gln
   145 150 155 160
   Trp Ser Gln Asp Leu His Ser Ser Asp Arg Thr Glu Leu Lys Tyr Ser
   165 170 175
   Tyr Arg Phe Val Cys Asp Glu Tyr Tyr Tyr Gly Glu Gly Cys Ser Asp
   180 185 190
   Tyr Cys Arg Pro Arg Asp Asp Ala Phe Gly His Phe Ser Cys Gly Glu
   195 200 205
   Lys Gly Glu Asn Leu Cys Asn Pro Gly Trp Lys Gly Leu Tyr Cys Thr
   210 215 220
   Glu Pro Ile Cys Leu Pro Gly Cys Asp Glu His His Gly Tyr Cys Asp
   225 230 235 240
   Lys Pro Gly Glu Cys Lys Cys Arg Val Gly Trp Gln Gly Arg Tyr Cys
   245 250 255
   Asp Glu Cys Ile Arg Tyr Pro Gly Cys Leu His Gly Thr Cys Gln Gln
   260 265 270
   Pro Trp Gln Cys Asn Cys Gln Glu Gly Trp Gly Gly Leu Phe Cys Asn
   275 280 285
   Gln Asp Leu Asn Tyr Cys Thr His His Lys Pro Cys Glu Asn Gly Ala
   290 295 300
   Thr Cys Thr Asn Thr Gly Gln Gly Ser Tyr Thr Cys Ser Cys Arg Pro
   305 310 315 320
   Gly Tyr Thr Gly Ser Asn Cys Glu Ile Glu Val Asn Glu Cys Asp Ala
   325 330 335
   Asn Pro Cys Lys Asn Gly Gly Ser Cys Ser Asp Leu Glu Asn Ser Tyr
   340 345 350
   Thr Cys Ser Cys Pro Pro Gly Phe Tyr Gly Lys Asn Cys Glu Leu Ser
   355 360 365
   Ala Met Thr Cys Ala Asp Gly Pro Cys Phe Asn Gly Gly Arg Cys Ala
   370 375 380
   Asp Asn Pro Asp Gly Gly Tyr Ile Cys Pro Cys Pro Val Gly Tyr Ser
   385 390 395 400
   Gly Phe Asn Cys Glu Lys Lys Ile Asp Tyr Cys Ser Ser Asn Pro Cys
   405 410 415
   Ala Asn Gly Ala Arg Cys Glu Asp Leu Gly Asn Ser Tyr Ile Cys Gln
   420 425 430
   Cys Gln Glu Gly Phe Ser Gly Arg Asn Cys Asp Asp Asn Leu Asp Asp
   435 440 445
   Cys Thr Ser Phe Pro Cys Gln Asn Gly Gly Thr Cys Gln Asp Gly Ile
   450 455 460
   Asn Asp Tyr Ser Cys Thr Cys Pro Pro Gly Tyr Ile Gly Lys Asn Cys
   465 470 475 480
   Ser Met Pro Ile Thr Lys Cys Glu His Asn Pro Cys His Asn Gly Ala
   485 490 495
   Thr Cys His Glu Arg Asn Asn Arg Tyr Val Cys Gln Cys Ala Arg Gly
   500 505 510
   Tyr Gly Gly Asn Asn Cys Gln Phe Leu Leu Pro Glu Glu Lys Pro Val
   515 520 525
   Val Val Asp Leu Thr Glu Lys Tyr Thr Glu Gly Gln Ser Gly Gln Phe
   530 535 540
   Pro Trp Ile Ala Val Cys Ala Gly Ile Val Leu Val Leu Met Leu Leu
   545 550 555 560
   Leu Gly Cys Ala Ala Val Val Val Cys Val Arg Val Arg Val Gln Lys
   565 570 575
   Arg Arg His Gln Pro Glu Ala Cys Arg Gly Glu Ser Lys Thr Met Asn
   580 585 590
   Asn Leu Ala Asn Cys Gln Arg Glu Lys Asp Ile Ser Val Ser Phe Ile
   595 600 605
   Gly Thr Thr Gln Ile Lys Asn Thr Asn Lys Lys Ile Asp Phe Leu Ser
   610 615 620
   Glu Ser Asn Asn Glu Lys Asn Gly Tyr Lys Pro Arg Tyr Pro Ser Val
   625 630 635 640
   Asp Tyr Asn Leu Val His Glu Leu Lys Asn Glu Asp Ser Pro Lys Glu
   645 650 655
   Glu Arg Ser Lys Cys Glu Ala Lys Cys Ser Ser Asn Asp Ser Asp Ser
   660 665 670
   Glu Asp Val Asn Ser Val His Ser Lys Arg Asp Ser Ser Glu Arg Arg
   675 680 685
   Arg Pro Asp Ser Ala Tyr Ser Thr Ser Lys Asp Thr Lys Tyr Gln Ser
   690 695 700
   Val Tyr Val Ile Ser Asp Glu Lys Asp Glu Cys Ile Ile Ala Thr Glu
   705 710 715 720
   Val
(2) INFORMATION FOR SEQ ID No:8:
   (i) SEQUENCE CHARACTERISTICS:
      (A) LENGTH: 729 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO:8:
   Met Gly Gly Arg Phe Leu Leu Thr Leu Ala Leu Leu Ser Ala Leu Leu
   1 5 10 15
   Cys Arg Cys Gln Val Asp Gly Ser Gly Val Phe Glu Leu Lys Leu Gln
   20 25 30
   Glu Phe Val Asn Lys Lys Gly Leu Leu Ser Asn Arg Asn Cys Cys Arg
   35 40 45
   Gly Gly Gly Pro Gly Gly Ala Gly Gln Gln Gln Cys Asp Cys Lys Thr
   50 55 60
   Phe Phe Arg Val Cys Leu Lys His Tyr Gln Ala Ser Val Ser Pro Glu
   65 70 75 80
   Pro Pro Cys Thr Tyr Gly Ser Ala Ile Thr Pro Val Leu Gly Ala Asn
   85 90 95
   Ser Phe Ser Val Pro Asp Gly Ala Gly Gly Ala Asp Pro Ala Phe Ser
   100 105 110
   Asn Pro Ile Arg Phe Pro Phe Gly Phe Thr Trp Pro Gly Thr Phe Ser
   115 120 125
   Leu Ile Ile Glu Ala Leu His Thr Asp Ser Pro Asp Asp Leu Thr Thr
   130 135 140
   Glu Asn Pro Glu Arg Leu Ile Ser Arg Leu Ala Thr Gln Arg His Leu
   145 150 155 160
   Ala Val Gly Glu Glu Trp Ser Gln Asp Leu His Ser Ser Gly Arg Thr
   165 170 175
   Asp Leu Lys Tyr Ser Tyr Arg Phe Val Cys Asp Glu His Tyr Tyr Gly
   180 185 190
   Glu Gly Cys Ser Val Phe Cys Arg Pro Arg Asp Asp Arg Phe Gly His
   195 200 205
   Phe Thr Cys Gly Glu Arg Gly Glu Lys Val Cys Asn Pro Gly Trp Lys
   210 215 220
   Gly Gln Tyr Cys Thr Glu Pro Ile Cys Leu Pro Gly Cys Asp Glu Gln
   225 230 235 240
   His Gly Phe Cys Asp Lys Pro Gly Glu Cys Lys Cys Arg Val Gly Trp
   245 250 255
   Gln Gly Arg Tyr Cys Asp Glu Cys Ile Arg Tyr Pro Gly Cys Leu His
   260 265 270
   Gly Thr Cys Gln Gln Pro Trp Gln Cys Asn Cys Gln Glu Gly Trp Gly
   275 280 285
   Gly Leu Phe Cys Asn Gln Asp Leu Asn Tyr Cys Thr His His Lys Pro
   290 295 300
   Cys Lys Asn Gly Ala Thr Cys Thr Asn Thr Gly Gln Gly Ser Tyr Thr
   305 310 315 320
   Cys Ser Cys Arg Pro Gly Tyr Thr Gly Ser Ser Cys Glu Ile Glu Ile
   325 330 335
   Asn Glu Cys Asp Ala Asn Pro Cys Lys Asn Gly Gly Ser Cys Thr Asp
   340 345 350
   Leu Glu Asn Ser Tyr Ser Cys Thr Cys Pro Pro Gly Phe Tyr Gly Lys
   355 360 365
   Asn Cys Glu Leu Ser Ala Met Thr Cys Ala Asp Gly Pro Cys Phe Asn
   370 375 380
   Gly Gly Arg Cys Thr Asp Asn Pro Asp Gly Gly Tyr Ser Cys Arg Cys
   385 390 395 400
   Pro Leu Gly Tyr Ser Gly Phe Asn Cys Glu Lys Lys Ile Asp Tyr Cys
   405 410 415
   Ser Ser Ser Pro Cys Ala Asn Gly Ala Gln Ala Cys Val Asp Leu Gly
   420 425 430
   Asn Ser Tyr Ile Cys Gln Cys Gln Ala Gly Phe Thr Gly Arg His Cys
   435 440 445
   Asp Asp Asn Val Asp Asp Cys Ala Ser Phe Pro Cys Val Asn Gly Gly
   450 455 460
   Thr Cys Gln Asp Gly Val Asn Asp Tyr Ser Cys Thr Cys Pro Pro Gly
   465 470 475 480
   Tyr Asn Gly Lys Asn Cys Ser Thr Pro Val Ser Arg Cys Glu His Asn
   485 490 495
   Pro Cys His Asn Gly Ala Thr Cys His Glu Arg Ser Asn Arg Tyr Val
   500 505 510
   Cys Glu Cys Ala Arg Gly Tyr Gly Gly Leu Asn Cys Gln Phe Leu Leu
   515 520 525
   Pro Glu Pro Pro Gln Gly Pro Val Ile Val Asp Phe Thr Glu Lys Tyr
   530 535 540
   Thr Glu Gly Gln Asn Ser Gln Phe Pro Trp Ile Ala Val Cys Ala Gly
   545 550 555 560
   Ile Ile Leu Val Leu Met Leu Leu Leu Gly Cys Ala Ala Ile Val Val
   565 570 575
   Cys Val Arg Leu Lys Val Gln Lys Arg His His Gln Pro Glu Ala Cys
   580 585 590
   Arg Ser Glu Thr Glu Thr Met Asn Asn Leu Ala Asn Cys Gln Arg Glu
   595 600 605
   Lys Asp Ile Ser Ile Ser Val Ile Gly Ala Thr Gln Ile Lys Asn Thr
   610 615 620
   Asn Lys Lys Val Asp Phe His Ser Asp Asn Ser Asp Lys Asn Gly Tyr
   625 630 635 640
   Lys Val Arg Tyr Pro Ser Val Asp Tyr Asn Leu Val His Glu Leu Lys
   645 650 655
   Asn Glu Asp Ser Val Lys Glu Glu His Gly Lys Cys Glu Ala Lys Cys
   660 665 670
   Glu Thr Tyr Asp Ser Glu Ala Glu Glu Lys Ser Ala Val Gln Leu Lys
   675 680 685
   Ser Ser Asp Thr Ser Glu Arg Lys Arg Pro Asp Ser Val Tyr Ser Thr
   690 695 700
   Ser Lys Asp Thr Lys Tyr Gln Ser Val Tyr Val Ile Ser Glu Glu Lys
   705 710 715 720
   Asp Glu Cys Ile Ile Ala Thr Glu Val
   725
(2) INFORMATION FOR SEQ ID NO:9:
   (i) SEQUENCE CHARACTERISTICS:
      (A) LENGTH: 717 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO:9:
   Met Gly Arg Leu Met Ile Ala Val Leu Leu Cys Val Met Ile Ser Gln
   1 5 10 15
   Gly Phe Cys Ser Gly Val Phe Glu Leu Lys Leu Gln Glu Phe Leu Asn
   20 25 30
   Lys Lys Gly Val Thr Gly Asn Ala Asn Cys Cys Lys Gly Ser Ala Ala
   35 40 45
   Glu Gly His Gln Cys Glu Cys Lys Thr Phe Phe Arg Ile Cys Leu Lys
   50 55 60
   His Tyr Gln Ala Asn Val Ser Pro Asp Pro Pro Cys Thr Tyr Gly Gly
   65 70 75 80
   Ala Val Thr Pro Val Leu Gly Ser Asn Ser Phe Gln Val Pro Glu Ser
   85 90 95
   Phe Pro Asp Ser Ser Phe Thr Asn Pro Ile Pro Phe Ala Phe Gly Phe
   100 105 110
   Thr Trp Pro Gly Thr Phe Ser Leu Ile Ile Glu Ala Leu His Thr Asp
   115 120 125
   Ser Thr Asp Asp Leu Ser Thr Glu Asn Pro Asp Arg Leu Ile Ser Arg
   130 135 140
   Met Thr Thr Gln Arg His Leu Thr Val Gly Glu Glu Trp Ser Gln Asp
   145 150 155 160
   Leu Gln Val Gly Gly Arg Thr Glu Leu Lys Tyr Ser Tyr Arg Phe Val
   165 170 175
   Cys Asp Glu His Tyr Tyr Gly Glu Gly Cys Ser Val Phe Cys Arg Pro
   180 185 190
   Arg Asp Asp Thr Phe Gly His Phe Thr Cys Gly Glu Arg Gly Glu Ile
   195 200 205
   Ile Cys Asn Ser Gly Trp Lys Gly Gln Tyr Cys Thr Glu Pro Ile Cys
   210 215 220
   Leu Pro Gly Cys Asp Glu Asp His Gly Phe Cys Asp Lys Pro Gly Glu
   225 230 235 240
   Cys Lys Cys Arg Val Gly Phe Ser Gly Lys Tyr Cys Asp Asp Cys Ile
   245 250 255
   Arg Tyr Pro Gly Cys Leu His Gly Thr Cys Gln Gln Pro Trp Gln Cys
   260 265 270
   Asn Cys Gln Glu Gly Trp Gly Gly Leu Phe Cys Asn Gln Asp Leu Asn
   275 280 285
   Tyr Cys Thr His His Lys Pro Cys Gln Asn Gly Ala Thr Cys Thr Asn
   290 295 300
   Thr Gly Gln Gly Ser Tyr Thr Cys Ser Cys Arg Pro Gly Phe Thr Gly
   305 310 315 320
   Asp Ser Cys Glu Ile Glu Val Asn Glu Cys Ser Gly Ser Pro Cys Arg
   325 330 335
   Asn Gly Gly Ser Cys Thr Asp Leu Glu Asn Thr Tyr Ser Cys Thr Cys
   340 345 350
   Pro Pro Gly Phe Tyr Gly Arg Asn Cys Glu Leu Ser Ala Met Thr Cys
   355 360 365
   Ala Asp Gly Pro Cys Phe Asn Gly Gly His Cys Ala Asp Asn Pro Glu
   370 375 380
   Gly Gly Tyr Phe Cys Gln Cys Pro Met Gly Tyr Ala Gly Phe Asn Cys
   385 390 395 400
   Glu Lys Lys Ile Asp His Cys Ser Ser Asn Pro Cys Ser Asn Asp Ala
   405 410 415
   Gln Cys Leu Asp Leu Val Asp Ser Tyr Leu Cys Gln Cys Pro Glu Gly
   420 425 430
   Phe Thr Gly Thr His Cys Glu Asp Asn Ile Asp Glu Cys Ala Thr Tyr
   435 440 445
   Pro Cys Gln Asn Gly Gly Thr Cys Gln Asp Gly Leu Ser Asp Tyr Thr
   450 455 460
   Cys Thr Cys Pro Pro Gly Tyr Thr Gly Lys Asn Cys Thr Ser Ala Val
   465 470 475 480
   Asn Lys Cys Leu His Asn Pro Cys His Asn Gly Ala Thr Cys His Glu
   485 490 495
   Met Asp Asn Arg Tyr Val Cys Ala Cys Ile Pro Gly Tyr Gly Gly Arg
   500 505 510
   Asn Cys Gln Phe Leu Leu Pro Glu Asn Pro Gln Gly Gln Ala Ile Val
   515 520 525
   Glu Gly Ala Asp Lys Arg Tyr Ser Tyr Glu Glu Asp Asp Gly Gly Phe
   530 535 540
   Pro Trp Thr Ala Val Cys Ala Gly Ile Ile Leu Val Leu Leu Val Leu
   545 550 555 560
   Ile Gly Gly Ser Val Phe Val Ile Tyr Ile Arg Leu Lys Leu Gln Gln
   565 570 575
   Arg Ser Gln Gln Ile Asp Ser His Ser Glu Ile Glu Thr Met Asn Asn
   580 585 590
   Leu Thr Asn Asn Arg Ser Arg Glu Lys Asp Leu Ser Val Ser Ile Ile
   595 600 605
   Gly Ala Thr Gln Val Lys Asn Ile Asn Lys Lys Val Asp Phe Gln Ser
   610 615 620
   Asp Gly Asp Lys Asn Gly Phe Lys Ser Arg Tyr Ser Leu Val Asp Tyr
   625 630 635 640
   Asn Leu Val His Glu Leu Lys Gln Glu Asp Leu Gly Lys Glu Asp Ser
   645 650 655
   Glu Arg Ser Glu Ala Thr Lys Cys Glu Pro Leu Asp Ser Asp Ser Glu
   660 665 670
   Glu Lys His Arg Asn His Leu Lys Ser Asp Ser Ser Glu Arg Lys Arg
   675 680 685
   Thr Glu Ser Leu Cys Lys Asp Thr Lys Tyr Gln Ser Val Phe Val Leu
   690 695 700
   Ser Glu Glu Lys Asp Glu Cys Ile Ile Ala Thr Glu Val
   705 710 715
(2) INFORMATION FOR SEQ ID NO:10:
   (i) SEQUENCE CHARACTERISTICS:
      (A) LENGTH: 642 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO:10:
   Met Ala Ser Pro Leu Leu Leu Val Tyr Val Ala Ala Thr Leu Cys Leu
   1 5 10 15
   Pro Leu Val Tyr Pro Ala Gly Val Phe Glu Leu Lys Ile His Ser Phe
   20 25 30
   Ser Thr Pro Arg Pro Ala Cys Ala Ala Gly Lys Ser Cys Asn Ile Phe
   35 40 45
   Phe Arg Val Cys Leu Lys His Ala Gln Pro Val Val Ser Pro Asp Pro
   50 55 60
   Pro Cys Thr Phe Gly Ser Ala Val Ser Asp Ile Leu Pro Ser Asp Ser
   65 70 75 80
   Lys Ala Ile Thr Asp Ser Ser Pro Ile Arg Val Pro Phe His Phe Lys
   85 90 95
   Trp Pro Gly Ile Phe Ser Leu Ile Ile Glu Ser Trp Thr Thr Asn Ser
   100 105 110
   Ala Glu Gln Ser Thr Glu Asn Pro Asp Asn Leu Leu Ser Arg Leu Ala
   115 120 125
   Thr Arg Arg Arg Leu Ser Ile Gly Glu Asp Trp Ser Gln Asp Ile His
   130 135 140
   Leu Gly Gln Gln Ser Glu Leu Arg Tyr Ser Tyr His Val Ser Cys Asp
   145 150 155 160
   Glu His Tyr Tyr Gly Asp Ser Cys Ser Asp Tyr Cys Arg Pro Arg Asp
   165 170 175
   Asp Asn Phe Gly His Tyr Thr Cys Asp Glu Gln Gly Asn Arg Leu Cys
   180 185 190
   Met Ser Gly Trp Lys Gly Glu Tyr Cys Ala Glu Pro Ile Cys Leu Pro
   195 200 205
   Gly Cys Ser Glu Ser His Gly Phe Cys Glu Leu Pro Gly Glu Cys Lys
   210 215 220
   Cys Arg Met Gly Trp Gln Gly Glu Leu Cys Asp Glu Cys Leu Arg Tyr
   225 230 235 240
   Pro Gly Cys Gln His Gly Ser Cys Ser Gln Pro Trp Glu Cys Ile Cys
   245 250 255
   Gln Glu Gly Trp Gly Gly Leu Phe Cys Asn Gln Asp Leu Asn Tyr Cys
   260 265 270
   Thr Asn His Gln Pro Cys Arg Asn Gly Ala Ser Cys Ile Asn Ile Gly
   275 280 285
   Gln Gly Ser Tyr Ser Cys Ser Cys Arg Ala Gly Phe Thr Gly Thr Asn
   290 295 300
   Cys Glu Ile Asp Ile Asn Glu Cys Ala Ser Asn Pro Cys Lys Asn Gly
   305 310 315 320
   Gly Ser Cys Asn Asp Leu Glu Asn Asp Tyr Glu Cys Val Cys Pro Arg
   325 330 335
   Gly Phe Tyr Gly Lys Asn Cys Asp Ile Ser Ala Met Thr Cys Glu Asp
   340 345 350
   Gly Pro Cys Phe Asn Gly Gly Thr Cys Ile Glu Lys Ser Ser Gly Val
   355 360 365
   Gly Tyr Val Cys Arg Cys Pro Pro Asn Tyr His Gly Ser Asn Cys Glu
   370 375 380
   Lys Lys Ile Asp Arg Cys Thr Asn Ser Pro Cys Leu Asn Gly Gly Gln
   385 390 395 400
   Cys Leu Asp Met Gly Arg Asn Val Leu Cys Lys Cys Arg Pro Gly Pro
   405 410 415
   Ser Gly Pro Arg Cys Glu Leu Asn Ile Asp Asp Cys Ala Ser Ser Pro
   420 425 430
   Cys Ala Asn Gly Gly Thr Cys Val Asp Ala Val Asn Ser Tyr Thr Cys
   435 440 445
   Ser Cys Thr Leu Gly Tyr Gly Gly Lys Asp Cys Thr Leu Arg Val Asp
   450 455 460
   Ala Cys Ser Ser Lys Pro Cys Lys Asn Gly Gly Thr Cys Tyr Thr Lys
   465 470 475 480
   Phe Thr Gly Asn Val Cys Gln Cys Pro Thr Gly Phe Met Gly Thr Ser
   485 490 495
   Cys Glu Phe Arg Val His Asp Pro Thr Pro Ala Ser His Arg Ala Asp
   500 505 510
   Ser Ser Asn Thr Leu Thr Met Val Val Cys Leu Gly Leu Leu Thr Phe
   515 520 525
   Phe Leu Leu Gly Cys Gly Val Phe Met Val Met Arg Gly Met Arg Arg
   530 535 540
   Gly His Phe Asn Glu Lys Gly Arg Val Asn Asn Asp Leu Glu Pro Lys
   545 550 555 560
   Asn Asn Leu Ile Glu Lys Glu Pro His Phe Lys Met Pro Asn Pro Asp
   565 570 575
   Tyr Leu Arg Glu Lys Ser Ser Ser Lys Gln Lys Leu Leu Gln Gly Ser
   580 585 590
   Glu Ser Glu Glu Glu Arg Ser Gly Arg Arg Thr Asp Arg Lys Pro Asp
   595 600 605
   Thr Lys Gln Cys Asn Pro Thr Ser Arg Tyr Pro Glu Asp Gly Ala Tyr
   610 615 620
   His Pro Ile Tyr Ile Leu Pro Glu Pro Glu Gln Cys Ile Phe Ala Thr
   625 630 635 640
   Glu Val
(2) INFORMATION FOR SEQ ID NO:11:
   (i) SEQUENCE CHARACTERISTICS:
      (A) LENGTH: 830 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO:11:
   Met His Trp Ile Lys Cys Leu Leu Thr Ala Phe Ile Cys Phe Thr Val
   1 5 10 15
   Ile Val Gln Val His Ser Ser Gly Ser Phe Glu Leu Arg Leu Lys Tyr
   20 25 30
   Phe Ser Asn Asp His Gly Arg Asp Asn Glu Gly Arg Cys Cys Ser Gly
   35 40 45
   Glu Ser Asp Gly Ala Thr Gly Lys Cys Leu Gly Ser Cys Lys Thr Arg
   50 55 60
   Phe Arg Val Cys Leu Lys His Tyr Gln Ala Thr Ile Asp Thr Thr Ser
   65 70 75 80
   Gln Cys Thr Tyr Gly Asp Val Ile Thr Pro Ile Leu Gly Glu Asn Ser
   85 90 95
   Val Asn Leu Thr Asp Ala Gln Arg Phe Gln Asn Lys Gly Phe Thr Asn
   100 105 110
   Pro Ile Gln Phe Pro Phe Ser Phe Ser Trp Pro Gly Thr Phe Ser Leu
   115 120 125
   Ile Val Glu Ala Trp His Asp Thr Asn Asn Ser Gly Asn Ala Arg Thr
   130 135 140
   Asn Lys Leu Leu Ile Gln Arg Leu Leu Val Gln Gln Val Leu Glu Val
   145 150 155 160
   Ser Ser Glu Trp Lys Thr Asn Lys Ser Glu Ser Gln Tyr Thr Ser Leu
   165 170 175
   Glu Tyr Asp Phe Arg Val Thr Cys Asp Leu Asn Tyr Tyr Gly Ser Gly
   180 185 190
   Cys Ala Lys Phe Cys Arg Pro Arg Asp Asp Ser Phe Gly His Ser Thr
   195 200 205
   Cys Ser Glu Thr Gly Glu Ile Ile Cys Leu Thr Gly Trp Gln Gly Asp
   210 215 220
   Tyr Cys His Ile Pro Lys Cys Ala Lys Gly Cys Glu His Gly His Cys
   225 230 235 240
   Asp Lys Pro Asn Gln Cys Val Cys Gln Leu Gly Trp Lys Gly Ala Leu
   245 250 255
   Cys Asn Glu Cys Val Leu Glu Pro Asn Cys Ile His Gly Thr Cys Asn
   260 265 270
   Lys Pro Trp Thr Cys Ile Cys Asn Glu Gly Trp Gly Gly Leu Tyr Cys
   275 280 285
   Asn Gln Asp Leu Asn Tyr Cys Thr Asn His Arg Pro Cys Lys Asn Gly
   290 295 300
   Gly Thr Cys Phe Asn Thr Gly Glu Gly Leu Tyr Thr Cys Lys Cys Ala
   305 310 315 320
   Pro Gly Tyr Ser Gly Asp Asp Glu Asn Glu Ile Tyr Ser Cys Asp Ala
   325 330 335
   Asp Val Asn Pro Cys Gln Asn Gly Gly Thr Cys Ile Asp Glu Pro His
   340 345 350
   Thr Lys Thr Gly Lys Cys His Cys Ala Asn Gly Trp Ser Gly Lys Met
   355 360 365
   Cys Glu Glu Lys Val Leu Thr Cys Ser Asp Lys Pro Cys His Gln Gly
   370 375 380
   Ile Cys Arg Asn Val Arg Pro Gly Leu Gly Ser Lys Gly Gln Gly Tyr
   385 390 395 400
   Gln Cys Glu Cys Pro Ile Gly Tyr Ser Gly Pro Asn Cys Asp Leu Gln
   405 410 415
   Leu Asp Asn Cys Ser Pro Asn Pro Cys Ile Asn Gly Gly Ser Cys Gln
   420 425 430
   Pro Ser Gly Lys Cys Ile Cys Pro Ala Gly Phe Ser Gly Thr Arg Cys
   435 440 445
   Glu Thr Asn Ile Asp Asp Cys Leu Gly His Gln Cys Glu Asn Gly Gly
   450 455 460
   Thr Cys Ile Asp Met Val Asn Gln Tyr Arg Cys Gln Cys Val Pro Gly
   465 470 475 480
   Phe His Gly Thr His Cys Ser Ser Lys Val Asp Leu Cys Leu Ile Arg
   485 490 495
   Pro Cys Ala Asn Gly Gly Thr Cys Leu Asn Leu Asn Asn Asp Tyr Gln
   500 505 510
   Cys Thr Cys Arg Ala Gly Phe Thr Gly Lys Asp Cys Ser Val Asp Ile
   515 520 525
   Asp Glu Cys Ser Ser Gly Pro Cys His Asn Gly Gly Thr Cys Met Asn
   530 535 540
   Arg Val Asn Ser Phe Glu Cys Val Cys Ala Asn Gly Phe Arg Gly Lys
   545 550 555 560
   Gln Cys Asp Glu Glu Ser Tyr Asp Ser Val Thr Phe Asp Ala His Gln
   565 570 575
   Tyr Gly Ala Thr Thr Gln Ala Arg Ala Asp Gly Leu Thr Asn Ala Gln
   580 585 590
   Val Val Leu Ile Ala Val Phe Ser Val Ala Met Pro Leu Val Ala Val
   595 600 605
   Ile Ala Ala Cys Val Val Phe Cys Met Lys Arg Lys Arg Lys Arg Ala
   610 615 620
   Gln Glu Lys Asp Asp Ala Glu Ala Arg Lys Gln Asn Glu Gln Asn Ala
   625 630 635 640
   Val Ala Thr Met His His Asn Gly Ser Gly Val Gly Val Ala Leu Ala
   645 650 655
   Ser Ala Ser Leu Gly Gly Lys Thr Gly Ser Asn Ser Gly Leu Thr Phe
   660 665 670
   Asp Gly Gly Asn Pro Asn Ile Ile Lys Asn Thr Trp Asp Lys Ser Val
   675 680 685
   Asn Asn Ile Cys Ala Ser Ala Ala Ala Ala Ala Ala Ala Ala Ala Ala
   690 695 700
   Ala Asp Glu Cys Leu Met Tyr Gly Gly Tyr Val Ala Ser Val Ala Asp
   705 710 715 720
   Asn Asn Asn Ala Asn Ser Asp Phe Cys Val Ala Pro Leu Gln Arg Ala
   725 730 735
   Lys Ser Gln Lys Gln Leu Asn Thr Asp Pro Thr Leu Met His Arg Gly
   740 745 750
   Ser Pro Ala Gly Ser Ser Ala Lys Gly Ala Ser Gly Gly Gly Pro Gly
   755 760 765
   Ala Ala Glu Gly Lys Arg Ile Ser Val Leu Gly Glu Gly Ser Tyr Cys
   770 775 780
   Ser Gln Arg Trp Pro Ser Leu Ala Ala Ala Gly Val Ala Gly Ala Cys
   785 790 795 800
   Ser Ser Gln Leu Met Ala Ala Ala Ser Ala Ala Gly Thr Asp Gly Thr
   805 810 815
   Ala Gln Gln Gln Arg Ser Val Val Cys Gly Thr Pro His Met
   820 825 830
(2) INFORMATION FOR SEQ ID NO:12:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "primer"
   (xi) SEQUENCE DESCRIPTION: SEQ ID NO:12:
      AGCGCCTCTG GCTGGGCGCT 20
(2) INFORMATION FOR SEQ ID NO:13:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "primer"
   (xi) SEQUENCE DESCRIPTION: SEQ ID NO:13:
      CGGCCAGAGG CCTTGCCACC 20
(2) INFORMATION FOR SEQ ID NO:14:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "primer"
   (xi) SEQUENCE DESCRIPTION: SEQ ID NO:14:
      TTGCGCTCCC GGCTGGAGCC 20
(2) INFORMATION FOR SEQ ID NO:15:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "primer"
   (xi) SEQUENCE DESCRIPTION: SEQ ID NO:15:
      ATGCGGCTTG GACCTCGGTT 20
(2) INFORMATION FOR SEQ ID NO:16:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO:16:
      TGCCGCCATC CCTCGGGGCG T 21
(2) INFORMATION FOR SEQ ID NO:17:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO:17:
      GGAGCTGCC GCCATCCCCT 20
(2) INFORMATION FOR SEQ ID NO:18:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO:18:
      GGACGCTGCC GCCATCCCCT CGGGGCGT 20
(2) INFORMATION FOR SEQ ID NO:19:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "primer"
   (xi) SEQUENCE DESCRIPTION: SEQ ID NO:19:
      TCAATCTGGC TCTGTTCGCG 20
(2) INFORMATION FOR SEQ ID NO:20:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "primer"
   (xi) SEQUENCE DESCRIPTION: SEQ ID NO:20:
      CGCTCTCTCC ACCCGCGGGC CCTCAA 26
(2) INFORMATION FOR SEQ ID NO:21:
   (i) SEQUENCE CHARACTERISTICS:
      (A) LENGTH: 171 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:21:
   GCCCAGGCNG ACCCTGGTGT GGACTGTGAG CTGGAGCTCA GCGAGTGTGA CAGCAACCCC 60
   TGTCGCANTG GAGGCAGCTG TAAGGACCAN GAGGATGGCT ACCACTGCCT GTGTCCTCCG 120
   GGCTACTACG GCNTGCATCG TGAACACNGC ACCTCTTAGC TGNGCCGACT C 171
(2) INFORMATION FOR SEQ ID NO:22:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "primer"
   (xi) SEQUENCE DESCRIPTION: SEQ ID NO:22:
      GTTTACATTG CATCCTGGAT 20
(2) INFORMATION FOR SEQ ID NO:23:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "primer"
   (xi) SEQUENCE DESCRIPTION: SEQ ID NO:23:
      CTCTTCTGTT CCTCTGGTTG 20

## Description

### 1. Background of the Invention

In developed countries, stroke is the third leading cause of death and the primary cause of acquired physical or cognitive impairment. Vascular dementia is the second leading cause of dementia, after Alzheimer's disease. CADASIL (for cerebral autosomal dominant arteriopathy with subcortical infarcts and leukoencephalopathy) causes a type of stroke and dementia whose key features include recurrent subcortical ischaemic events, progressive vascular dementia, craniofacial paralysis, migraine and mood disorders with severe depression (Chabriat, H. et al., (1995) Lancet 346: 934-939). These symptoms usually start to appear at about 45 years of age, and patients typically die by 65. The condition is believed to be largely undiagnosed and therefore the prevalence is not precisely known.

CADASIL is associated with diffuse white-matter abnormalities on neuroimaging (Tournier-Lasserve, E. et. al., (1991) Stroke 22:1297-1302). Pathological examination reveals multiple small, deep cerebral infarcts, a leukoencephalopathy and a non-atherosclerotic, non-amyloid angiopathy involving mainly the small cerebral arteries.(Baudrimont, M. et al., (1993) Stroke 24: 122-125). Severe alterations of vascular smooth muscle cells are evident on ultrastructural analysis (Ruchoux, M. M. et al., (1995) Acta. Neuropathol. 89:500-512).

It has recently been shown that the human Notch3 gene, located on chromosome 19, is mutated in CADASIL patients (Joutel. A. et al., (1996) Nature 383: 707-710). Most of the mutations cause amino acid changes in the extracellular domain. Therefore, disruption of the Notch signaling pathway appears to be responsible for CADASIL stroke and dementia.

Defects in the Notch signaling pathway may also be involved in other neurological diseases, e.g., Alzheimer's disease. In fact, approximately 10% of cases of Alzheimer's disease are associated with mutations in genes encoding the amyloid precursor proteins, presenilin 1 (PS1) and presenilin 2 (PS2). About 25% of early-onset familial Alzheimer's cases are associated with a mutation in PS1. PS1 and PS2 are transmembrane proteins which are homologous to the C. elegans protein encoded by the sel-12 gene, which has been shown to be genetically linked to the C. elegans lin-12 gene, which encodes a Notch-family receptor (Levitan, D. and I. Greenwald (1995) Nature 377:351-354); PS1 and PS2 are further described in PCT Application WO 96/34099; Sel12 is further described in PCT Application WO 97/11956). Furthermore, targeted disruption of PS1 in mice results in reduced expression of mRNA encoding Notch 1 and Delta-like gene 1 (DI11 a vertebrate Notch ligand, in the presomitic mesoderm compared to control mice (Wong et al. (1997) Nature 387:288). This indicates that PS 1 is required for the spatiotemporal expression of genes involved in the Notch signaling pathway.

The Notch signaling pathway comprises Notch proteins, which are membrane proteins, and proteins interacting with Notch proteins, termed Delta proteins. The product of the Delta gene, acting as a ligand, and that of the Notch gene, acting as a receptor, are key components in a lateral-inhibition signaling pathway that regulates the detailed patterning of many different tissues in Drosophila (Vassin,H., et al., (1987) EMBO J 6:3431-3440; Kopczynski, C. et al., (1988) Genes Dev. 2:1723-1735; Fehon, R.G. et al., (1990) Cell 61:523-534; Artavanis-Tsakonas, S. et., al., (1991) Trends, Genet. Sci. 7:403-407; Heitzler, P. et. al., (1991) Cell 64: 1083-1092; Greenwald, I. et al., (1992) Cell 68: 271-281; Fortini, M et al., (1993) Cell 75: 124501247; and Muskavitch, M. (1994) Devi. Biol. 166:415-430). During neurogenesis in particular, neural precursors, by expressing Delta, inhibit neighboring Notch-expressing cells from becoming committed to a neural fate. Mutations leading to a failure of lateral inhibition cause an overproduction of neurons, giving rise to a phenotype termed the "neurogenic phenotype" in Drosophila. For example, loss of Notch 1 leads to somite defects and embryonic death in mice, whereas constitutively active mutant forms of Notch 1 appear to inhibit cell differentiation in Xenopus and in cultured mammalian cells (Swiatek et at. (1994) Genes Dev. 8:707; Conlon et al. (1995) J. Development 121:1533; Lopan et al. (1994) Development 120:2385; and Nye et al. (1994) Development 120:2421). Similarly, reduced activity of X-Deltal causes more cells to become primary neurons, whereas raised activity causes fewer cells to become primary neurons (Chitnis et al. (1995) Nature 375:761). Furthermore, loss of D111 function in mice leads to excessive neuronal differentiation, resulting in severe patterning defects in the paraxial mesoderm and a hyperplastic central nervous system (CNS) (Hrabe de Angelis et al. (1997) Nature 386:717). Thus, the Notch signaling pathway, in particular Delta proteins, mediate lateral inhibition during neurogenesis so that only a limited proportion of cells having the potential to become neurons will in fact differentiate into neurons.

The Notch family of proteins are transmembrane receptors containing several conserved peptide motifs. The extracellular domains contain many tandemly repeated copies of an epidermal growth factor (EGF) like motif. The intracellular domains contain six copies of another conserved motif, termed the Cdc10/ankyrin repeat. Both the EGF and the ankyrin-repeat motifs are found in many different proteins and, in at least some cases, they have been shown to be involved in protein-protein interactions.

The Drosophila Notch protein encodes a glycosylated transmembrane receptor having a molecular mass of 350KD, which is involved in cell-fate specification during development (Wharton, K.A, et al., (1985) Cell 43:567-581); Artavanis-Tsakonas, S. et al., (1995) Science 268: 225-232). Based on analysis of Drosophila mutants, it is thought that Notch is a receptor having different functional domains, with the intracellular domain having the intrinsic signal-transducing activity of the intact protein and the extracellular domain a regulating activity (Rebay, I et al., (1993) Cell 74: 319-329).

Several Notch homologues have been identified in vertebrates (Larsson, C., et al., (1994) Genomics 24: 253-258). Three Notch proteins (Notch1, also called TAN1, Notch2, and Notch3) have been characterized in humans (Ellisen, L.W. et al., (1991) Cell 66:649-661; Stifani, S. et al., (1992) Nature Genet. 2: 119-127). Notch1 gene translocations have been associated with a minority of T-cell lymphoblastic leukemias (Aster, J. (1994) Cold Spring Harb. Symp. Quant. Biol. 59:125-136) and Notch3 has been linked with CADASIL.

A protein interacting with Notch was first discovered in Drosophila and has been called Delta protein. This protein encodes a transmembrane protein ligand, which contains tandem arrays of epidermal growth factor-like repeats in the extracellular domain. The Delta and Notch proteins can directly bind to each other and specific EGF-like repeats are sufficient and necessary for this binding (Fehon, R.G. et al., (1990) Cell 61:523-534; Rebay I., et al., (1991) Cell 67:687-699; and Lieber, T., et al., (1992) Neuron 9: 847-859).

In addition to the *Drosophila* Delta protein, a chick Delta homologue, GDeIta protein (Henrique, D et al., (1995) Nature 375: 787-790 and GenBank Accession No. U26590) two *Xenopus* homologues, X-Delta-1 and X-Delta-2 (Chitnis, A et al., (1995) Nature 375:761-766 and GenBank Accession Nos. L42229 and U70843), a mouse homologue (GenBank Accession No. X80903), a delta-like human gene 1(Dlk) (Bettenhausen, B. et al., (1995) Development 121:2407-2418) a rat homologue (GenBank Accession No. U78889), and a Zebrafish homologue (GenBank Accession No. Y11760) have been identified *Xenopus,* chick and mouse Delta genes are also disclosed in International Patent Application No. PCT/US96/11178 (Publication No. WO 97/01571. The patent application also discloses a partial and error prone human Delta homolog (hD1). Nucleotide sequence of human Notch genes are disclosed in International Patent Application No. PCT/US92/03651 (Publication No. WO 92/19734) and International Patent Application No. PCT/US93/09338 (Publication No. WO 94/07474).

Therapeutics, which modulate (agonize or antagonize) the Delta/Notch signaling pathway would be useful for prevention and treatment of various diseases and disorders, including neurological and vascular disorders. In addition, mutations in Delta genes that correlate with the existence of or a predisposition to development of a disease can provide useful diagnostics.

### 2. Summary of the Invention

In a first aspect of the present invention, there is provided an isolated nucleic acid molecule selected from the group consisting of:
a) a nucleic acid molecule whose nucleotide sequence is at least 95% identical to the nucleotide sequence of SEQ ID NO:1 or SEQ ID NO:3, the cDNA insert of the plasmid deposited with the ATCC as Accession Number 98348, or a complement thereof;
b) a nucleic acid molecule which encodes a polypeptide which has the amino acid sequence of SEQ ID-NO:2 or the amino acid sequence encoded by the cDNA insert of the plasmid deposited with the ATCC as Accession Number 98348.

The invention is based at least in part on the discovery of a human gene encoding a novel Delta protein which differs substantially from the previously described Delta proteins. Accordingly, the novel Delta protein of the invention is referred to herein as Delta3. Thus, the invention provides Delta3 proteins, and nucleic acids encoding Delta3 proteins, as defined in the claims. An exemplary hDelta3 is contained in a plasmid which was deposited with the American Type Culture Collection (ATCC) on March 5, 1997, and has been assigned ATCC accession number 98348.

Based on Northern blot analysis of RNA prepared from a number of human tissues, a 3.5kb message was expressed in fetal brain, lung, liver and kidney; and adult heart, placenta, lung, skeletal muscle, kidney, pancreas, spleen, thymus, prostate, testis, ovary, small intestine and colon. In addition, the hDelta3 gene was found to be expressed at relatively high levels in at least some tumor cells (e.g. colon carcinoma) and its expression could be up-regulated in response to various growth factors (e.g., bFGF and VEGF). Furthermore, the expression of hDelta3 was also shown to be increased in response to a signal induced differentiation of endothelial cells, indicating a role for hDelta3 in modulating the growth and/or differentiation of cells, in particular endothelial cells.

As described herein, the hDelta3 gene has been localized on human chromosome 15, close to framework markers D15S1244 and D15S144, a chromosomal region that has been associated with the neurological disease Agenesis of the Corpus Callosum with Peripheral Neuropathy (ACCPN) (Casaubon et al. (1996) Am J. Hum. Genet. (58:28).

For expression, the subject Delta3 nucleic acids can include a transcriptional regulatory sequence, e.g. at least one of a transcriptional promoter (e.g., for constitutive expression or inducible expression) or transcriptional enhancer sequence, the regulatory sequence is operably linked to the Delta3 gene sequence. Such regulatory sequences in conjunction with Delta3 nucleic acid molecules can be useful in vectors for gene expression. This invention also describes host cells transfected with said expression vectors whether prokaryotic or eukaryotic. In vitro (e.g. cell culture) and *in vivo* (e.g. transgenic) methods for producing Delta3 proteins by employing said expression vectors are also described.

In another aspect, the invention features isolated Delta3 polypeptides whose amino acid sequence is at least 95% identical to the amino acid sequence of SEQ ID NO:2, preferably substantially pure preparations, e.g. of plasma purified or recombinantly produced Delta3 polypeptides.

A still further aspect of the present invention features antibodies and antibody fragments specifically reactive with an isolated polypeptide whose amino acid sequence is at least 95% identical to the amino acid sequence of SEQ ID No: 2.

Other features and advantages of the invention will be apparent from the following detailed description and claims.

### 3. Brief Description of the Figures

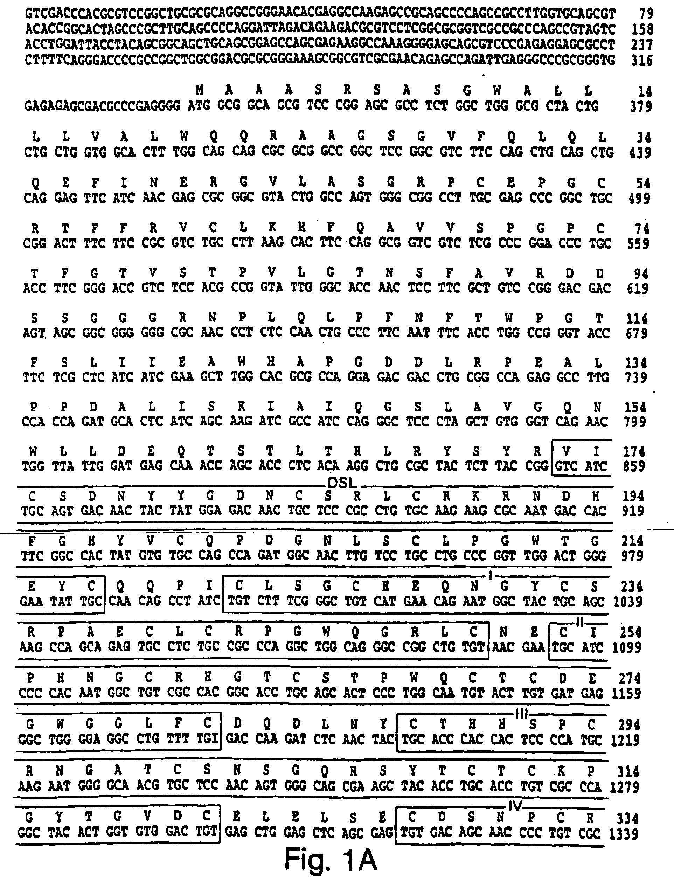

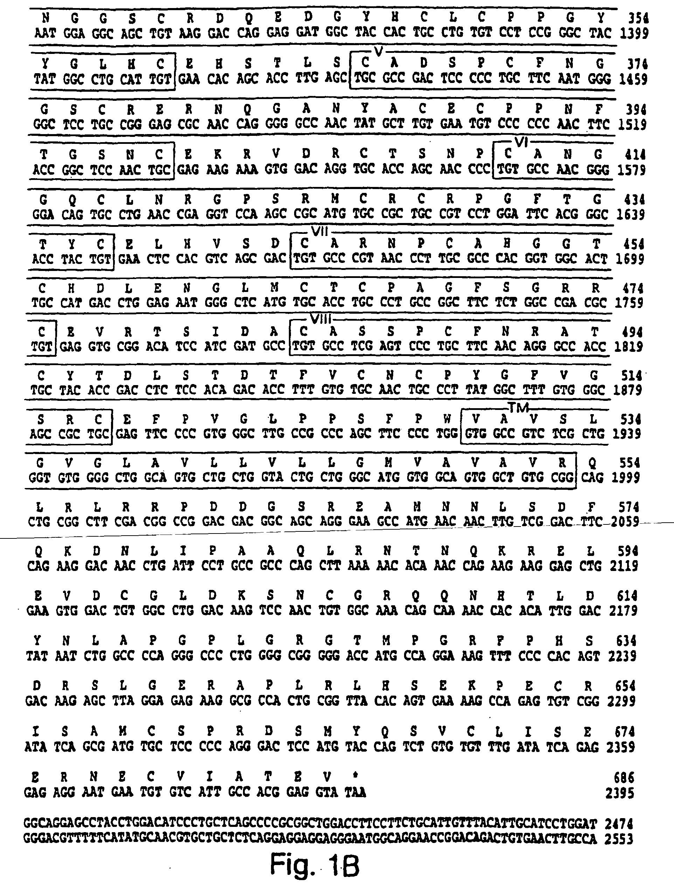

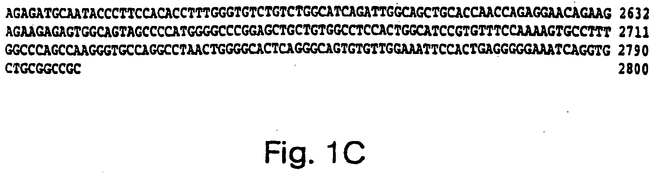

Figure 1 shows a DNA sequence of the human Delta3 gene including 5' and 3' noncoding sequences (SEQ ID No 1), as well as the deduced amino acid sequence of the human Delta3 protein (SEQ ID No 2). The various domains of the protein are indicated.

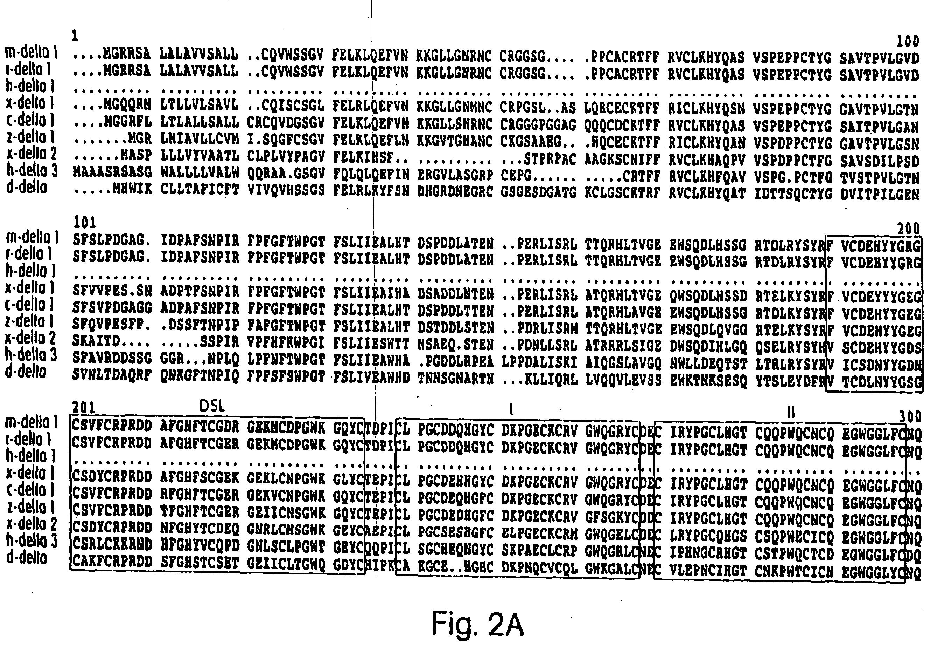

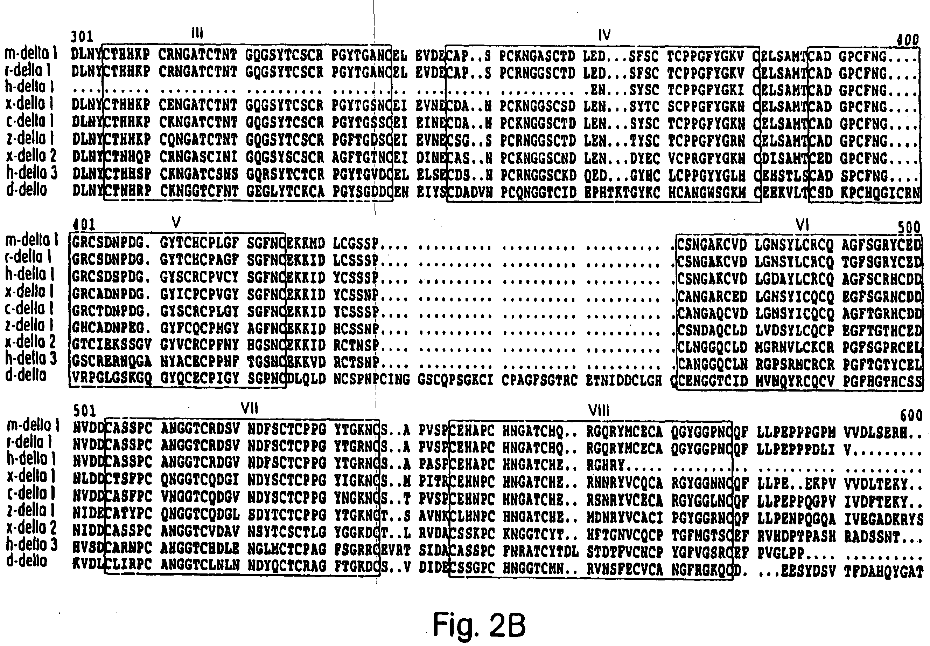

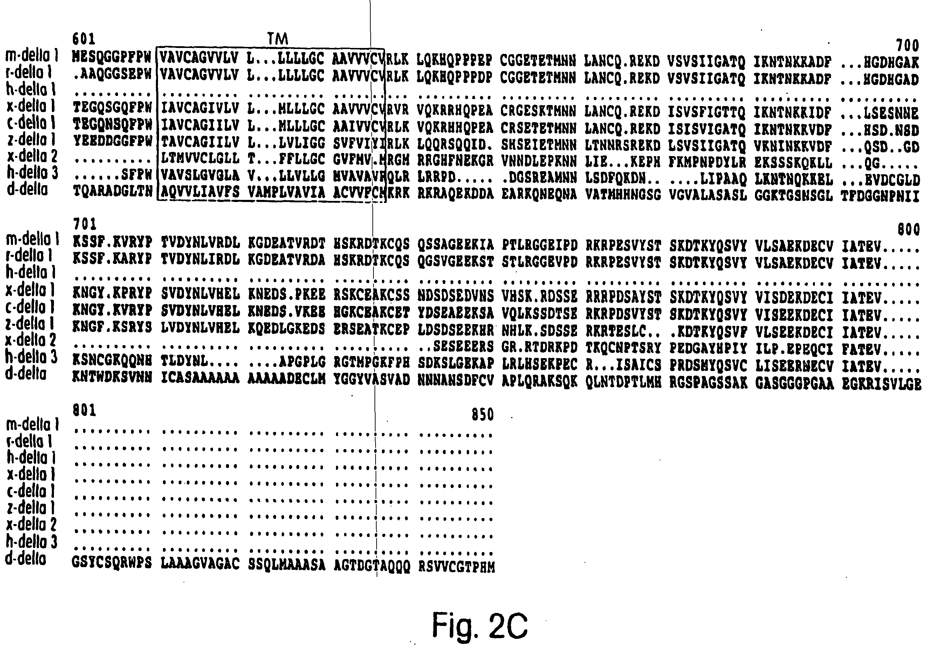

Figure 2 shows a multiple sequence alignment of the novel human Delta3 protein (h-Delta3) (SEQ ID No 2) with the mouse Delta1 protein (m-delta1) (SEQ ID No 4), the rat Deltal protein (r-deltal) (SEQ ID No 5), the partial human Deltal (WO 97/01571) protein (SEQ ID No 6), the *Xenopus* Delta1 protein (x-delta 1) (SEQ ID No 7), the chick Delta1 protein (c-deltal) (SEQ ID No 8), the zebrafish Delta1 protein (z-delta1) (SEQ ID No 9) the *Xenopus* Delta2 protein (x-delta2) (SEQ ID No 10) and the *Drosophila* Delta1 protein (d-delta1) (SEQ ID No 11). Conservation of the Delta3 similarity (DSL) domain, the epidermal growth factor-like (EGF) repeats and the transmembrane domain (TM) is indicated. The GenBank Accession No. of each of these Delta proteins (with the exception of the partial human sequence, which is not in GenBank) is indicated in Table I.

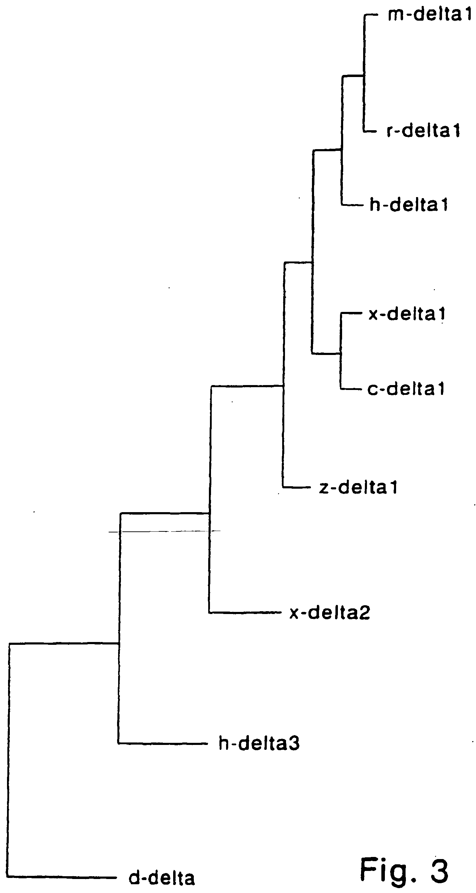

Figure 3 shows a phylogenic tree indicating the relationship of hDelta3 with the partial human delta1 (WO 97/01571) protein, the mouse Delta1 protein (m-delta1), the rat Delta1 protein (r-delta1), the *Xenopus* Delta1 protein (x-delta1), the chick Delta1 protein (c-delta1), the *Xenopus* Delta2 protein (x-delta2), the zebrafish Delta1 protein (z-delta1), and the *Drosophila* Delta1 protein (d-delta1). The GenBank Accession No. of each of these Delta proteins (with the exception of the partial human sequence, which is not in GenBank) is indicated in Table I.

### 4. Detailed Description of the Invention

### General

The present invention is based at least in part on the discovery of a novel gene encoding a human Delta protein, referred to herein as "hDelta3" polypeptide. An exemplary hDelta3 has been deposited with the American Type Culture Collection (ATCC) on March 5, 1997 and has been assigned ATCC accession number 98348. The human Delta3 gene maps to human chromosome 15.

Figure 1 shows the DNA sequence of the human Delta3 gene including 5' and 3' noncoding sequences (SEQ ID No.1), the coding sequence (SEQ ID No. 3), as well as the deduced amino acid sequence of the human Delta3 protein (SEQ ID No. 2).

Human Delta3 is expressed in endothelial cells and in fact was cloned from a human microvascular endothelial cell library. Northern blot analysis of RNA prepared from a number of different human tissues, indicate that a 3.5kb Delta3 mRNA transcript is present in fetal brain, lung, liver and kidney, and adult heart, placenta, lung, skeletal muscle, kidney, pancreas, spleen, thymus, prostate, testis, ovary, small intestine and colon. Low levels of Delta3 mRNA was also detected in adult brain and adult liver. However, no Delta3 mRNA was detected in peripheral blood leukocytes. These results indicate that Delta3 is expressed in a tissue specific manner. Further, expression in human MV endothelial cells was found to be up-regulated (about 2-3 fold) in cells that had been stimulated with certain growth factors (e.g. basic fibroblast growth factor (bFGF) or vascular endothelial growth factor (VEGF)). In addition, strong expression of human Delta3 was observed in the colorectal carcinoma cell line, SW480. Furthermore, expression of hDelta3 has been shown to be induced in response to proliferation and differentiation signals (See Examples). Thus, the hDelta3 gene is a gene whose expression in a cell changes with the proliferative and/or differentiative state.

As predicted from the nucleotide sequence of the nucleic acid encoding hDelta3, the full length hDelta3 polypeptide comprises 685 amino acids and is similar in sequence and structure to Delta proteins obtained from other organisms (See Figure 2 and discussed below). An amino acid sequence analysis of the human Delta3 protein predicts that the protein comprises at least the following structural domains: a signal peptide, corresponding to about amino acid 1 to about amino acid 17 of SEQ ID No. 2, a Delta Similarity (DSL) motif corresponding to about amino acid 173 to about amino acid 217 of SEQ ID NO: 2 (Figure 2), eight epidermal growth factor (EGF)-like repeats corresponding essentially to the sequences indicated in Figure 2, a transmembrane domain corresponding to about amino acid 530 to about amino acid 553 of SEQ ID No. 2 (Figure 2), and a cytoplasmic domain corresponding to about amino acid 554 to about amino acid 685 of SEQ ID No. 2 (Figure 2). Accordingly, the amino acid sequence of hDelta3 predicts that the protein is a transmembrane protein having an extracellular domain corresponding to about amino acid 1 or amino acid 18 to about amino acid 529 of SEQ ID No. 2 (Figure 2) comprising the DSL motif and the EGF-like repeats, a transmembrane domain and a cytoplasmic domain. It is possible that the signal peptide comprises more than 17 amino acids of SEQ ID No. 2, since the region from amino acid 1 to about amino acid 20 form a hydrophobic region.

It is possible that soluble forms of the protein also exist. Such soluble isoforms can arise through variable splicing of the Delta3 gene or alternatively as a result of proteolysis of a membranous isoform. In fact, a splice variant of a chicken Delta protein have been described in PCT Application PCT/US96/11178 having Publication No. WO 97/01571. Furthermore, the human Delta-like polypeptide Dlk is a soluble protein (Jansen et al. [1994] Eur. J. Biochem. 225:83-92).

Human Delta3 protein is similar in structure and in sequence to the Delta proteins identified in *Drosophila*, *Xenopus,* zebrafish, chicken, rat, mouse, and human. An alignment of the amino acid sequences of the Delta proteins known to date is shown in Figure 2. This alignment contains the following Delta proteins, encoded by genes for which the GenBank Accession Nos. are shown in Table 1: a mouse Delta1 protein (m-delta1), rat Delta1 protein (r-delta1), a human Delta-1 protein (h-delta1), a *Xenopus* Deltal protein (x-delta1), a chicken Delta1 protein (c-delta1), a zebrafish Delta1 protein (z-delta1), a second *Xenopus* Delta protein (x-delta2), the human Delta3 protein (h-delta3), and a *Drosophila* Delta1 protein (d-deltal). The amino acid sequence of h-delta1 is the amino acid sequence published in PCT application WO 97/01571 which is incomplete and contains numerous errors, as stated in the application. Since the amino acid sequence alignment has been done using the pileup computer program (GCG Package), the order of the amino acid sequences in the figure reflects the homology between the different Delta proteins. Accordingly, the *Drosophila* protein, which corresponds to the bottom sequence in the alignment is most distant from the other Delta proteins. Thus, Figure 2 shows that hDelta3, which is listed second to last in Figure 2, is the second to most distant Delta protein from the previously identified mouse, rat, human, *Xenopus,* zebrafish, and chicken delta protein. Accordingly, the hDelta3 protein is significantly different from the previously described human Delta protein, as well as the Delta proteins from the other species. Interestingly, the hDelta3 protein has an amino acid sequence which is equally distant from both *Xenopus* proteins, i.e., Delta1 and Delta2, indicating that hDelta3 does not correspond to either of the *Xenopus* Delta proteins. Therefore, the newly isolated polypeptide has been termed hDelta3 and the previously identified mouse, rat, human, zebrafish, and *Xenopus* Delta proteins are termed Delta1 proteins herein and the two *Xenopus* proteins are termed Delta1 and Delta2 proteins. The difference between hDelta3 protein and previously isolated Delta proteins can also be visualized by comparing the percent homology or identity between hDelta3 and the previously identified Delta1 and Delta2 proteins on one hand (Table I), and the percent homology or identity of a Delta1 protein with the other Delta1 and Delta2 proteins (Table II).

**Table I:**

| **Percent similarity between the amino acid sequence of human Delta3 (SEQ ID No.2) and that of the various Delta proteins** | | | |
|---|---|---|---|
| | GenBank Accession No. | % identity | % similarity |
| human Delta1 | | 50 | 66 |
| mouse Delta1 | X80903 | 53 | 70 |
| rat Delta1 | U78889 | 54 | 70 |
| chicken Delta1 | U26590 | 52 | 68 |
| *Xenopus* Delta1 | L42229 | 51 | 68 |
| zebrafish Deltal1 | Y11760 | 48 | 67 |
| *Xenopus* Delta2 | U70843 | 47 | 65 |
| *Drosophila* Delta1 | AA142228 | 40 | 58 |
| hDelta-like (dlk) | U15979 | 33 | 55 |

Table II indicates the percent similarity and identity between human Delta 1 disclosed in PCT Application PCT/US96/11178 (Publication No. WO 97/01571) and non-human Deltal proteins. Since the amino acid sequence of the human Delta1 protein that is disclosed in this PCT application is incomplete, the percentage similarity and identity was determined using a portion of the human Delta1 amino acid sequence which seems most reliable. The portion of the amino acid sequence used corresponds to amino acids 214-370 of the human Delta1 amino acid sequence shown in Figure 14A of the PCT application.

**Table II:**

| **Percent similarity between human Delta1 and the various non-human Delta1 or Delta2 proteins** | | | |
|---|---|---|---|
| | GenBank Accession No. | % identity | % similarity |
| human Delta1 | | 100 | 100 |
| mouse Delta1 | X80903 | 86 | 95 |
| rat Delta1 | U78889 | 88 | 94 |
| chicken Delta1 | U26590 | 85 | 89 |
| *Xenopus* Deltal | L42229 | 78 | 84 |
| zebrafish Delta1 | Y11760 | 69 | 80 |
| *Xenopus* Delta2 | U70843 | 57 | 70 |
| Drosophila Delta1 | AA142228 | 45 | 62 |
| hDelta-like (dlk) | U15979 | 37 | 55 |

Accordingly, Table I indicates that hDelta3 is only approximately 66% similar to the human Deltal protein; approximately 70% similar to the mouse Deltal protein; approximately 70% similar to the rat Delta1 protein; approximately 68% similar to the chick Delta1 protein; approximately 68% similar to the *Xenopus* Delta1 protein, approximately 70% similar to the *Xenopus* Delta2 protein and approximately 58% similar to the *Drosophila* Delta1 protein. However, as shown in Table II, the human-Delta1 protein is very similar to the mouse, rat, chick, *Xenopus,* zebrafish, and *Drosophila* Delta1 and the *Xenopus* Delta2 proteins. In addition, mouse and rat Delta1 proteins are about 95% similar. Thus, the amino acid sequence of Delta1 proteins share more homology with each other than with the human Delta3 protein of the invention, indicating that at least two families of Delta proteins exist.

The difference between the newly isolated hDelta3 protein and the previously identified Delta1 and Delta2 proteins can also be seen by creating a phylogenic tree using the Growtree Phylogram computer program (GCG Package). The result of this analysis, which is shown in Figure 3, indicate that h-Delta3 is on a different "branch" in the phylogenic tree from the other Delta proteins, thus confirming that hDelta3 protein is more distant from the other Delta1 and Delta2 proteins than they are distant from each other. According to the analysis, and as predicted by the sequence alignment, only the *Drosophila* Delta protein is more distantly related to the previously identified mouse, rat, *Xenopus,* chicken, zebrafish and human Delta proteins than hDelta3. Thus, the newly isolated hDelta3 protein is a member of a different subspecies of the family of Delta proteins.

Notwithstanding that each animal species is likely to have at least two or three members (e.g. Delta1, Delta2, and Delta3), it can be seen from Figure 2, that the DSL region, the eight EGF repeats and the TM appear to be highly conserved throughout. However, as can be seen in Figure 2. these domains of the hDelta3 protein differ more from the corresponding domains in the other Delta proteins than the corresponding domains in the other Delta differ from one another.

The DSL region or motif is shared by all known members of the family of presumed ligands of Notch-like proteins (Delta1 and Serrate in *Drosophila;* Lag-2 and Apx-1 in Caenorhabditis elegans) (Henderson et al. (1994) Development 120:2913; Tax et al. (1994) Nature 368:150; Fleming et al. (1990) Genes Dev. 4:2188; Thomas et al. (1991) Development 11:749; Mello et al. (1994) Cell 77:95). The DSL motif is located in the amino terminal portion of the protein which is closely related to a similar domain in the *Drosophila* Delta1 protein and which has been described as being necessary and sufficient for in vitro binding to Notch (Henrique et al. (1995) Nature 375:787; Muskavitch (1994) Dev. Biol. 166:415).

Furthermore, as set forth in Example, 5.5., Delta3 has been localized to human chromosome 15 in a region dose to the framework markers D15S1244 and D15S144. Interestingly, the region on chromosome 15 that is flanked by the markers D15S1040 and D15S118 has been shown to be genetically linked with the disease called Agenesis of the Corpus Callosum with Peripheral Neuropathy (ACCPN) (Casaubon et al., *supra).* No specific gene has so far been linked to this disease.

Accordingly, certain aspects of the present invention relate to Delta3 proteins, nucleic acid molecules encoding Delta3 proteins, antibodies immunoreactive with Delta3 proteins, and preparations of such compositions. In addition, drug discovery assays are described for identifying agents that modulate the biological function of Delta proteins, e.g,. Delta3 proteins, such as by binding to Delta3 or by altering the interaction of Delta3 with either downstream or upstream elements in the Delta/Notch signal transduction pathway, e.g. by altering the interaction between Delta3 and a Delta3 binding protein. Such agents can be useful therapeutically, for example, to alter cell growth and/or differentiation or induction of apoptosis. Moreover, the present disclosure describes diagnostic and therapeutic assays and reagents for detecting and treating disorders involving an aberrant Delta3 activity, for example, aberrant expression (or loss thereof) of Delta3 gene or which are associated with a specific Delta allele, e.g,. a Delta3 allele. Other aspects of the invention are described below or will be apparent to one of skill in the art in light of the present disclosure.

### 4.2 Definitions

For convenience, the meaning of certain terms and phrases employed in the specification, examples, and appended claims are provided below.

The term "allele", which is used interchangeably herein with "allelic variant" refers to alternative forms of a gene or portions thereof. Alleles occupy the same locus or position on homologous chromosomes. When a subject has two identical alleles of a gene, the subject is said to be homozygous for the gene or allele. When a subject has two different alleles of a gene, the subject is said to be heterozygous for the gene. Alleles of a specific gene can differ from each other in a single nucleotide, or several nucleotides, and can include substitutions, deletions, and insertions of nucleotides. An allele of a gene can also be a form of a gene containing a mutation.

The term "allelic variant of a polymorphic region of a Delta gene" refers to a region of a Delta gene having one of several nucleotide sequences found in that region of the gene in other individuals.

The term "agonist", as used herein, is meant to refer to an agent that upregulates (e.g. potentiates or supplements) a Delta3 bioactivity. A Delta3 agonist can be a compound that upregulates expression of a Delta3 gene. Alternatively, a Delta3 agonist can be a compound which increases signalling from a Delta3 protein, e.g., a compound bound to Delta3, such as a stimulatory form of a toporythmic protein or a small molecule. A Delta3 agonist can also be a compound which modulates the expression or activity of a protein which is located downstream of Delta3 or which interacts with Delta3.

"Antagonist" as used herein is meant to refer to an agent that downregulates (e.g. suppresses or inhibits) a Delta3 bioactivity. A Delta3 antagonist can be a compound that downregulates expression of a Delta3 gene. Alternatively, Delta3 antagonist can be a compound which decreases signalling from a Delta3 protein, e.g., a compound binding to Delta3 such as an inhibitory form of a toporythmic protein, or a small molecule. A preferred Delta3 antagonist inhibits the interaction between a Delta3 protein and another molecule, e.g., a toporythmic protein. A Delta3 antagonist can also be a compound which modulates the expression or activity of a protein which is located downstream of Delta3 or which interacts with Delta3.

"Biological activity" used interchangeably with the terms "bioactivity" or "activity" for the purposes herein means an effector or antigenic function that is directly or indirectly performed by a Delta3 polypeptide (whether in its native or denatured conformation), or by any subsequence thereof. Effector functions include receptor binding or activation, induction of differentiation, mitogenic or growth promoting activity, induction of apoptosis, signal transduction, immune modulation, DNA regulatory functions and the like, whether presently known or inherent. Antigenic functions include possession of an epitope or antigenic site that is capable of cross-reacting with antibodies raised against a naturally occurring or denatured Delta3 polypeptide or fragment thereof. Accordingly, a biological activity of a Delta3 protein can be binding to a receptor, such as Notch. A biological activity of a Delta3 protein can also be modulation of cell proliferation and/or differentiation, or cell death in a target cell having an appropriate receptor. A target cell can be, e.g., a neural cell, an endothelial cell, or a cancer cell.

Biologically active Delta3 polypeptides include polypeptides having both an effector and antigenic function, or only one of such functions. Delta3 includes antagonist polypeptides and native Delta3 , provided that such antagonists include an epitope of a native Delta3 or antagonize a biologic activity of native Delta3.

The term "aberrant Delta3 activity" or "abnormal Delta3 activity" is intended to encompass an activity of Delta3 which differs from the same Delta3 activity in a healthy subject. An aberrant Delta3 activity can result, e.g., from a mutation in the protein, which results, e.g., in lower or higher binding affinity to a receptor. An aberrant Delta3 activity can also result from a lower or higher level of Delta3 on cells, which can result, e.g., from aberrant transcription, splicing, or translation of the Delta3 gene. For example, an aberrant Delta3 activity can result from an abnormal promoter activity. An aberrant Delta3 activity can also result from an aberrant signalling through the cytoplasmic domain of the Delta3 protein, such that, e.g., an aberrant signal is transduced. Aberrant signalling can result from a mutation in the cytoplasmic domain of Delta3 or, alternatively, from the contact with an abnormal cytoplasmic protein. An aberrant Delta3 activity can also result from contact of a Delta3 protein with an aberrant receptor, e.g., abnormal Notch protein.

"Cells," "host cells" or "recombinant host cells" are terms used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A "chimeric protein" or "fusion protein" is a fusion of a first amino acid sequence encoding one of the subject Delta3 polypeptides with a second amino acid sequence defining a domain (e.g. polypeptide portion) foreign to and not substantially homologous with any domain of one of the Delta3 proteins. A chimeric protein may present a foreign domain which is found (albeit in a different protein) in an organism which also expresses the first protein, or it may be an "interspecies", "intergenic", e.g. fusion of protein structures expressed by different kinds of organisms. In general, a fusion protein can be represented by the general formula X- Delta3-Y, wherein Delta3 represents a portion of the protein which is derived from one of the Delta3 proteins, and X and Y are independently absent or represent amino acid sequences which are not related to one of the Delta3 sequences in an organism, including naturally occurring mutants. A preferred Delta3 fusion protein is a Delta3-Ig fusion protein.

"Complementary" sequences as used herein refer to sequences which have sufficient complementarity to be able to hybridize, forming a stable duplex.

A "delivery complex" shall mean a targeting means (e.g. a molecule that results in higher affinity binding of a gene, protein, polypeptide or peptide to a target cell surface and/or increased cellular uptake by a target cell). Examples of targeting means include: sterols (e.g. cholesterol), lipids (e.g. a cationic lipid, virosome or liposome), viruses (e.g. adenovirus, adeno-associated virus, and retrovirus) or target cell specific binding agents (e.g. ligands recognized by target cell specific receptors). Preferred complexes are sufficiently stable *in vivo* to prevent significant uncoupling prior to internalization by the target cell. However, the complex is cleavable under appropriate conditions within the cell so that the gene, protein, polypeptide or peptide is released in a functional form.

As is well known, genes for a particular polypeptide may exist in single or multiple copies within the genome of an individual. Such duplicate genes may be identical or may have certain modifications, including nucleotide substitutions, additions or deletions, which all still code for polypeptides having substantially the same activity. The term "DNA sequence encoding a Delta3 polypeptide" may thus refer to one or more genes within a particular individual. Moreover, certain differences in nucleotide sequences may exist between individual organisms, which are called alleles. Such allelic differences may or may not result in differences in amino acid sequence of the encoded polypeptide yet still encode a protein with the same biological activity.

The term "Delta3 therapeutic" refers to various compositions of Delta3 polypeptides, as well as peptidomimetics, small molecules and nucleic acids which are capable of mimicking or potentiating (agonizing) or inhibiting (antagonizing) the effects of a naturally-occurring Delta3 protein. A Delta3 therapeutic which mimics or potentiates the activity of a wild-type Delta3 protein is a " Delta3 agonist". Conversely, a Delta3 therapeutic which inhibits the activity of a wild-type Delta3 protein is a " Delta3 antagonist"

The terms "Delta3 polypeptide" and "Delta3 protein" are intended to encompass Delta3 polypeptides which have at least one biological activity, i.e., antagonizing at least one biological activity of a native Delta3 polypeptide.

As used herein, the term "gene" or "recombinant gene" , as applied to Delta3, refers to a nucleic acid molecule comprising an open reading frame encoding one of the Delta3 polypeptides of the present invention, including both exon and (optionally) intron sequences. A "recombinant Delta3 gene" refers to nucleic acid encoding a Delta3 polypeptide and comprising Delta-encoding exon sequences, though it may optionally include intron sequences which are either derived from a chromosomal Delta3 gene or from an unrelated chromosomal gene. Exemplary recombinant genes encoding the subject Delta3 polypeptides are represented in the appended Sequence Listing. The term "intron" refers to a DNA sequence present in a given Delta3 gene which is not translated into protein and is generally found between exons.

The term "growth state" of a cell refers to the proliferative state of a cell as well as to its differentiative state. Accordingly, the term refers to the phase of the cell cycle in which the cell is, e.g., G0, G1, G2, prophase, metaphase, or telophase, as well as to its state of differentiation, e.g., undiffereniated, partially differentiated, or fully differentiated. Without wanting to be limited, differentiation of a cell is usually accompanied by a decrease in the proliferative rate of a cell.

"Homology" or "identity" or "similarity" refers to sequence similarity between two peptides or between two nucleic acid molecules Homology can be determined by comparing a position in each sequence which may be aligned for purposes of comparison. When a position in the compared sequence is occupied by the same base or amino acid, then the molecules are identical at that position. A degree of homology or similarity or identity between nucleic acid sequences is a function of the number of identical or matching nucleotides at positions shared by the nucleic acid sequences. A degree of identity of amino acid sequences is a function of the number of identical amino acids at positions shared by the amino acid sequences. A degree of homology or similarity of amino acid sequences is a function of the number of conserved amino acids at positions shared by the amino acid sequences. A sequence which is "unrelated" or "non-homologous" with one of the hDelta3 sequences of the present invention is a sequence which shares less than 40 % identity, though preferably less than 25 % identity with one of the hDelta3 sequences of the present invention.

The term "interact" as used herein is meant to include detectable interactions between molecules, such as can be detected using, for example, a yeast two hybrid assay. The term interact is also meant to include "binding" interactions between molecules. Interactions may be protein-protein, protein-nucleic acid, protein-small molecule, or nucleic acid-small molecule in nature.

The term "isolated" as used herein with respect to nucleic acids, such as DNA or RNA, refers to molecules separated from other DNAs, or RNAs, respectively, that are present in the natural source of the macromolecule. For example, an isolated nucleic acid encoding one of the subject Delta3 polypeptides preferably includes no more than 10 kilobases (kb) of nucleic acid sequence which naturally immediately flanks the Delta3 gene in genomic DNA, more preferably no more than 5kb of such naturally occurring flanking sequences, and most preferably less than 1.5kb of such naturally occurring flanking sequence. The term isolated as used herein also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides.

The term "modulation" as used herein refers to both upregulation, i.e., stimulation, and downregulation, i. e. suppression, of a response.

The term "mutated gene" refers to an allelic form of a gene, which is capable of altering the phenotype of a subject having the mutated gene relative to a subject which does not have the mutated gene. If a subject must be homozygous for this mutation to have an altered phenotype, the mutation is said to be recessive. If one copy of the mutated gene is sufficient to alter the genotype of the subject, the mutation is said to be dominant. If a subject has one copy of the mutated gene and has a phenotype that is intermediate between that of a homozygous and that of a heterozygous (for that gene) subject, the mutation is said to be co-dominant.

The "non-human animals" of the invention include mammalians such as rodents, non-human primates, sheep, dog, cow, chickens, amphibians, reptiles, etc. Preferred non-human animals are selected from the rodent family including rat and mouse, most preferably mouse, though transgenic amphibians, such as members of the *Xenopus* genus, and transgenic chickens can also provide important tools for understanding and identifying agents which can affect, for example, embryogenesis and tissue formation. The term "chimeric animal" is used herein to refer to animals in which the recombinant gene is found, or in which the recombinant is expressed in some but not all cells of the animal. The term "tissue-specific chimeric animal" indicates that one of the recombinant Delta3 genes is present and/or expressed or disrupted in some tissues but not others.

As used herein, the term "nucleic acid" refers to polynucleotides such as deoxyribonucleic acid (DNA), and, where appropriate, ribonucleic acid (RNA). The term should also be understood to include, as equivalents, analogs of either RNA or DNA made from nucleotide analogs, and, as applicable to the embodiment being described, single (sense or antisense) and double-stranded polynucleotides.

The term "polymorphism" refers to the coexistence of more than one form of a gene or portion thereof. A portion of a gene of which there are at least two different forms, i.e., two different nucleotide sequences, is referred to as a "polymorphic region of a gene". A polymorphic region can be a single nucleotide, the identity of which differs in different alleles. A polymorphic region can also be several nucleotides long.

A "polymorphic gene" refers to a gene having at least one polymorphic region.

The terms "protein", "polypeptide" and "peptide" are used interchangably herein when referring to a gene product.

The term "recombinant protein" refers to a polypeptide of the present invention which is produced by recombinant DNA techniques, wherein generally, DNA encoding a Delta3 polypeptide is inserted into a suitable expression vector which is in turn used to transform a host cell to produce the heterologous protein. Moreover, the phrase "derived from", with respect to a recombinant Delta3 gene, is meant to include within the meaning of "recombinant protein" those proteins having an amino acid sequence of a native Delta3 protein, or an amino acid sequence similar thereto which is generated by mutations including substitutions and deletions (including truncation) of a naturally occurring form of the protein.

As used herein, the term "specifically hybridizes" or "specifically detects" refers to the ability of a nucleic acid molecule of the invention to hybridize to at least approximately 6, 12, 20, 30, 50, 100, 150, 200, 300, 350, 400 or 425 consecutive nucleotides of a vertebrate, preferably mammalian, Delta3 gene, such as a Delta3 sequence designated in one of SEQ ID Nos:1 or 3, or a sequence complementary thereto, or naturally occurring mutants thereof, such that it shows more than 10 times more hybridization, preferably more than 100 times more hybridization, and even more preferably more than 100 times more hybridization than it does to a cellular nucleic acid (e.g., mRNA of genomic DNA) encoding a protein other than a vertebrate preferably Delta3 protein as defined herein.

As used herein, the term "tissue-specific promoter" means a DNA sequence that serves as a promoter, i.e., regulates expression of a selected DNA sequence operably linked to the promoter, and which effects expression of the selected DNA sequence in specific cells of a tissue, such as cells of hepatic or pancreatic origin, neuronal cells, or immune cells. The term also covers so-called "leaky" promoters, which regulate expression of a selected DNA primarily in one tissue, but cause expression in other tissues as well.

"Transcriptional regulatory sequence" is a generic term used throughout the specification to refer to DNA sequences, such as initiation signals, enhancers, and promoters, which induce or control transcription of protein coding sequences with which they are operably linked. In preferred embodiments, transcription of one of the recombinant Delta3 genes is under the control of a promoter sequence (or other transcriptional regulatory sequence) which controls the expression of the recombinant gene in a cell-type in which expression is intended. It will also be understood that the recombinant gene can be under the control of transcriptional regulatory sequences which are the same or which are different from those sequences which control transcription of the naturally-occurring forms of Delta3 proteins.

As used herein, the term "transfection" means the introduction of a nucleic acid, e.g., an expression vector, into a recipient cell by nucleic acid-mediated gene transfer. "Transformation", as used herein, refers to a process in which a cell's genotype is changed as a result of the cellular uptake of exogenous DNA or RNA, and, for example, the transformed cell expresses a recombinant form of a Delta3 polypeptide or, in the case of anti-sense expression from the transferred gene, the expression of a naturally-occuming form of the Delta3 protein is disrupted.

As used herein, the term "transgene" means a nucleic acid sequence (encoding, e.g., one of the Delta3 polypeptides, or an antisense transcript thereto), which is partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can include one or more transcriptional regulatory sequences and any other nucleic acid, such as introns, that may be necessary for optimal expression of a selected nucleic acid.

As used herein, the term "vector" refers to a nucleic acid molecule capable of transporting another nucleic acid to which it has been linked. One type of preferred vector is an episome, i.e., a nucleic acid capable of extra-chromosomal replication. Preferred vectors are those capable of autonomous replication and/expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer generally to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

The term "treating" as used herein is intended to encompass curing as well as ameliorating at least one symptom of the condition or disease.

### 4.3 Nucleic Acids of the Present Invention

The nucleic acids of the invention are defined in claim 1. As described below, the disclosure describes isolated nucleic acids comprising nucleotide sequences encoding Delta3 polypeptides, and/or equivalents of such polypeptides or nucleic acids. The term equivalent is understood to include nucleotide sequences encoding functionally equivalent Delta3 polypeptides or functionally equivalent peptides having an activity of a Delta3 protein such as described herein. Equivalent nucleotide sequences will include sequences that differ by one or more nucleotide substitutions, additions or deletions, such as allelic variants; and will, therefore, include sequences that differ from the nucleotide sequence of the Delta3 gene shown in any of SEQ ID Nos:1 or 3 due to the degeneracy of the genetic code.

In a preferred embodiment, the nucleic acid encodes a protein which has the amino acid set forth in SEQ ID No. 2.

The nucleic acids of the disclosure can encode a Delta3 protein from any species, including insects. Nucleic acids can encode vertebrate Delta3 proteins. Other nucleic acids encode primate Delta3 proteins including mammalian Delta3 proteins, e.g., human Delta3 proteins. Other nucleic acids can encode avian, equine, canine, feline, bovine or porcine Delta3 proteins.

In a part of the disclosure, the nucleic acid encodes a polypeptide comprising an extracellular domain of Delta3, e.g., Delta3 having SEQ ID No. 2. Accordingly, nucleic acids can encode a polypeptide comprising about amino acid 1 to about amino acid 529 or SEQ ID No. 2. Other nucleic acids can encode a polypeptide corresponding to an extracellular domain of Delta3 essentially lacking the signal peptide, e.g, a polypeptide comprising about amino acid 18 to about amino acid 529 of SEQ ID No. 2. Yet other nucleic acids encode a polypeptide comprising at least one of the conserved motifs in the extracellular domain of Delta3, e.g., a DSL motif or an EGF-like motif, such as those shown in Figure 2 (SEQ ID No. 2). The nucleic acid can encode a protein having at least one EGF-like motif. Other nucleic acid encodes proteins having at least 2, at least 3, at least 4, at least 5, at least 6, at least 7, or 8 EGF-like repeats, such as those shown in Figure 2 (SEQ ID No.2). The polypeptide encoded by a nucleic acid encoding any of these numbers of EGF-like repeats can further comprise an amino acid sequence encoding a DSL motif

Polypeptides encoded by any of the above-described nucleic acids can be soluble. Soluble peptides can comprise at least a portion of the extracellular domain of a Delta3 protein. Soluble polypeptides can comprise an amino acid sequence corresponding to about amino acid 1 to about amino acid 529 of SEQ ID No. 2 or homolog thereof Yet other soluble Delta3 polypeptides comprise at least one EGF-like repeat. Such polypeptides may in addition comprise a DSL domain and optionally a signal peptide.

Also described are nucleic acids that encode a Delta3 polypeptide which is a fusion protein. A preferred fusion protein is a Delta3-Ig fusion protein. Such fusion proteins can comprise at least a portion of the extracellular domain of a Delta3 domain. A portion can be any portion of at least about 10 amino acids, such as the portions described above. Nucleic acids encoding such fusion proteins can be prepared as described in U.S. Patent No. 5,434,131.

Alternatively, polypeptides encoded by the nucleic acid of the disclosure can be membrane bound. Membrane bound polypeptides of the disclosure can comprise a transmembrane domain. The transmembrane domain can be from a Delta3 protein, such as a transmembrane domain comprising about amino acid 530 to about amino acid 553 of SEQ ID No. 2, shown in Figure 2. Alternatively, the transmembrane domain can be from another membrane protein, such as to produce a chimeric membraneous Delta3 protein. Yet other polypeptides of the disclosure can be intracellular proteins. Accordingly, also described are proteins which do not comprise a transmembrane domain. Other proteins of the invention do not include an extracellular domain. Additional proteins of the disclosure do not include an extracellular domain nor a transmembrane domain.

Polypeptides encoded by the nucleic acid of the disclosure can comprise a cytoplasmic domain. A nucleic acid of the disclosure can encode a polypeptide comprising a Delta3 cytoplasmic domain. The cytoplasmic domain can have an amino acid sequence corresponding to a sequence from about amino acid 554 to about amino acid 685 of SEQ ID No. 2 (Figure 2), or a portion thereof.

In yet other parts of the disclosure, the nucleic acid encodes a polypeptide comprising at least one domain of a Delta3 protein selected from the group consisting of: a signal peptide, a DSL motif, an EGF-like repeat, a transmembrane domain, and a cytoplamic domain. The polypeptide of the disclosure can comprise several of these domains from a Delta3 protein. Alternatively, a polypeptide of the disclosure can be a chimeric protein, i.e., comprising several of these conserved domains, at least some of which are derived from a protein other than a Delta3 protein. Accordingly, one nucleic acid of the disclosure encodes a polypeptide having a DSL motif having an amino acid sequence from a Delta3 protein other than Delta3 . Such an amino acid sequence can be any sequence shown in Figure 2 as being a DSL motif. Yet another nucleic acid encodes a Delta3 protein having a signal peptide from a protein other than a Delta3 protein. Also described are nucleic acids encoding a Delta3 polypeptide having a cytoplasmic domain from a protein other than a Delta3 protein and nucleic acids encoding a Delta3 cytoplasmic domain and a extracellular domain from a protein other than a Delta3 protein. Proteins other than Delta3 proteins can be, e.g., toporythmic proteins. "Toporythmic proteins" is intended to include Notch, Delta, Serrate, Enhancer of Split, Deltex, and other members of this family of proteins sharing structural similarities. (See e.g. International Patent Application Nos. WO 97/01571; WO 92/19734 and WO 92/19734 and WO 94/07474, *supra*).

Nucleic acids encoding polypeptides having an amino acid sequence that is homologous to any of the above described portions of SEQ ID No. 2 are also described. Some nucleic acids of the disclosure encode polypeptides comprising an amino acid sequence which is at least about 70%, at least about 75%, at least about 80%, or at least about 85% homologous or identical to the amino acid sequence of any of the Delta3 domains shown in Figure 2. Even more nucleic acids of the disclosure encode polypeptides comprising an amino acid sequence which is at least about 90%, at least about 95%, at least about 98%, or at least about 99% homologous or identical to the amino acid sequence of any of the Delta3 domains shown in Figure 2.

In one part of the disclosure, the nucleic acid, e.g.,cDNA, encodes a peptide having at least one bioactivity of the subject Delta3 polypeptide, such as the ability to bind to a Delta3 receptor, e.g., Notch. Proteins or peptides capable of interacting with a Delta3 protein or fragment thereof can be identified by various methods, e.g., methods based on binding assays. For example, various types of expression libraries can be screened with a Delta3 protein or portion thereof. A 2-hybrid system can be used to isolate cytoplasmic proteins interacting with the cytoplasmic domain of Delta3. Portions of Delta3 proteins which are capable of interacting with a ligand can be determined by preparing fragments of Delta3 proteins and screening these fragments for those that are capable of interacting with the ligand. Based at least in part on the observation that the N-terminal portion of Drosophila Delta protein, which contains a DSL domain and EGF-like domain, is necessary and sufficient for *in vitro* binding to Notch (Henrique et al., *supra;* Muskavitch et al., *supra*), it is likely that the domain of Delta3 proteins capable of interacting with a ligand includes the DSL domain and/or at least a portion of the EGF-like domain. However, other portions of the extracellular domain of Delta3 could be necessary for binding to at least some Delta3 ligands.

In other preferred embodiments, the subject Delta3 polypeptide can modulate proliferation and/or differentiation or cell death of specific target cells, e.g., neural cells or endothelial cells. Assays for determining that a Delta3 polypeptide has at least one bioactivity of a Delta3 protein are described infra.

The disclosure further describes nucleic acid molecules for use as probes/primer or antisense molecules (i.e. noncoding nucleic acid molecules), which can comprise at least about 6, 12, 20, 30, 50, 100, 125, 150 or 200 nucleotides or base pairs. Yet other nucleic acids of the disclosure comprise at least about 300, at least about 350, at least about 400, at least about 450, at least about 500, or at least about 600 nucleotides of SEQ ID No.1 or 3. Some nucleic acids of the disclosure correspond to a 5' portion of nucleic acid sequence SEQ ID No. 1. For example, a nucleic acid of the disclosure can correspond to a portion of about nucleotide 1 to about nucleotide 2000 of nucleic acid sequence SEQ ID No. 1.

Disclosed nucleic acids for use as a probe according to the methods of the disclosure include nucleic acids comprising a nucleotide sequence having at least about 6, preferably at least about 9, more preferably at least about 12 and even more preferably at least about 15 consecutive nucleotides from SEQ ID No. 1 or from a portion thereof The portion can correspond to about nucleotide 1 to about nucleotide 2060 of SEQ ID No. 1. Alternatively a portion can be a nucleotide sequence encoding a conserved motif of hDelta3 protein. Alternatively, the portion can be a nucleotide sequence located between nucleic acid sequences encoding conserved motifs of hDelta3 protein.

The disclosure further provides for a combination of at least two nucleic acids corresponding to at least a portion of SEQ ID No. 1 or a homolog thereof Accordingly, the disclosure describes a combination of two nucleic acids of at least about 6, at least about 9, at least about 12 and y at least about 15 consecutive nucleotides from SEQ ID No. 1 or from a portion thereof. At least one of the nucleic acids can be labeled.

Also described is a nucleic acid which hybridizes under stringent conditions to a nucleic acid represented by one of SEQ ID Nos:1 or 3. Appropriate stringency conditions which promote DNA hybridization, for example, 6.0 x sodium chloride/sodium citrate (SSC) at about 45°C, followed by a wash of 2.0 x SSC at 50°C, are known to those skilled in the art or can be found in Current Protocols in Molecular Biology, John Wiley & Sons, N.Y. (1989), 6.3.1-6.3.6. For example, the salt concentration in the wash step can be selected from a low stringency of about 2.0 x SSC at 50°C to a high stringency of about 0.2 x SSC at 50°C or at 65°C. In addition, the temperature in the wash step can be increased from low stringency conditions at room temperature, about 22°C, to high stringency conditions at about 65°C. Both temperature and salt may be varied, or temperature of salt concentration may be held constant while the other variable is changed. In a part of the disclosure, a conservin nucleic acid of the present disclosure will bind to one of SEQ ID Nos 1 or 3 under moderately stringent conditions, for example at about 2.0 x SSC and about 40°C. A particular Delta3 nucleic acid of the present disclosure will bind to one of SEQ ID Nos: 1 or 3 under high stringency conditions.

Nucleic acids having a sequence that differs from the nucleotide sequences shown in one of SEQ ID Nos: 1 or 3 due to degeneracy in the genetic code are also within the scope of the invention. Such nucleic acids encode functionally equivalent peptides (i.e., a peptide having a biological activity of a Delta3 polypeptide) but differ in sequence from the sequence shown in the sequence listing due to degeneracy in the genetic code. For example, a number of amino acids are designated by more than one triplet. Codons that specify the same amino acid, or synonyms (for example, CAU and CAC each encode histidine) may result in "silent" mutations which do not affect the amino acid sequence of a Delta3 polypeptide. However, it is expected that DNA sequence polymorphisms that do lead to changes in the amino acid sequences of the subject Delta3 polypeptides will exist. One skilled in the art will appreciate that these variations in one or more nucleotides (e.g., up to about 3-5% of the nucleotides) of the nucleic acids encoding polypeptides having an activity of a Delta3 polypeptide may exist among individuals of a given species due to natural allelic variation.

As indicated by the examples set out below, Delta3 protein-encoding nucleic acids can be obtained from mRNA present in any of a number of eukaryotic cells. It should also be possible to obtain nucleic acids encoding Delta3 polypeptides of the present invention from genomic DNA from both adults and embryos. For example, a gene encoding a Delta3 protein can be cloned from either a cDNA or a genomic library in accordance with protocols described herein, as well as those generally known to persons skilled in the art. Examples of tissues and/or libraries suitable for isolation of the subject nucleic acids include endothelial cell libraries, among others. A cDNA encoding a Delta3 protein can be obtained by isolating total mRNA from a cell, e.g. a vertebrate cell, a mammalian cell, or a human cell, including embryonic cells. Double stranded cDNAs can then be prepared from the total mRNA, and subsequently inserted into a suitable plasmid or bacteriophage vector using any one of a number of known techniques. The gene encoding a Delta3 protein can also be cloned using established polymerase chain reaction techniques in accordance with the nucleotide sequence information provided by the invention. The nucleic acid of the invention can be DNA or RNA. A preferred nucleic acid is a cDNA represented by a sequence selected from the group consisting of SEQ ID Nos:1 and 3.

### 4.3.1. Vectors.

This invention also provides expression vectors containing a nucleic acid encoding a Delta3 polypeptide, operably linked to at least one transcriptional regulatory sequence. "Operably linked" is intended to mean that the nucleotide sequence is linked to a regulatory sequence in a manner which allows expression of the nucleotide sequence. Regulatory sequences are art-recognized and are selected to direct expression of the subject Delta3 proteins. Accordingly, the term "transcriptional regulatory sequence" includes promoters, enhancers and other expression control elements. Such regulatory sequences are described in Goeddel; Gene Expression Technology: Methods in Enzymology 185, Academic Press, San Diego, CA (1990). In one embodiment, the expression vector includes a recombinant gene encoding a peptide having an agonistic activity of a subject Delta3 polypeptide, or alternatively, encoding a peptide which is an antagonistic form of the Delta3 protein. Such expression vectors can be used to transfect cells and thereby produce polypeptides, including fusion proteins, encoded by nucleic acids as described herein. Moreover, the gene constructs of the present invention can also be used as a part of a gene therapy protocol to deliver nucleic acids encoding either an agonistic or antagonistic form of one of the subject Delta3 proteins. Thus, another aspect of the invention features expression vectors for *in vivo* or *in vitro* transfection and expression of a Delta3 polypeptide in particular cell types so as to reconstitute the function of, or alternatively, abrogate the function of Delta-induced signaling in a tissue. This could be desirable, for example, when the naturally-occurring form of the protein is misexpressed; or to deliver a form of the protein which alters differentiation of tissue. Expression vectors may also be employed to inhibit neoplastic transformation.

In addition to viral transfer methods, such as those illustrated above, non-viral methods can also be employed to cause expression of a subject Delta3 polypeptide in the tissue of an animal. Most nonviral methods of gene transfer rely on normal mechanisms used by mammalian cells for the uptake and intracellular transport of macromolecules. In preferred embodiments, non-viral targeting means of the present invention rely on endocytic pathways for the uptake of the subject Delta3 polypeptide gene by the targeted cell. Exemplary targeting means of this type include liposomal derived systems, poly-lysine conjugates, and artificial viral envelopes.

### 4.3.2. Probes and Primers

Moreover, the nucleotide sequences determined from the cloning of hDelta3 genes will further allow for the generation of probes and primers designed for use in identifying and/or cloning Delta3 homologs in other cell types, e.g. from other tissues, as well as Delta3 homologs from other mammalian organisms. Probes and primers can also be used to determine the identity of a Delta3 allele and/or the presence or absence of one or more mutations in a Delta3 gene of a subject. A probe or primer can be used to determine whether a subject has or is at risk of developing a disease or condition associated with a specific Delta3 allele, such as an allele carrying a mutation.

The disclosure also describes a probe/primer comprising a substantially purified oligonucleotide, which oligonucleotide comprises a region of nucleotide sequence that hybridizes under stringent conditions to at least about 12, preferably about 25, more preferably about 40, 50 or 75 consecutive nucleotides of sense or anti-sense sequence selected from the group consisting of SEQ ID No: 1 and 3, or naturally occurring mutants thereof For instance, primers based on the nucleic acid represented in SEQ ID Nos: 1 and 3 can be used in PCR reactions to clone Delta3 homologs, e.g. specific Delta3 alleles. Such primers are preferably selected in a region which does not share significant homology to other genes, e.g., other Delta genes. Primers are set forth as SEQ ID Nos. 4-8 set forth below:

| | |
|---|---|
| 5' end primers: | |
| 5'AGCGCCTCTGGCTGGGCGCT3' | (SEQ ID No. 12; corresponding to nucleotides 356 to 375 of SEQ ID No. 1); |
| 5' CGGCCAGAGGCCTTGCCACC 3' | (SEQ ID No. 13; corresponding to nucleotides 725 to 744 of SEQ ID No. 1); |
| 3' end primers | |
| 5' TTGCGCTCCCGGCTGGAGCC 3' 1479 of | (SEQ ID No. 14; corresponding to the complement of nucleotides 1460 to SEQ ID No. 1); and |
| 5' ATGCGGCTTGGACCTCGGTT 3' 2611 of | (SEQ ID No. 15; corresponding to the complement of nucleotides 1592 to SEQ ID No. 1). |

Likewise, probes based on the subject Delta3 sequences can be used to detect transcripts or genomic sequences encoding the same or homologous proteins. The probe may further comprise a label group attached thereto and able to be detected, e.g. the label group is selected from amongst radioisotopes, fluorescent compounds, enzymes, and enzyme co-factors.

As discussed in more detail below, such probes can also be used as a part of a diagnostic test kit for identifying cells or tissue which misexpress a Delta3 protein, such as by measuring a level of a Delta-encoding nucleic acid in a sample of cells from a patient; e.g. detecting Delta3 mRNA levels or determining whether a genomic Delta3 gene has been mutated or deleted. Briefly, nucleotide probes can be generated from the subject Delta3 genes which facilitate histological screening of intact tissue and tissue samples for the presence (or absence) of Delta-encoding transcripts. Similar to the diagnostic uses of anti-Delta3 antibodies, the use of probes directed to Delta3 messages, or to genomic Delta3 sequences, can be used for both predictive and therapeutic evaluation of allelic mutations which might be manifest in, for example, neoplastic or hyperplastic disorders (e.g. unwanted cell growth) or abnormal differentiation of tissue. Used in conjunction with immunoassays as described herein, the oligonucleotide probes can help facilitate the determination of the molecular basis for a developmental disorder which may involve some abnormality associated with expression (or lack thereof) of a Delta3 protein. For instance, variation in polypeptide synthesis can be differentiated from a mutation in a coding sequence.

Also described are kits for determining whether a subject is at risk of developing a disease or condition caused by or contributed by an aberrant Delta3 bioactivity and/or which is associated with one or more specific Delta3 alleles. In a preferred embodiment, the kit can be used for determining whether a subject is at risk of developing a neurological disease or disorder, e.g., a peripheral neuropathy, e.g., ACCPN.

### 4.3.3. Antisense, Ribozyme and Triplex technique

One part of the disclosure relates to the use of the isolated nucleic acid in "antisense" therapy. As used herein, "antisense" therapy refers to administration or in situ generation of oligonucleotide molecules or their derivatives which specifically hybridize (e.g. bind) under cellular conditions, with the cellular mRNA and/or genomic DNA encoding one or more of the subject Delta3 proteins so as to inhibit expression of that protein, e.g. by inhibiting transcription and/or translation. The binding may be by conventional base pair complementarity, or, for example, in the case of binding to DNA duplexes, through specific interactions in the major groove of the double helix. In general, "antisense" therapy refers to the range of techniques generally employed in the art, and includes any therapy which relies on specific binding to oligonucleotide sequences.

An antisense construct can be delivered, for example, as an expression plasmid which, when transcribed in the cell, produces RNA which is complementary to at least a unique portion of the cellular mRNA which encodes a Delta3 protein. Alternatively, the antisense construct is an oligonucleotide probe which is generated ex vivo and which, when introduced into the cell causes inhibition of expression by hybridizing with the mRNA and/or genomic sequences of a Delta3 gene. Such oligonucleotide probes are preferably modified oligonucleotides which are resistant to endogenous nucleases, e.g. exonucleases and/or endonucleases, and are therefore stable *in vivo.* Exemplary nucleic acid molecules for use as antisense oligonucleotides are phosphoramidate, phosphothioate and methylphosphonate analogs of DNA (see also U.S. Patents 5,176,996; 5,264,564; and 5,256,775). Additionally, general approaches to constructing oligomers useful in antisense therapy have been reviewed, for example, by Van der Krol et al. (1988) Biotechniques 6:958-976; and Stein et al. (1988) Cancer Res 48:2659-2668. With respect to antisense DNA, oligodeoxyribonucleotides derived from the translation initiation site, e.g., between the -10 and +10 regions of the Delta3 nucleotide sequence of interest, are preferred. Particular antisense molecules are shown below:
5' TGCCGCCATCCCTCGGGGCGT 3' (SEQ ID NO.16)
   (complement to nucleotides 326-346 of SEQ ID NO.1)
5' GGACGCTGCCGCCATCCCCT 3' (SEQ ID NO.17)
   (complement to nucleotides 333-352 of SEQ ID NO.1)
5' GGACGCTGCCGCCATCCCCTCGGGGCGT 3' (SEQ ID NO18)
   (complement to nucleotides 326-352 of SEQ ID NO.1)

Antisense approaches involve the design of oligonucleotides (either DNA or RNA) that are complementary to Delta3 mRNA. The antisense oligonucleotides will bind to the Delta3 mRNA transcripts and prevent translation. Absolute complementarity, although preferred, is not required. A sequence "complementary" to a portion of an RNA, as referred to herein, means a sequence having sufficient complementarity to be able to hybridize with the RNA, forming a stable duplex; in the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

Oligonucleotides that are complementary to the 5' end of the message, e.g., the 5' untranslated sequence up to and including the AUG initiation codon, should work most efficiently at inhibiting translation. However, sequences complementary to the 3' untranslated sequences of mRNAs have recently been shown to be effective at inhibiting translation of mRNAs as well. (Wagner, R. 1994. Nature 372:333). Therefore, oligonucleotides complementary to either the 5' or 3' untranslated, non-coding regions of a Delta3 gene could be used in an antisense approach to inhibit translation of endogenous Delta3 mRNA. Oligonucleotides complementary to the 5' untranslated region of the mRNA should include the complement of the AUG start codon. Antisense oligonucleotides complementary to mRNA coding regions are less efficient inhibitors of translation but could be used in accordance with the invention. Whether designed to hybridize to the 5', 3' or coding region of Delta3 mRNA, antisense nucleic acids should be at least six nucleotides in length, and are preferably oligonucleotides ranging from 6 to about 50 nucleotides in length. In certain embodiments, the oligonucleotide is at least 10 nucleotides, at least 17 nucleotides, at least 25 nucleotides, or at least 50 nucleotides.

Regardless of the choice of target sequence, it is preferred that *in vitro* studies are first performed to quantitate the ability of the antisense oligonucleotide to quantitate the ability of the antisense oligonucleotide to inhibit gene expression. It is preferred that these studies utilize controls that distinguish between antisense gene inhibition and nonspecific biological effects of oligonucleotides. It is also preferred that these studies compare levels of the target RNA or protein with that of an internal control RNA or protein. Additionally, it is envisioned that results obtained using the antisense oligonucleotide are compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridization to the target sequence.

The oligonucleotides can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides (e.g.. for targeting host cell receptors *in vivo*), or agents facilitating transport across the cell membrane (see, e.g., Letsinger et al., 1989, Proc. Natl. Acad. Sci. U.S.A 86:6553-6556; Lemaitre et al., 1987, Proc. Natl. Acad. Sci. 84:648-652; PCT Publication No. WO 88/09810, published December 15, 1988) or the blood-brain barrier (see, e.g., PCT Publication No. WO 89/10134, published April 25, 1988), hybridization-triggered cleavage agents. (See, e.g., Krol et al., 1988, BioTechniques 6:958-976) or intercalating agents. (See, e.g., Zon, 1988, Pharm. Res. 5:539-549). To this end, the oligonucleotide may be conjugated to another molecule, e.g., a peptide, hybridization triggered crosslinking agent, transport agent, hybridization-triggered cleavage agent, etc.

The antisense oligonucleotide may comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including but not limited to arabinose, 2-fluoroarabinose, xylulose, and hexose.

In yet another embodiment, the antisense oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier et al., 1987, Nucl. Acids Res. 15:6625-6641). The oligonucleotide is a 2'-0-methylribonucleotide (Inoue et al., 1987, Nucl. Acids Res. 15:6131-6148), or a chimeric RNA-DNA analogue (Inoue et al., 1987, FEBS Lett. 215:327-330).

Oligonucleotides may be synthesized by standard methods known in the art, e.g. by use of an automated DNA synthesizer (such as are commercially available from Biosearch, Applied Biosystems, etc.). As examples, phosphorothioate oligonucleotides may be synthesized by the method of Stein et al. (1988, Nucl. Acids Res. 16:3209), methylphosphonate oligonucleotides can be prepared by use of controlled pore glass polymer supports (Sarin et al., 1988, Proc. Natl. Acad. Sci. U.S.A. 85:7448-7451), etc.

While antisense nucleic acids complementary to the coding region sequence could be used, those complementary to the transcribed untranslated region are preferred. Antisense nucleic acids overlapping the site of initiation of translation are even more preferred. For example, antisense oligonucleotides as set forth below can be utilized in accordance with the disclosure.
5' TCAATCTGGCTCTGTTCGCG 3' (SEQ ID NO.19)
   (complement to nucleotides 284-3030 of SEQ ID NO.1)
5' CGCTCTCTCCACCCGCGGGCCCTCAA 3' (SEQ ID NO.20)
   (complement to nucleotides 300-325 of SEQ ID NO.1)

The antisense molecules should be delivered to cells which express the Delta3 *in vivo.* A number of methods have been developed for delivering antisense DNA or RNA to cells; eg., antisense molecules can be injected directly into the tissue site, or modified antisense molecules, designed to target the desired cells (e.g., antisense linked to peptides or antibodies that specifically bind receptors or antigens expressed on the target cell surface) can be administered systematically.

However, it is often difficult to achieve intracellular concentrations of the antisense sufficient to suppress translation on endogenous mRNAs. Therefore a preferred approach utilizes a recombinant DNA construct in which the antisense oligonucleotide is placed under the control of a strong pol III or pol II promoter. The use of such a construct to transfect target cells in the patient will result in the transcription of sufficient amounts of single stranded RNAs that will form complementary base pairs with the endogenous Delta3 transcripts and thereby prevent translation of the Delta3 mRNA. For example, a vector can be introduced *in vivo* such that it is taken up by a cell and directs the transcription of an antisense RNA. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in mammalian cells. Expression of the sequence encoding the antisense RNA can be by any promoter known in the art to act in mammalian, preferably human cells. Such promoters can be inducible or constitutive. Such promoters include but are not limited to: the SV40 early promoter region (Bernoist and Chambon, 1981, Nature 290:304-310), the promoter contained in the 3' long terminal repeat ofRous sarcoma virus (Yamamoto et al., 1980, Cell 22:787-797), the herpes thymidine kinase promoter (Wagner et al., 1981., Proc Natl Acad. Sci U.S.A. 78:4441-1445), the regulatory sequences of the metallothionein gene (Brinster et al, 1982, Nature 296:39-42), etc. Any type of plasmid, cosmid, YAC or viral vector can be used to prepare the recombinant DNA construct which can be introduced directly into the tissue site; eg., the choroid plexus or hypothalamus. Alternatively, viral vectors can be used which selectively infect the desired tissue; (eg., for brain, herpesvirus vectors may be used), in which case administration may be accomplished by another route (e.g., systematically).

Likewise, the antisense constructs, by antagonizing the normal biological activity of one of the Delta3 proteins, can be used in the modulation of cellular activity both *in vivo* and for ex vivo tissue cultures.

Furthermore, the anti-sense techniques (e.g. microinjection of antisense molecules, or transfection with plasmids whose transcripts are anti-sense with regard to a Delta3 mRNA or gene sequence) can be used to investigate the role of Delta3 in developmental events, as well as the normal cellular function of Delta3 in adult tissue. Such techniques can be utilized in cell culture.

Ribozyme molecules designed to catalytically cleave Delta3 mRNA transcripts can also be used to prevent translation of mRNA and expression of Delta3. (See, e.g., PCT International Publication WO 94/11364, published October 4, 1990; Sarver et al., 1990, Science 247:1222-1225). Ribozymes are enzymatic RNA molecules capable of catalyzing the specific cleavage of RNA. The mechanism of ribozyme action involves sequence specific hybridization of the ribozyme molecule to complementary target RNA, followed by an endonucleolytic cleavage. The composition of ribozyme molecules must include one or more sequences complementary to the target gene mRNA, and must include the well known catalytic sequence responsible for mRNA cleavage. For this sequence, see U.S. Pat. No. 5,093,246, which is incorporated by reference herein in its entirety. As such within the scope of the invention are engineered hammerhead motif ribozyme molecules that specifically and efficiently catalyze endonucleolytic cleavage of RNA sequences encoding Delta3 proteins.

Specific ribozyme cleavage sites within any potential RNA target are initially identified by scanning the molecule of interest for ribozyme cleavage sites which include the following sequences, GUA, GUU and GUC. Once identified, short RNA sequences of between 15 and 20 ribonucleotides corresponding to the region of the target gene containing the cleavage site may be evaluated for predicted structural features, such as secondary structure, that may render the oligonucleotide sequence unsuitable. The suitability of candidate sequences may also be evaluated by testing their accessibility to hybridization with complementary oligonucleotides, using ribonuclease protection assays.

While ribozymes that cleave mRNA at site specific recognition sequences can be used to destroy Delta3 mRNAs, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach, 1988, Nature, 334:585-591. There are hundreds of potential hammerhead ribozyme cleavage sites within the nucleotide sequence of human Delta3 cDNA (Fig. 1). Preferably the ribozyme is engineered so that the cleavage recognition site is located near the 5' end of the Delta3 mRNA; i.e., to increase efficiency and minimize the intracellular accumulation of non-functional mRNA transcripts.

The ribozymes also include RNA endoribonucleases (hereinafter "Cech-type ribozymes") such as the one which occurs naturally in Tetrahymena Thermophila (known as the IVS, or L-19 IVS RNA) and which has been extensively described by Thomas Cech and collaborators (Zaug, et al., 1984, Science, 224:574-578; Zaug and Cech, 1986, Science, 231:470-475; Zaug, et al., 1986, Nature, 324:429-433; published International patent application No. WO88/04300 by University Patents Inc.; Been and Cech, 1986, Cell, 47:207-216). The Cech-type ribozymes have an eight base pair active site which hybridizes to a target RNA sequence whereafter cleavage of the target RNA takes place. The disclosure encompasses those Cech-type ribozymes which target eight base-pair active site sequences that are present in Delta3.

As in the antisense approach, the ribozymes can be composed of modified oligonucleotides (e.g. for improved stability, targeting, etc.) and should be delivered to cells which express the Delta3 *in vivo* e.g., hypothalamus and/or the choroid plexus. A preferred method of delivery involves using a DNA construct "encoding" the ribozyme under the control of a strong constitutive pol III or pol II promoter, so that transfected cells will produce sufficient quantities of the ribozyme to destroy endogenous Delta3 messages and inhibit translation. Because ribozymes unlike antisense molecules, are catalytic, a lower intracellular concentration is required for efficiency.

Endogenous Delta3 gene expression can also be reduced by inactivating or "knocking out" the Delta3 gene or its promoter using targeted homologous recombination. (E.g., see Smithies et al., 1985, Nature 317:230-234; Thomas & Capecchi, 1987, Cell 51:503-512; Thompson et al., 1989 Cell 5:313-321; each of which is incorporated by reference herein in its entirety). For example, a mutant, non-functional Delta3 (or a completely unrelated DNA sequence) flanked by DNA homologous to the endogenous Delta3 gene (either the coding regions or regulatory regions of the Delta3 gene) can be used, with or without a selectable marker and/or a negative selectable marker, to transfect cells that express Delta3 *in vivo.* Insertion of the DNA construct, via targeted homologous recombination, results in inactivation of the Delta3 gene. Such approaches are particularly suited in the agricultural field where modifications to ES (embryonic stem) cells can be used to generate animal offspring with an inactive Delta3 (e.g., see Thomas & Capecchi 1987 and Thompson 1989, *supra*). However this approach can be adapted for use in humans provided the recombinant DNA constructs are directly administered or targeted to the required site *in vivo* using appropriate viral vectors, e.g., herpes virus vectors.

Alternatively endogenous Delta3 gene expression can be reduced by targeting deoxyribonucleotide sequences complementary to the regulatory region of the Delta3 gene (i.e., the Delta3 promoter and/or enhancers) to form triple helical structures that prevent transcription of the Delta3 gene in target cells in the body. (See generally, Helene, C. 1991, Anticancer Drug Des., 6(6):569-84; Helene, C., et al., 1992, Ann, N.Y. Acad. Sci., 660:27-36; and Maher, L.J., 1992, Bioassays 14(12):807-15).

Nucleic acid molecules to be used in triple helix formation for the inhibition of transcription are preferably single stranded and composed of deoxyribonucleotides. The base composition of these oligonucleotides should promote triple helix formation via Hoogsteen base pairing rules, which generally require sizable stretches of either purines or pyrimidines to be present on one strand of a duplex. Nucleotide sequences may be pyrimidine-based, which will result in TAT and CGC triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, containing a stretch of G residues. These molecules will form a triple helix with a DNA duplex that is rich in GC pairs, in which the majority of the purine residues are located on a single strand of the targeted duplex, resulting in CGC triplets across the three strands in the triplex.

Alternatively, the potential sequences that can be targeted for triple helix formation may be increased by creating a so called "switchback" nucleic acid molecule. Switchback molecules are synthesized in an alternating 5'-3', 3'-5' manner, such that they base pair with first one strand of a duplex and then the other, eliminating the necessity for a sizable stretch of either purines or pyrimidines to be present on one strand of a duplex.

Antisense RNA and DNA, ribozyme, and triple helix molecules may be prepared by any method known in the art for the synthesis of DNA and RNA molecules. These include techniques for chemically synthesizing oligodeoxyribonucleotides and oligoribonucleotides well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

Moreover, various well-known modifications to nucleic acid molecules may be introduced as a means of increasing intracellular stability and half-life. Possible modifications include but are not limited to the addition of flanking sequences of ribonucleotides or deoxyribonucleotides to the 5' and/or 3' ends of the molecule or the use of phosphorothioate or 2' O-methyl rather than phosphodiesterase linkages within the oligodeoxyribonucleotide backbone.

### 4.4 Polypeptides of the Present Invention

The present invention also makes available Delta3 polypeptides which are isolated from, or otherwise substantially free of other cellular proteins, especially other signal transduction factors and/or transcription factors which may normally be associated with the Delta3 polypeptide. The term "substantially free of other cellular proteins" (also referred to herein as "contaminating proteins") or "substantially pure or purified preparations" are defined as encompassing preparations of Delta3 polypeptides having less than about 20% (by dry weight) contaminating protein, and preferably having less than about 5% contaminating protein. Functional forms of the subject polypeptides can be prepared, for the first time, as purified preparations by using a cloned gene as described herein. By "purified", it is meant, when referring to a peptide or DNA or RNA sequence, that the indicated molecule is present in the substantial absence of other biological macromolecules, such as other proteins. The term "purified" as used herein preferably means at least 80% by dry weight, more preferably in the range of 95-99% by weight, and most preferably at least 99.8% by weight, of biological macromolecules of the same type present (but water, buffers, and other small molecules, especially molecules having a molecular weight of less than about 5000, can be present). The term "pure" as used herein preferably has the same numerical limits as "purified" immediately above. "Isolated" and "purified" do not encompass either natural materials in their native state or natural materials that have been separated into components (e.g., in an acrylamide gel) but not obtained either as pure (e.g. lacking contaminating proteins, or chromatography reagents such as denaturing agents and polymers, e.g. acrylamide or agarose) substances or solutions. In preferred embodiments, purified Delta3 preparations will lack any contaminating proteins from the same animal from which Delta3 is normally produced, as can be accomplished by recombinant expression of, for example, a human Delta3 protein in a non-human cell.

Full length proteins or fragments corresponding to one or more particular motifs and/or domains or to arbitrary sizes, for example, at least about 5, 10, 25, 50, 75, 100, 125, 150 amino acids in length are described. The disclosure encompasses all proteins encoded by the nucleic acids described in the above section describing the nucleic acids of the invention.

For example, isolated Delta3 polypeptides can include all or a portion of an amino acid sequences corresponding to a Delta3 polypeptide represented in SEQ ID No: 2. Isolated peptidyl portions of Delta3 proteins can be obtained by screening peptides recombinantly produced from the corresponding fragment of the nucleic acid encoding such peptides. In addition, fragments can be chemically synthesized using techniques known in the art such as conventional Merrifield solid phase f-Moc or t-Boc chemistry. For example, a Delta3 polypeptide of the present invention may be arbitrarily divided into fragments of desired length with no overlap of the fragments, or preferably divided into overlapping fragments of a desired length. The fragments can be produced (recombinantly or by chemical synthesis) and tested to identify those peptidyl fragments which can function as either agonists or antagonists of a wild-type (e.g., "authentic") Delta3 protein.

Another aspect of the present invention concerns recombinant forms of the Delta3 proteins. Recombinant polypeptides of the present invention, in addition to native Delta3 proteins, have an overall amino acid sequence identity of at least 95%, at least about 98%, or at least about 99% with the amino acid sequence SEQ ID No. 2. In a particularly preferred embodiment a Delta3 protein has the amino acid sequence SEQ ID No: 2. In other particularly preferred embodiments, the Delta3 protein has a Delta3 bioactivity.

The present disclosure further pertains to recombinant forms of one of the subject Delta3 polypeptides which are encoded by genes derived from a mammalian organism, and which have amino acid sequences evolutionarily related to the Delta3 protein represented in SEQ ID No: 2. Such recombinant Delta3 polypeptides preferably are capable of functioning in one of either role of an agonist or antagonist of at least one biological activity of a wild-type ("authentic") Delta3 protein of the appended sequence listing. The term "evolutionarily related to", with respect to amino acid sequences of human Delta3 proteins, refers to both polypeptides having amino acid sequences which have arisen naturally, and also to mutational variants of the Delta3 polypeptides which are derived, for example, by combinatorial mutagenesis. Such evolutionarily derived Delta3 polypeptides can have a Delta3 bioactivity and are at least 80% homologous and more preferably 85% homologous and most preferably 90% homologous with the amino acid sequence of SEQ ID No: 2. In a particularly preferred embodiment, a Delta3 protein comprises the amino acid coding sequence of SEQ ID No: 2.

In general, polypeptides referred to herein as having an activity (e.g., "bioactivity") of a Delta3 protein are defined as polypeptides which include an amino acid sequence corresponding (e.g., identical or substantially identical) to all or a portion of the amino acid sequences of a Delta3 protein shown in SEQ ID No: 2 and which mimic or antagonize all or a portion of the biological/biochemical activities of a naturally occurring Delta3 protein. In preferred embodiments a Delta3 protein of the present invention specifically interacts with a Notch polypeptide.

The disclosure provides various forms of Delta3 proteins, specifically including all of the Delta3 proteins described in the section "4.3" relating to nucleic acids of the invention.

The present disclosure further pertains to methods of producing the subject Delta3 polypeptides. For example, a host cell transfected with a nucleic acid vector directing expression of a nucleotide sequence encoding the subject polypeptides can be cultured under appropriate conditions to allow expression of the peptide to occur. The cells may be harvested, lysed and the protein isolated. A cell culture includes host cells, media and other byproducts. Suitable media for cell culture are well known in the art. The recombinant Delta3 polypeptide can be isolated from cell culture medium, host cells, or both using techniques known in the art for purifying proteins including ion-exchange chromatography, gel filtration chromatography, ultrafiltration, electrophoresis, and immunoaffinity purification with antibodies specific for such peptide. In a preferred embodiment, the recombinant Delta3 polypeptide is a fusion protein containing a domain which facilitates its purification, such as GST fusion protein or poly(His) fusion protein.

Moreover, it will be generally appreciated that, under certain circumstances, it may be advantageous to provide variants of one of the subject Delta3 polypeptides which function in a limited capacity as one of either a Delta3 agonist (mimetic) or a Delta3 antagonist, in order to promote or inhibit only a subset of the biological activities of the naturally-occurring form of the protein. Thus, specific biological effects can be elicited by treatment with a variant having a limited function, and with fewer side effects relative to treatment with agonists or antagonists which are directed to all of the biological activities of naturally occurring forms of Delta3 proteins.

Variants and/or mutants of each of the subject Delta3 proteins can be generated by mutagenesis, such as by discrete point mutation(s), or by truncation. For instance, mutation can give rise to homologs which retain substantially the same, or merely a subset, of the biological activity of the Delta3 polypeptide from which it was derived. Alternatively, antagonistic forms of the protein can be generated which are able to inhibit the function of the naturally occurring form of the protein, such as by competitively binding to a downstream or upstream member of the Delta3 cascade which includes the Delta3 protein. In addition, agonistic forms of the protein may be generated which are constitutively active.

The recombinant Delta3 polypeptides of the present invention also include homologs of the authentic Delta3 proteins, such as versions of those protein which are resistant to proteolytic cleavage, as for example, due to mutations which alter ubiquitination or other enzymatic targeting associated with the protein.

Delta3 polypeptides may also be chemically modified to create Delta3 derivatives by forming covalent or aggregate conjugates with other chemical moieties, such as glycosyl groups, lipids, phosphate, acetyl groups and the like. Covalent derivatives of Delta3 proteins can be prepared by linking the chemical moieties to functional groups on amino acid sidechains of the protein or at the N-terminus or at the C-terminus of the polypeptide.

Modification of the structure of the subject Delta3 polypeptides can be for such purposes as enhancing therapeutic or prophylactic efficacy, stability (e.g., ex vivo shelf life and resistance to proteolytic degradation *in vivo*)*,* or post-translational modifications (e.g., to alter phosphorylation pattern of protein). Such modified peptides, when designed to retain at least one activity of the naturally-occurring form of the protein, or to produce specific antagonists thereof, are considered functional equivalents of the Delta3 polypeptides described in more detail herein. Such modified peptides can be produced, for instance, by amino acid substitution, deletion, or addition.

For example, it is reasonable to expect that an isolated replacement of a leucine with an isoleucine or valine, an aspartate with a glutamate, a threonine with a serine, or a similar replacement of an amino acid with a structurally related amino acid (i.e. isosteric and/or isoelectric mutations) will not have a major effect on the biological activity of the resulting molecule. Conservative replacements are those that take place within a family of amino acids that are related in their side chains. Genetically encoded amino acids are can be divided into four families: (1) acidic = aspartate, glutamate; (2) basic = lysine, arginine, histidine; (3) nonpolar = alanine, valine, leucine, isoleucine, proline, phenylalanine, methionine, tryptophan; and (4) uncharged polar = glycine, asparagine, glutamine, cysteine, serine, threonine, tyrosine. In similar fashion, the amino acid repertoire can be grouped as (1) acidic = aspartate, glutamate; (2) basic = lysine, arginine histidine, (3) aliphatic = glycine, alanine, valine, leucine, isoleucine, serine, threonine, with serine and threonine optionally be grouped separately as aliphatic-hydroxyl; (4) aromatic = phenylalanine, tyrosine, tryptophan; (5) amide = asparagine, glutamine; and (6) sulfur -containing = cysteine and methionine. (see, for example, Biochemistry, 2nd ed., Ed. by L. Stryer, WH Freeman and Co.: 1981). Whether a change in the amino acid sequence of a peptide results in a functional Delta3 homolog (e.g. functional in the sense that the resulting polypeptide mimics or antagonizes the wild-type form) can be readily determined by assessing the ability of the variant peptide to produce a response in cells in a fashion similar to the wild-type protein, or competitively inhibit such a response. Polypeptides in which more than one replacement has taken place can readily be tested in the same manner.

This disclosure further contemplates a method for generating sets of combinatorial mutants of the subject Delta3 proteins as well as truncation mutants, and is especially useful for identifying potential functional variant sequences (e.g. homologs). The purpose of screening such combinatorial libraries is to generate, for example, novel Delta3 homologs which can act as either agonists or antagonist, or alternatively, possess novel activities all together.

In one embodiment, the variegated library of Delta3 variants is generated by combinatorial mutagenesis at the nucleic acid level, and is encoded by a variegated gene library. For instance, a mixture of synthetic oligonucleotides can be enzymatically ligated into gene sequences such that the degenerate set of potential Delta3 sequences are expressible as individual polypeptides, or alternatively, as a set of larger fusion proteins (e.g. for phage display) containing the set of Delta3 sequences therein.

There are many ways by which such libraries of potential Delta3 homologs or variants can be generated from a degenerate oligonucleotide sequence. Chemical synthesis of a degenerate gene sequence can be carried out in an automatic DNA synthesizer, and the synthetic genes then ligated into an appropriate expression vector. The purpose of a degenerate set of genes is to provide, in one mixture, all of the sequences encoding the desired set of potential Delta3 sequences. The synthesis of degenerate oligonucleotides is well known in the art (see for example, Narang, SA (1983) Tetrahedron 39:3; Itakura et al. (1981) Recombinant DNA, Proc 3rd Cleveland Sympos. Macromolecules, ed. AG Walton, Amsterdam: Elsevier ppg. 273-289; Itakura et al. (1984) Annu. Rev. Biochem. 53:323; Itakura et al. (1984) Science 198:1056; Ike et al. (1983) Nucleic Acid Res. 11:477. Such techniques have been employed in the directed evolution of other proteins (see, for example, Scott et al. (1990) Science 249:386-390; Roberts et al. (1992) PNAS 89:2429-2433; Devlin et al. (1990) Science 249: 404-406; Cwirla et al. (1990) PNAS 87: 6378-6382; as well as U.S. Patents Nos. 5,223,409, 5,198,346, and 5,096,815).

Likewise, a library of coding sequence fragments can be provided for a Delta3 clone in order to generate a variegated population of Delta3 fragments for screening and subsequent selection of bioactive fragments. A variety of techniques are known in the art for generating such libraries, including chemical synthesis. In one embodiment, a library of coding sequence fragments can be generated by (i) treating a double stranded PCR fragment of a Delta3 coding sequence with a nuclease under conditions wherein nicking occurs only about once per molecule; (ii) denaturing the double stranded DNA; (iii) renaturing the DNA to form double stranded DNA which can include sense/antisense pairs from different nicked products; (iv) removing single stranded portions from reformed duplexes by treatment with S 1 nuclease; and (v) ligating the resulting fragment library into an expression vector. By this exemplary method, an expression library can be derived which codes for N-terminal, C-terminal and internal fragments of various sizes.

A wide range of techniques are known in the art for screening gene products of combinatorial libraries made by point mutations or truncation, and for screening cDNA libraries for gene products having a certain property. Such techniques will be generally adaptable for rapid screening of the gene libraries generated by the combinatorial mutagenesis of Delta3 homologs. The most widely used techniques for screening large gene libraries typically comprises cloning the gene library into replicable expression vectors, transforming appropriate cells with the resulting library of vectors, and expressing the combinatorial genes under conditions in which detection of a desired activity facilitates relatively easy isolation of the vector encoding the gene whose product was detected. Each of the illustrative assays described below are amenable to high through-put analysis as necessary to screen large numbers of degenerate Delta3 sequences created by combinatorial mutagenesis techniques. Combinatorial mutagenesis has a potential to generate very large libraries of mutant proteins, e.g., in the order of 10²⁶ molecules. Combinatorial libraries of this size may be technically challenging to screen even with high throughput screening assays. To overcome this problem, a new technique has been developed recently, recrusive ensemble mutagenesis (REM), which allows one to avoid the very high proportion of non-functional proteins in a random library and simply enhances the frequency of functional proteins, thus decreasing the complexity required to achieve a useful sampling of sequence space. REM is an algorithm which enhances the frequency of functional mutants in a library when an appropriate selection or screening method is employed (Arkin and Yourvan, 1992, PNAS USA 89:7811-7815; Yourvan et al., 1992, Parallel Problem Solving from Nature, 2., In Maenner and Manderick, eds., Elsevir Publishing Co., Amsterdam, pp. 401-410; Delgrave et al., 1993, Protein Engineering 6(3):327-331).

The disclosure also provides for reduction of the Delta3 proteins to generate mimetics, e.g. peptide or non-peptide agents, which are able to bind to a Delta3 protein and/or to disrupt binding of a Delta3 polypeptide of the present invention with either upstream or downstream components of a Delta/Notch signaling cascade, such as binding proteins or interactors. Thus, such mutagenic techniques as described above are also useful to map the determinants of the Delta3 proteins which participate in protein-protein interactions involved in, for example, binding of the subject Delta3 polypeptide to proteins which may function upstream (including both activators and repressors of its activity) or to proteins or nucleic acids which may function downstream of the Delta3 polypeptide, whether they are positively or negatively regulated by it, for example, Notch. To illustrate, the critical residues of a subject Delta3 polypeptide which are involved in molecular recognition of, for example, the Notch gene product or other component upstream or downstream of a Delta3 gene can be determined and used to generate Delta-derived peptidomimetics which competitively inhibit binding of the authentic Delta3 protein with that moiety. By employing, for example, scanning mutagenesis to map the amino acid residues of each of the subject Delta3 proteins which are involved in binding other extracellular proteins, peptidomimetic compounds can be generated which mimic those residues of the Delta3 protein which facilitate the interaction. Such mimetics may then be used to interfere with the normal function of a Delta3 protein. For instance, non-hydrolyzable peptide analogs of such residues can be generated using benzodiazepine (e.g., see Freidinger et al. in Peptides: Chemistry and Biology, G.R Marshall ed., ESCOM Publisher: Leiden, Netherlands, 1988), azepine (e.g., see Huffman et al. in Peptides: Chemistry and Biology, G.R. Marshall ed., ESCOM Publisher: Leiden, Netherlands, 1988), substituted gamma lactam rings (Garvey et al. in Peptides: Chemistry and Biology, G.R. Marshall ed., ESCOM Publisher Leiden, Netherlands, 1988), keto-methylene pseudopeptides (Ewenson et al. (1986) J Med Chem 29:295; and Ewenson et al. in Peptides: Structure and Function (Proceedings of the 9th American Peptide Symposium) Pierce Chemical Co. Rockland, IL, 1985), b-turn dipeptide cores (Nagai et aL (1985) Tetrahedron Lett 26:647; and Sato et al. (1986) J Chem Soc Perkin Trans 1:1231), and b-aminoalcohols (Gordon et al. (1985) Biochem Biophys Res Commun126;419; and Dann et al. (1986) Biochem Biophys Res Commun 134:71).

### 4.4.1. Cells expressing recombinant Delta3 polypeptides.

This invention also pertains to a host cell transfected to express a recombinant form of the subject Delta3 polypeptides. The host cell may be any prokaryotic or eukaryotic cell. Thus, a nucleotide sequence derived from the cloning ofDelta3 proteins, encoding all or a selected portion of the full-length protein can be used to produce a recombinant form of a Delta3 polypeptide via microbial or eukaryotic cellular processes. Ligating the polynucleotide sequence into a gene construct, such as an expression vector, and transforming or transfecting into hosts, either eukaryotic (yeast, avian, insect or mammalian) or prokaryotic (bacterial) cells, are standard procedures used in producing other well-known proteins, e.g. MAP kinase, pg. 53, WT1, PTP phosphotases, SRC, and the like. Similar procedures, or modifications thereof, can be employed to prepare recombinant Delta3 polypeptides by microbial means or tissue-culture technology in accord with the subject invention.

The recombinant Delta3 genes can be produced by ligating a nucleic acid encoding a Delta3 protein, or a portion thereof, into a vector suitable for expression in either prokaryotic cells, eukaryotic cells, or both. Expression vectors for production of recombinant forms of the subject Delta3 polypeptides include plasmids and other vectors. For instance, suitable vectors for the expression of a Delta3 polypeptide include plasmids of the types: pBR322-derived plasmids, pEMBL-derived plasmids, pEX-derived plasmids, pBTac-derivcd plasmids and pUC-derived plasmids for expression in prokaryotic cells, such as E. coli.

A number of vectors exist for the expression of recombinant proteins in yeast. For instance, YEP24, YIP5, YEP51, YEP52, pYES2, and YRP17 are cloning and expression vehicles useful in the introduction of genetic constructs into S. cerevisiae (see, for example, Broach et al. (1983) in Experimental Manipulation of Gene Expression, ed. M. Inouye Academic Press, p. 83, incorporated by reference herein). These vectors can replicate in E. coli due the presence of the pBR322 ori, and in S. cerevisiae due to the replication determinant of the yeast 2 micron plasmid. In addition, drug resistance markers such as ampicillin can be used. In an illustrative embodiment, a Delta3 polypeptide is produced recombinantly utilizing an expression vector generated by sub-cloning the Delta3 gene represented in SEQ ID No:1.

The preferred mammalian expression vectors contain both prokaryotic sequences, to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papillomavirus (BPV-1), or Epstein-Barr virus (pHEBo, pREP-derived and p205) can be used for transient expression of proteins in eukaryotic cells. The various methods employed in the preparation of the plasmids and transformation of host organisms are well known in the art. For other suitable expression systems for both prokaryotic and eukaryotic cells, as well as general recombinant procedures, see Molecular Cloning A Laboratory Manual, 2nd Ed., ed. by Sambrook, Fritsch and Maniatis (Cold Spring Harbor Laboratory Press: 1989) Chapters 16 and 17.

In some instances, it may be desirable to express the recombinant Delta3 polypeptide by the use of a baculovirus expression system. Examples of such baculovirus expression systems include pVL-derived vectors (such as pVL1392, pVL1393 and pVL941), pAcUW-derived vectors (such as pAcUW1 and pBIueBac-derived vectors (such as the β-gal containing pBlueBac III).

When it is desirable to express only a portion of a Delta3 protein, such as a form lacking a portion of the N-terminus, i.e. a truncation mutant which lacks the signal peptide, it may be necessary to add a start codon (ATG) to the oligonucleotide fragment containing the desired sequence to be expressed. It is well known in the art that a methionine at the N-terminal position can be enzymatically cleaved by the use of the enzyme methionine aminopeptidase (MAP). MAP has been cloned from E. coli (Ben-Bassat et al. (1987) J. Bacteriol. 169:751-757) and Salmonella typhimurium and its *in vitro* activity has been demonstrated on recombinant proteins (Miller et al. (1987) PNAS 84:2718-1722). Therefore, removal of an N-terminal methionine, if desired, can be achieved either *in vivo* by expressing Delta-derived polypeptides in a host which produces MAP (e.g., E. coli or CM89 or S. cerevisiae), or *in vitro* by use of purified MAP (e.g., procedure of Miller et al., *supra*).

In other embodiments transgenic animals, described in more detail below could be used to produce recombinant proteins.

### 4.4.2 Fusion proteins and Immunogens.

In another part of the disclosure, the coding sequences for the polypeptide can be incorporated as a part of a fusion gene including a nucleotide sequence encoding a different polypeptide.

A Delta3-Ig polypeptide is described. The Delta3-Ig polypeptide can comprise the entire extracellular domain of Delta3, e.g, human Delta3, or a variant thereof. For example, a Delta3-Ig polypeptide can comprise an amino acid sequences from about amino acid 1 to about amino acid 529 of SEQ ID No. 2. Other Delta3-Ig proteins do not comprise a signal peptide and thus, preferably do not comprise about amino acid 1 to about amino acid 17 of SEQ ID No. 2. Alternatively, a Delta3-Ig fusion protein can comprise a portion of the extracellular domain of a Delta3 protein or a variant of a portion of the extracellular domain of a Delta3 protein. Preferred portions of the extracellular domain include portions having at least one motif shown in Figure 2. For example a Delta3-Ig fusion protein can comprise at least one EGF-like repeat. A Delta3-Ig fusion protein can further comprise a DSL domain. A Delta3-Ig fustion protein can also further comprise a signal peptide. Delta3-Ig fusion proteins can be prepared as described e.g., in U.S. Patent No.5,434,131.

This type of expression system can be useful under conditions where it is desirable to produce an immunogenic fragment of a Delta3 protein. For example, the VP6 capsid protein of rotavirus can be used as an immunologic carrier protein for portions of the Delta3 polypeptide, either in the monomeric form or in the form of a viral particle. The nucleic acid sequences corresponding to the portion of a subject Delta3 protein to which antibodies are to be raised can be incorporated into a fusion gene construct which includes coding sequences for a late vaccinia virus structural protein to produce a set of recombinant viruses expressing fusion proteins comprising Delta3 epitopes as part of the virion. It has been demonstrated with the use of immunogenic fusion proteins utilizing the Hepatitis B surface antigen fusion proteins that recombinant Hepatitis B virions can be utilized in this role as well. Similarly, chimeric constructs coding for fusion proteins containing a portion of a Delta3 protein and the poliovirus capsid protein can be created to enhance immunogenicity of the set of polypeptide antigens (see, for example, EP Publication No: 0259149; and Evans et al. (1989) Nature 339:385; Huang et al. (1988) J. Virol. 62:3855; and Schlienger et al. (1992) J. Virol. 66:2).

The Multiple Antigen Peptide system for peptide-based immunization can also be utilized to generate an immunogen, wherein a desired portion of a Delta3 polypeptide is obtained directly from organo-chemical synthesis of the peptide onto an oligomeric branching lysine core (see, for example, Posnett et al. (1988) JBC 263:1719 and Nardelli et al. (1992) J. Immunol. 148:914). Antigenic determinants of Delta3 proteins can also be expressed and presented by bacterial cells.

In addition to utilizing fusion proteins to enhance immunogenicity, it is widely appreciated that fusion proteins can also facilitate the expression of proteins, and accordingly, can be used in the expression of the Delta3 polypeptides of the present invention. For example, Delta3 polypeptides can be generated as glutathione-S-transferase (GST-fusion) proteins. Such GST-fusion proteins can enable easy purification of the Delta3 polypeptide, as for example by the use of glutathione-derivatized matrices (see, for example, Current Protocols in Molecular Biology, eds. Ausubel et al. (N.Y.: John Wiley & Sons, 1991)).

In another embodiment, a fusion gene coding for a purification leader sequence, such as a poly-(His)/enterokinase cleavage site sequence at the N-terminus of the desired portion of the recombinant protein, can allow purification of the expressed fusion protein by affinity chromatography using a Ni2+ metal resin. The purification leader sequence can then be subsequently removed by treatment with enterokinase to provide the purified protein (e.g., see Hochuli et al. (1987) J. Chromatography 411:177; and Janknecht et al. PNAS 88:8972).

Techniques for making fusion genes are known to those skilled in the art. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed to generate a chimeric gene sequence (see, for example, Current Protocols in Molecular Biology, eds. Ausubel et al. John Wiley & Sons: 1992).

### 4.4.3. Antibodies

Another aspect of the invention pertains to an antibody or antibody fragment that is specifically reactive with an isolated polypeptide whose amino acid sequence is at least 95% identical to the amino acid sequence of SEQ ID NO:2. For example, by using immunogens derived from a Delta3 protein, e.g. based on the cDNA sequences, anti-protein/anti-peptide antisera or monoclonal antibodies can be made by standard protocols (See, for example, Antibodies: A Laboratory Manual ed. by Harlow and Lane (Cold Spring Harbor Press: 1988)). A mammal, such as a mouse, a hamster or rabbit can be immunized with an immunogenic form of the peptide (e.g., a Delta3 polypeptide or an antigenic fragment which is capable of eliciting an antibody response, or a fusion protein as described above). Techniques for conferring immunogenicity on a protein or peptide include conjugation to carriers or other techniques well known in the art. An immunogenic portion of a Delta3 protein can be administered in the presence of adjuvant. The progress of immunization can be monitored by detection of antibody titers in plasma or serum. Standard ELISA or other immunoassays can be used with the immunogen as antigen to assess the levels of antibodies. The subject antibodies are immunospecific for antigenic determinants of a protein represented by SEQ ID No: 2 or closely related homologs having at least 95% identity.

Following immunization of an animal with an antigenic preparation of a Delta3 polypeptide, anti- Delta3 antisera can be obtained and, if desired, polyclonal anti-Delta3 antibodies isolated from the serum. To produce monoclonal antibodies, antibody-producing cells (lymphocytes) can be harvested from an immunized animal and fused by standard somatic cell fusion procedures with immortalizing cells such as myeloma cells to yield hybridoma cells. Such techniques are well known in the art, and include, for example, the hybridoma technique (originally developed by Kohler and Milstein, (1975) Nature, 256: 495-497), the human B cell hybridoma technique (Kozbar et al., (1983) Immunology Today, 4: 72), and the EBV-hybridoma technique to produce human monoclonal antibodies (Cole et al., (1985) Monoclonal Antibodies and Cancer Therapy, Alan R Liss, Inc. pp. 77-96). Hybridoma cells can be screened immunochemically for production of antibodies specifically reactive with a Delta3 polypeptide of the present invention and monoclonal antibodies isolated from a culture comprising such hybridoma cells. In one embodiment anti-human Delta3 antibodies specifically react with the proteins encoded by the DNA of ATCC Deposit Accession Number 98348.

The term antibody as used herein is intended to include fragments thereof which are also specifically reactive with one of the subject Delta3 polypeptides. Antibodies can be fragmented using conventional techniques and the fragments screened for utility in the same manner as described above for whole antibodies. For example, F(ab)₂ fragments can be generated by treating antibody with pepsin. The resulting F(ab)₂ fragment can be treated to reduce disulfide bridges to produce Fab fragments. The antibody of the present invention is further intended to include bispecific and chimeric molecules having affinity for a Delta3 protein conferred by at least one CDR region of the antibody.

Antibodies which specifically bind Delta3 epitopes can also be used in immunohistochemical staining of tissue samples in order to evaluate the abundance and pattern of expression of each of the subject Delta3 polypeptides. Anti-Delta3 antibodies can be used diagnostically in immuno-precipitation and immuno-blotting to detect and evaluate Delta3 protein levels in tissue as part of a clinical testing procedure. For instance, such measurements can be useful in predictive valuations of the onset or progression of neurodegenerative, neoplastic or hyperplastic disorders. Likewise, the ability to monitor Delta3 protein levels in an individual can allow determination of the efficacy of a given treatment regimen for an individual afflicted with such a disorder. The level of Delta3 polypeptides may be measured from cells in bodily fluid, such as in samples of cerebral spinal fluid or amniotic fluid, or can be measured in tissue, such as produced by biopsy. Diagnostic assays using anti-Delta3 antibodies can include, for example, immunoassays designed to aid in early diagnosis of a neurodegenerative disorder, particularly ones which are manifest at birth. Diagnostic assays using anti-Delta3 polypeptide antibodies can also include immunoassays designed to aid in early diagnosis and phenotyping neurodegenerative, neoplastic or hyperplastic disorders.

Another application of anti-Delta3 antibodies of the present invention is in the immunological screening of cDNA libraries constructed in expression vectors such as λgt11, λgt18-23, λZAP, and ORF8. Messenger libraries of this type, having coding sequences inserted in the correct reading frame and orientation, can produce fusion proteins. For instance, λgt11 will produce fusion proteins whose amino termini consist of β-galactosidase amino acid sequences and whose carboxy termini consist of a foreign polypeptide. Antigenic epitopes of a Delta3 protein, e.g. other orthologs of a particular Delta3 protein or other paralogs from the same species, can then be detected with antibodies, as, for example, reacting nitrocellulose filters lifted from infected plates with anti-Delta3 antibodies. Positive phage detected by this assay can then be isolated from the infected plate. Thus, the presence of Delta3 homologs can be detected and cloned from other animals, as can alternate isoforms (including splicing variants) from humans.

### 4.5 Methods of Treating Disease

'Based at least in pan on the fact that the Notch signaling pathway has been implicated in development of the nervous system, in particular in regulating neuronal differentiation and vasculature, e.g., CNS vasculature, a wide variety of pathological diseases or conditions can benefit from treatment with Delta3 therapeutics. The Notch signaling pathway plays a role in the development of vasculature. For example, loss of DIII function mutants become severally hemorrhagic after embryonic day 10. Furthermore, mutations in Notch3 result in CADASIL, a disease characterized by stroke. In addition, mice with a functionally ablated PSI gene exhibit haemorages in the brain and /or spinal cord after embryonic day 11.5 (Wong et al., *supra*). Furthermore, since the Notch signaling pathway is involved in cell fate determination at least in the nervous system and endothelial system, it is likely that the Notch signaling pathway, and in particular Delta3 is involved in cell fate determination in additional biological systems. Accordingly, the disclosure also describes methods for treating diseases or disorders arising from an abnormal cell proliferation and/or differentiation of cells other than cells from the nervous system and vasculature.

Preferred disorders that can be treated or prevented according to the described methods include pathological neurogenic, neoplastic or hyperplastic conditions.

Disorders of the vasculature, also termed "vascular disorders", in addition to CADASIL and stroke, that can be treated or prevented according to the methods of the invention include atheroma, tumor angiogenesis, wound healing, diabetic retinopathy, hamangioma, psoriasis, and restenosis, e.g, restenosis resulting from balloon angioplasty.

Diseases or disorders caused or contributed to by aberrant Delta3 activity, such as aberrant Delta3 protein levels or an aberrant biological activity or which are associated with one or more specific Delta3 alleles, e.g., a mutant Delta3 allele, can be treated with Delta3 therapeutics. Aberrant protein levels can be caused, e.g., by aberrant gene expression. Such aberrant activity can result, for example, in aberrant cell proliferation and/or differentiation or cell death. For example, aberrant Delta3 activity in a subject can result in increased proliferation of certain cells in the subject. Subjects having a disorder characterized by abnormal cell proliferation can be treated by administration of a Delta3 therapeutic inhibiting or decreasing such proliferation. The specific Delta3 therapeutic used may vary depending on the type of the cell that is proliferating aberrantly. The appropriate Delta3 therapeutic to use can be determined, e.g., by *in vitro* culture of a sample of such cells which can be obtained from the subject, in the presence and in the absence of Delta3 therapeutics.

Diseases or conditions associated with aberrant cell proliferation which can be treated or prevented with Delta3 therapeutics include cancers, malignant conditions, premalignant conditions, benign conditions. The condition to be treated or prevented can be a solid tumor, such as a tumor arising in an epithelial tissue. For example, the cancer can be colon or cervix cancer. Cancer of the colon and cervix have in fact been found to have increased levels of expression of Notch as compared to normal tissue (PCT Application WO/07474). Accordingly, treatment of such a cancer could comprise administration to the subject of a Delta3 therapeutic decreasing the interaction of Notch with Delta3 . Other cancers that can be treated or prevented with a Delta3 protein include sarcomas and carcinomas, e.g., lung cancer, cancer of the esophagus, lung cancer, melanoma, seminoma, and squamous adenocarcinoma. Additional solid tumors include those that can be found in a medical textbook. The condition to be treated or prevented can also be a soluble tumor, such as leukemia, either chronic or acute, including chronic or acute myelogenous leukemia, chronic or acute lymphocytic leukemia, promyelocytic leukemia, monocytic leukemia, myelomonocytic leukemia, and erythroleukemia. Yet other proliferative disorders that can be treated with a Delta3 therapeutic of the invention include heavy chain disease, multiple myeloma, lymphoma, e.g., Hodgkin's lymphoma and non-Hodgkin's lymphoma, Waldenstroem's macroglobulemia, and fibroproliferative disorders, particularly of cerebravascular tissue.

Diseases or conditions characterized by a solid or soluble tumor can be treated by administrating a Delta3 therapeutic either locally or systemically, such that proliferation of the cells having an aberrant proliferation is inhibited or decreased. Methods for administering the compounds are further described below.

The disclosure also provides methods for preventing the formation and/or development of tumors. For example, the development of a tumor can be preceded by the presence of a specific lesion, such as a preneoplastic lesion, e.g., hyperplasia, metaplasia, and dysplasia. Such lesions can be found, e.g., in epithelial tissue. Thus, the disclosure provides a method for inhibiting progression of such a lesion into a neoplastic lesion, comprising administering to the subject having a preneoplastic lesion a amount of a Delta3 therapeutic sufficient to inhibit progression of the preneoplastic lesion into a neoplastic lesion.

The disclosure describes a method for inhibiting endothelial cell proliferation and/or differentiation, comprising contacting a Delta3 therapeutic with a tissue in which endothelial cells are proliferating, such as a developing tumor or a hyperproliferative disease, i.e., a disease associated with abnormal cellular proliferation. Blocking the proliferation of endothelial cells will result in inhibition of development of endothelium and blood vessels, thus limiting access to the tumor of compounds necessary for tumor development.

The disclosure also describes methods for treating or preventing diseases or conditions associated with insufficient cell proliferation. For example, Delta3 therapeutics can be used to stimulate tissue repair, regeneration, and/or wound healing, e.g. of neural tissue, such as after surgery or to stimulate tissue healing from bums. Other disease in which proliferation of cells is desired are hypoproliferative diseases, i.e, diseases characterized by an abnormally low proliferation of certain cells.

The disclosure also describes a method for treating or preventing diseases or conditions characterized by aberrant cell differentiation. Accordingly, the disclosure provides methods for stimulating cellular differentiation in conditions characterized by an inhibition of normal cell differentiation which mayor may not be accompanied by excessive proliferation. Alternatively, Delta3 therapeutics can be used to inhibit differentiation of specific cells.

In a preferred method, the aberrantly proliferating and/or differentiating cell is a cell present in the nervous system. Accordingly, the disclosure provides methods for treating diseases or conditions associated with a central or peripheral nervous system. For example, the disclosure provides methods for treating lesions of the nervous system involving an aberrant Delta3 activity in neurons, in Schwann cells, glial cells, or other types of neural cells. Disorders of the nervous system are set forth above.

The disclosure also describes a method for enhancing the survival and/or stimulating proliferation and/or differentiation of cells and tissues *in vitro.* For example, tissues from a subject can be obtained and grown *in vitro* in the presence of a Delta3 therapeutic, such that the tissue cells are stimulated to proliferate and/or differentiate. The tissue can then be readministered to the subject.

Since, in some cases, genes may be upregulated in a disease state and in other cases may be downregulated, it will be desirable to activate and/or potentiate or suppress and/or downmodulate Delta3 bioactivity depending on the condition to be treated using the techniques compounds and methods described herein. Some genes may be underexpressed in certain disease states. The activity ofDelta3 gene products may be in some way impaired, leading to the development of neurodegenerative disease symptoms. Such down-regulation of Delta3 gene expression or decrease in the activity of a Delta3 protein may have a causative or exacerbating effect on the disease state.

Among the approaches which may be used to ameliorate disease symptoms involving the misexpression of a Delta3 gene are, for example, antisense, ribozyme, and triple helix molecules described above. Compounds that compete with a Delta3 protein for binding to upstream or downstream elements in a Delta/Notch signaling cascade will antagonize a Delta3 protein, thereby inducing a therapeutic effect. Examples of suitable compounds include the antagonists or homologues described in detail above. In other instances, the increased expression or bioactivity of a Delta3 protein may be desirable and may be accomplished by, for example the use of the Delta3 agonists or mimetics or by gene replacement therapy, as described herein.

Yet other Delta3 therapeutics consist of a first peptide comprising a Delta3 peptide capable of binding to a receptor, e.g., a Notch receptor, and a second peptide which is cytotoxic. Such therapeutics can be used to specifically target and lyse cells expressing or overexpressing a receptor for Delta3. For example, a fusion protein containing a Delta3 peptide fused to a cytotoxic peptide can be used to eliminate or reduce a tumor overexpressing Notch, e.g., colon and cervix neoplastic tumors. Alternatively, cells expressing or overexpressing Delta3 can be targeted for lysis, by, for example, targeting to the cell an antibody binding specifically to a Delta3 protein linked to a cytotoxic peptide.

Based at least in part on the similarity of protein structure, it is likely that Delta3 therapeutics can also be used to treat diseases or conditions caused by or contributed by an aberrant Delta activity, e.g, an aberrant Delta1 or Delta2 activity or diseases or disorders which are associated with one or more specific Delta alleles, e.g., Delta1 or Delta2 allelles. Such diseases or conditions could include neurological diseases and cancer. Similarly, Delta therapeutics, e.g., Delta1 or Delta2 therapeutics, could be used to prevent or treat diseases or disorders caused by or contributed to by an aberrant Delta3 activity, or diseases or disorders which are associated with a specific Delta3 allele. Delta therapeutics can be prepared using, e.g, the nucleotide and protein sequence information disclosed in the PCT Patent Application WO 97/01571 and tested using the assays described herein for testing Delta3 therapeutics.

Compounds identified as increasing or decreasing Delta3 gene expression or protein activity can be administered to a subject at therapeutically effective dose to treat or ameliorate cardiovascular disease. A therapeutically effective dose refers to that amount of the compound sufficient to result in amelioration of symptoms associated with the particular disease.

### 4.5.1.Effective Dose

Toxicity and therapeutic efficacy of such compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

### 5.2. Formulation and Use

Pharmaceutical compositions for use in accordance with the present disclosure may be formulated in conventional manner using one or more physiologically acceptable carriers or excipients. Thus, the compounds and their physiologically acceptable salts and solvates may be formulated for administration by, for example, injection, inhalation or insufflation (either through the mouth or the nose) or oral, buccal, parenteral or rectal administration.

For such therapy, the oligomers of the invention can be formulated for a variety of loads of administration, including systemic and topical or localized administration. Techniques and formulations generally may be found in Remmington's Pharmaceutical Sciences, Meade Publishing Co., Easton, PA. For systemic administration, injection is preferred, including intramuscular, intravenous, intraperitoneal, and subcutaneous. For injection, the oligomers of the invention can be formulated in liquid solutions, preferably in physiologically compatible buffers such as Hank's solution or Ringer's solution. In addition, the oligomers may be formulated in solid form and redissolved or suspended immediately prior to use. Lyophilized forms are also included.

For oral administration, the pharmaceutical compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); nonaqueous vehicles (e.g., almond oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound.

For buccal administration the compositions may take the form of tablets or lozenges formulated in conventional manner.

For administration by inhalation, the compounds for use according to the present disclosure are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

The compounds may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

The compounds may also be formulated in rectal compositions such as suppositories or retention enemas, e.g., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays or using suppositories. For topical administration, the oligomers are formulated into ointments, salves, gels, or creams as generally known in the art.

In clinical settings, the gene delivery systems for the therapeutic Delta3 gene can be introduced into a patient by any of a number of methods, each of which is familiar in the art. For instance, a pharmaceutical preparation of the gene delivery system can be introduced systemically, e.g. by intravenous injection, and specific transduction of the protein in the target cells occurs predominantly from specificity of transfection provided by the gene delivery vehicle, cell-type or tissue-type expression due to the transcriptional regulatory sequences controlling expression of the receptor gene, or a combination thereof In other embodiments, initial delivery of the recombinant gene is more limited with introduction into the animal being quite localized. For example, the gene delivery vehicle can be introduced by catheter (see U.S. Patent 5,328,470) or by stereotactic injection (e.g. Chen et al. (1994) PNAS 91: 3054-3057). A Delta3 gene, such as any one of the sequences represented in the group consisting of SEQ ID NO:1 or 3, or a sequence homologous thereto can be delivered in a gene therapy construct by electroporation using techniques described, for example, by Dev et al. ((1994) Cancer Treat Rev 20:105-115).

The pharmaceutical preparation of the gene therapy construct can consist essentially of the gene delivery system in an acceptable diluent, or can comprise a slow release matrix in which the gene delivery vehicle is imbedded. Alternatively, where the complete gene delivery system can be produced intact from recombinant cells, e.g. retroviral vectors, the pharmaceutical preparation can comprise one or more cells which produce the gene delivery system.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration.

### 4.6 Diagnostic and Prognostic Assays

The present methods provides means for determining if a subject is at risk of developing a disorder characterized by an aberrant Delta3 activity, such as aberrant cell proliferation, degeneration, and/or differentiation resulting for example in a neurodegenerative disease or cancer. The disclosure also provides methods for determining whether a subject is at risk of developing a disease or disorder associated with one or more specific alleles of a Delta3 gene. In fact, specific Delta3 alleles may be associated with specific diseases or disorders. Accordingly, the disclosure provides methods for determining whether a subject has or is at risk of developing a neurological disease, e.g., ACCPN. The disclosure also describes methods for determining whether a subject has or is at risk of developing a vascular disorder or a disorder associated with cell fate determination.

In one embodiment, the disclosure describes a method for determining whether a subject has genetic lesion in a Delta3 gene or a specific allelic variant of a polymorphic region in a Delta3 gene. The specific allele can be a mutant allele. The disclosure also describes methods for determining whether a subject has an aberrant Delta3 protein, resulting from aberrant post-translational modifications of the protein, such as aberrant phosphoregulation or glycosylation Also, described are methods for determining whether a subject has an aberrant expression level of a Delta3 protein, which could be due to a genetic lesion in the Delta3 gene or due to an aberrant level or activity of a protein regulating the expression of a Delta3 gene.

The methods can be characterized as comprising detecting, in a sample of cells from the subject, the presence or absence of a genetic lesion characterized by at least one of (i) an alteration affecting the integrity of a gene encoding a Delta-protein, or (ii) the mis-expression of a Delta3 gene. To illustrate, such genetic lesions can be detected by ascertaining the existence of at least one of (i) a deletion of one or more nucleotides from a Delta3 gene, (ii) an addition of one or more nucleotides to a Delta3 gene, (iii) a substitution of one or more nucleotides of a Delta3 gene, (iv) a gross chromosomal rearrangement of a Delta3 gene, (v) a gross alteration in the level of a messenger RNA transcript of a Delta3 gene, (vii) aberrant modification of a Delta3 gene, such as of the methylation pattern of the genomic DNA, (vii) the presence of a non-wild type splicing pattern of a messenger RNA transcript of a Delta3 gene, (viii) a non-wild type level of a Delta-protein, (ix) allelic loss of a Delta3 gene, and (x) inappropriate post-translational modification of a Delta-protein. As set out below, the present invention provides a large number of assay techniques for detecting lesions in a Delta3 gene, and importantly, provides the ability to discern between different molecular causes underlying Delta-dependent aberrant cell proliferation and/or differentiation.

For determining whether a subject has or is at risk of developing a disease or condition associated with a specific allele of a Delta3 gene, preliminary experiments can be performed to determine the identity of the allele associated with a disease. For example, for determining the identity of the hDelta3 allele associated with ACCPN, one can perform mutation detection studies of the Delta3 gene in populations having a high risk of developing ACCPN. For example, one can perform mutation detection analysis of the genomic DNA from subjects in the French Canadian population in the Charlevoix and Saguenay-Lac St Jean regions of the province of Quebec (Casaubon et al., *supra*). Such an analysis will reveal the identity of the Delta3 allele or alleles associated with ACCPN. Comparison of the Delta3 allele of a subject with this allele or alleles associated with ACCPN will indicate whether a subject has a Delta3 allele associated with ACCPN and thus whether the subject has or is likely to develop ACCPN. Similarly, mutation detection analysis can also be carried out to determine the identity of Delta3 alles associated with other diseases or conditions.

In an exemplary embodiment, there is provided a nucleic acid composition comprising a (purified) oligonucleotide probe including a region of nucleotide sequence which is capable of hybridizing to a sense or antisense sequence of a Delta3 gene, such as represented by any of SEQ ID Nos: 1 and 3, alleles thereof, naturally occurring mutants thereof, or 5' or 3' flanking sequences or intronic sequences naturally associated with the subject Delta3 genes or naturally occurring mutants thereof. The nucleic acid of a cell is rendered accessible for hybridization, the probe is exposed to nucleic acid of the sample, and the hybridization of the probe to the sample nucleic acid is detected. Such techniques can be used to detect lesions at either the genomic or mRNA level, including deletions, substitutions, etc., as well as to determine mRNA transcript levels.

As set out above, one part of the disclosure relates to diagnostic assays for determining, in the context of cells isolated from a patient, if mutations have arisen in one or more Delta3 genes of the sample cells. The present method provides a method for determining if a subject is at risk for a disorder characterized by aberrant Delta3 activity, e.g., cell proliferation and/or differentiation. The method can be generally characterized as comprising detecting, in a sample of cells from the subject, the presence or absence of a genetic lesion characterized by an alteration affecting the integrity of a gene encoding a Delta protein. To illustrate, such genetic lesions can be detected by ascertaining the existence of at least one of (i) a deletion of one or more nucleotides from a Delta-gene, (ii) an addition of one or more nucleotides to a Delta-gene, (iii) a substitution of one or more nucleotides of a Delta-gene, and (iv) the presence of a non-wild type splicing pattern of a messenger RNA transcript of a Delta-gene. As set out below, the present disclosure describes a large number of assay techniques for detecting lesions in Delta3 genes, and importantly, provides the ability to discern between different molecular causes underlying Delta-dependent aberrant cell proliferation and/or differentiation.

In certain methods, detection of the lesion in a Delta gene or the identity of an allelic variant of a polymorphic region of a Delta gene comprises utilizing the probe/primer in a polymerase chain reaction (PCR) (see, e.g. U.S. Patent Nos. 4,683,195 and 4,683,202), such as anchor PCR or RACE PCR, or, alternatively, in a ligation chain reaction (LCR) (see, e.g., Landegran et al. (1988) Science 241:1077-1080; and Nakazawa et al. (1994) PNAS 91:360-364), the latter of which can be particularly useful for detecting point mutations in the Delta-gene (see Abravaya et al. (1995) Nuc Acid Res 23:675-682). In a merely illustrative method, the method includes the steps of (i) collecting a sample of cells from a patient, (ii) isolating nucleic acid (e.g., genomic, mRNA or both) from the cells of the sample, (iii) contacting the nucleic acid sample with one or more primers which specifically hybridize to a Delta gene under conditions such that hybridization and amplification of the Delta-gene (if present) occurs, and (iv) detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli, J.C. et al., 1990, Proc. Natl. Acad. Sci. USA 87:1874-1878), transcriptional amplification system (Kwoh, D.Y. et al., 1989, Proc. Natl. Acad. Sci. USA 86:1173-1177), Q-Beta Replicase (Lizardi, P.M. et al., 1988, Bio/Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In a preferred subject assay, mutations in a Delta3 gene or specific alleles of a Delta3 gene from a sample cell are identified by alterations in restriction enzyme cleavage patterns. For example, sample and control DNA is isolated, amplified (optionally), digested with one or more restriction endonucleases, and fragment length sizes are determined by gel electrophoresis. Moreover, the use of sequence specific ribozymes (see, for example, U.S. Patent No. 5,498,531) can be used to score for the presence of specific mutations by development or loss of a ribozyme cleavage site.

In yet another assay, any of a variety of sequencing reactions known in the art can be used to directly sequence the Delta3 gene and detect mutations or allelic variants of polymorphic regions by comparing the sequence of the sample Delta3 with the corresponding wild-type (control) sequence. Exemplary sequencing reactions include those based on techniques developed by Maxim and Gilbert (Proc. Natl Acad Sci USA (1977) 74:560) or Sanger (Sanger et al (1977) Proc. Nat. Acad. Sci 74:5463). It is also contemplated that any of a variety of automated sequencing procedures may be utilized when performing the subject assays (Biotechniques (1995) 19:448), including by sequencing by mass spectrometry (see, for example PCT publication WO 94/16101; Cohen et al. (1996) Adv Chromatogr 36:127-162; and Griffin et al. (1993) Appl Biochem Biotechnol 38:147-159). It will be evident to one skilled in the art that, for certain embodiments, the occurrence of only one, two or three of the nucleic acid bases need be determined in the sequencing reaction. For instance, A-tract or the like, e.g., where only one nucleic acid is detected, can be carried out.

In a further assay, protection from cleavage agents (such as a nuclease, hydroxylamine or osmium tetroxide and with piperidine) can be used to detect mismatched bases in RNA/RNA or RNA/DNA heteroduplexes (Myers, et al. (1985) Science 230:1242). In general, the art technique of "mismatch cleavage" starts by providing heteroduplexes of formed by hybridizing (labelled) RNA or DNA containing the wild-type Delta3 sequence with potentially mutant RNA or DNA obtained from a tissue sample. The double-stranded duplexes are treated with an agent which cleaves single-stranded regions of the duplex such as which will exist due to basepair mismatches between the control and sample strands. For instance, RNA/DNA duplexes can be treated with RNase and DNA/DNA hybrids treated with S1 nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al (1988) Proc. Natl Acad Sci USA 85:4397; Saleeba et al (1992) Methods Enzymod. 217:286-295. In a preferred embodiment, the control DNA or RNA can be labeled for detection.

In still another assay, the mismatch cleavage reaction employs one or more proteins that recognize mismatched base pairs in double-stranded DNA (so called "DNA mismatch repair" enzymes) in defined systems for detecting and mapping point mutations in Delta3 cDNAs obtained from samples of cells. For example, the mutY enzyme ofE. coli cleaves A at G/A mismatches and the thymidine DNA glycosylase from HeLa cells cleaves T at G/T mismatches (Hsu et al. (1994) Carcinogenesis 15:1657-1662). According to an exemplary assay, a probe based on a Delta3 sequence, e.g., a wild-type Delta3 sequence, is hybridized to a cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

In other assays, alterations in electrophoretic mobility will be used to identify mutations in Delta3 genes or for determining the identity of the Delta3 allele. For example, single strand conformation polymorphism (SSCP) may be used to detect differences in electrophoretic mobility between mutant and wild type nucleic acids (Orita et al. (1989) Proc Natl. Acad. Sci USA 86:2766, see also Cotton (1993) Mutat Res 285:125-144; and Hayashi (1992) Genet Anal Tech Appl 9:73-79). Single-stranded DNA fragments of sample and control Delta3 nucleic acids will be denatured and allowed to renature. The secondary structure of single-stranded nucleic acids varies according to sequence, the resulting alteration in electrophoretic mobility enables the detection of even a single base change. The DNA fragments may be labelled or detected with labelled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet 7:5).

In yet another assay the movement of mutant or wild-type fragments in polyacrylamide gels containing a gradient of denaturant is assayed using denaturing gradient gel electrophoresis (DGGE) (Myers et al (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing agent gradient to identify differences in the mobility of control and sample DNA (Rosenbaum and Reissner (1987) Biophys Chem 265:12753).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki et al. (1986) Nature 324:163); Saiki et al (1989) Proc. Natl Acad. Sci USA 86:6230). Such allele specific oligonucleotide hybridization techniques may be used to test one mutation per reaction when oligonucleotides are hybridized to PCR amplified target DNA or a number of different mutations when the oligonucleotides are attached to the hybridizing membrane and hybridized with labelled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et al (1989) Nucleic Acids Res. 17:2437-2448) or at the extreme 3' end of one primer where, under appropriate conditions, mismatch can prevent, or reduce polymerase extension (Prossner (1993) Tibtech 11:238. In addition it may be desirable to introduce a novel restriction site in the region of the mutation to create cleavage-based detection (Gasparini et al (1992) Mol. Cell Probes 6: 1). It is anticipated that in certain embodiments amplification may also be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

Another described nucleic acid composition comprises a (purified) oligonucleotide probe including a region of nucleotide sequence which is capable of hybridizing to a sense or antisense sequence of a Delta-gene, or naturally occurring mutants thereof, or 5' or 3' flanking sequences or intronic sequences naturally associated with the subject Delta-genes or naturally occurring mutants thereof. The nucleic acid of a cell is rendered accessible for hybridization, the probe is exposed to nucleic acid of the sample, and the hybridization of the probe to the sample nucleic acid is detected. Such techniques can be used to detect lesions at either the genomic or mRNA level, including deletions, substitutions, etc., as well as to determine mRNA transcript levels. Such oligonucleotide probes can be used for both predictive and therapeutic evaluation of allelic mutations which might be manifest in, for example, a neurodegenerative, neoplastic or hyperplastic disorders (e.g. aberrant cell growth).

The methods described herein may be performed, for example, by utilizing pre-packaged diagnostic kits comprising at least one probe nucleic acid or antibody reagent described herein, which may be conveniently used, e.g., in clinical settings to diagnose patients exhibiting symptoms or family history of a disease or illness involving a Delta3 gene.

Any cell type or tissue, preferably neural or endothelial cells, in which the Delta3 is expressed may be utilized in the diagnostics described below. For example, a subject's bodily fluid (e.g. blood) can be obtained by known techniques (e.g. venipuncture). Alternatively, nucleic acid tests can be performed on dry samples (e.g. hair or skin). Fetal nucleic acid samples can be obtained from maternal blood as described in International Patent Application No. WO91/07660 to Bianchi. Alternatively, amniocytes or chorionic villi may be obtained for performing prenatal testing, e.g., of ACCPN, which is a disease which is usually fatal in the third decade of life.

Diagnostic procedures may also be performed in situ directly upon tissue sections (fixed and/or frozen) of patient tissue obtained from biopsies or resections, such that no nucleic acid purification is necessary. Nucleic acid reagents may be used as probes and/or primers for such in situ procedures (see, for example, Nuovo, G.J., 1992, PCR in situ hybridization: protocols and applications, Raven Press, NY).

In addition to methods which focus primarily on the detection of one nucleic acid sequence, profiles may also be assessed in such detection schemes. Fingerprint profiles may be generated, for example, by utilizing a differential display procedure, Northern analysis and/or RT-PCR.

Antibodies directed against wild type or mutant Delta3 proteins, which are discussed, above, may also be used in disease diagnostics and prognostics. Such diagnostic methods, may be used to detect abnormalities in the level of Delta3 protein expression, or abnormalities in the structure and/or tissue, cellular, or subcellular location of Delta3 proteins. Structural differences may include, for example, differences in the size, electronegativity, or antigenicity of the mutant Delta3 protein relative to the normal Delta3 protein. Protein from the tissue or cell type to be analyzed may easily be detected or isolated using techniques which are well known to one of skill in the art, including but not limited to western blot analysis. For a detailed explanation of methods for carrying out western blot analysis, see Sambrook et al, 1989, *supra*, at Chapter 18. The protein detection and isolation methods employed herein may also be such as those described in Harlow and Lane, for example, (Harlow, E. and Lane, D., 1988, "Antibodies: A Laboratory Manual", Cold Spring Harbor Laboratory Press, Cold Spring Harbor, New York), which is incorporated herein by reference in its entirety.

This can be accomplished, for example, by immunofluorescence techniques employing a fluorescently labeled antibody (see below) coupled with light microscopic, flow cytometric, or fluorimetric detection. The antibodies (or fragments thereof) useful in the present invention may, additionally, be employed histologically, as in immunofluorescence or immunoelectron microscopy, for in situ detection of Delta3 proteins. In situ detection may be accomplished by removing a histological specimen from a patient, and applying thereto a labeled antibody of the present invention. The antibody (or fragment) is preferably applied by overlaying the labeled antibody (or fragment) onto a biological sample. Through the use of such a procedure, it is possible to determine not only the presence of the Delta3 protein, but also its distribution in the examined tissue. Using the present invention, one of ordinary skill will readily perceive that any of a wide variety of histological methods (such as staining procedures) can be modified in order to achieve such in situ detection.

Often a solid phase support or carrier is used as a support capable of binding an antigen or an antibody. Well-known supports or carriers include glass, polystyrene, polypropylene, polyethylene, dextran, nylon, amylases, natural and modified celluloses, polyacrylamides, gabbros, and magnetite. The nature of the carrier can be either soluble to some extent or insoluble for the purposes of the present invention. The support material may have virtually any possible structural configuration so long as the coupled molecule is capable of binding to an antigen or antibody. Thus, the support configuration may be spherical, as in a bead, or cylindrical, as in the inside surface of a test tube, or the external surface of a rod. Alternatively, the surface may be flat such as a sheet, test strip, etc. Preferred supports include polystyrene beads. Those skilled in the art will know many other suitable carriers for binding antibody or antigen, or will be able to ascertain the same by use of routine experimentation.

One means for labeling an anti-Delta3 protein specific antibody is via linkage to an enzyme and use in an enzyme immunoassay (EIA) (Voller, "The Enzyme Linked Immunosorbent Assay (ELISA)", Diagnostic Horizons 2:1-7, 1978, Microbiological Associates Quarterly Publication, Walkersville, MD; Voller, et al., J. Clin. Pathol. 31:507-520 (1978); Butler, Meth. Enzymol. 73:482-523 (1981); Maggio, (ed.) Enzyme Immunoassay, CRC Press, Boca Raton, FL, 1980; Ishikawa, et al., (eds.) Enzyme Immunoassay, Kgaku Shoin, Tokyo, 1981). The enzyme which is bound to the antibody will react with an appropriate substrate, preferably a chromogenic substrate, in such a manner as to produce a chemical moiety which can be detected, for example, by spectrophotometric, fluorimetric or by visual means. Enzymes which can be used to detectably label the antibody include, but are not limited to, malate dehydrogenase, staphylococcal nuclease, delta-5-steroid isomerase, yeast alcohol dehydrogenase, alpha-glycerophosphate, dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholinesterase. The detection can be accomplished by colorimetric methods which employ a chromogenic substrate for the enzyme. Detection may also be accomplished by visual comparison of the extent of enzymatic reaction of a substrate in comparison with similarly prepared standards.

Detection may also be accomplished using any of a variety of other immunoassays. For example, by radioactively labeling the antibodies or antibody fragments, it is possible to detect fingerprint gene wild type or mutant peptides through the use of a radioimmunoassay (RIA) (see, for example, Weintraub, B., Principles of Radioimmunoassays, Seventh Training Course on Radioligand Assay Techniques, The Endocrine Society, March, 1986, which is incorporated by reference herein). The radioactive isotope can be detected by such means as the use of a gamma counter or a scintillation counter or by autoradiography.

It is also possible to label the antibody with a fluorescent compound. When the fluorescently labeled antibody is exposed to light of the proper wave length, its presence can then be detected due to fluorescence. Among the most commonly used fluorescent labeling compounds are fluorescein isothiocyanate, rhodamine, phycoerythrin, phycocyanin, allophycocyanin, o-phthaldehyde and fluorescamine.

The antibody can also be detectably labeled using fluorescence emitting metals such as ¹⁵²Eu, or others of the lanthanide series. These metals can be attached to the antibody using such metal chelating groups as diethylenetriaminepentacetic acid (DTPA) or ethylenediaminetetraacetic acid (EDTA).

The antibody also can be detectably labeled by coupling it to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise a biolumineseent compound may be used to label the antibody of the present invention. Bioluminescence is a type of chemiluminescence found in biological systems in, which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

Moreover, it will be understood that any of the above methods for detecting alterations in a Delta3 gene or gene product can be used to monitor the course of treatment or therapy.

### 4.7. Drug Screening Assays

The disclosure describes compounds, e.g., therapeutic compounds, for treating diseases or conditions caused by, or contributed to by an abnormal Delta3 activity. The compounds that can be used for this purpose can be any type of compound, including a protein, a peptide, peptidomimetic, small molecule, and nucleic acid. A nucleic acid can be, e.g., a gene, an antisense nucleic acid, a ribozyme, or a triplex molecule. A compound of the disclosure can be an agonist or an antagonist. A compound can act on a Delta3 gene, e.g., to modulate its expression. A compound can also act on a Delta3 protein, e.g, to modulate signal transduction from the receptor. Accordingly, a compound of the disclosure can be a compound which binds to Delta3 and induces signal transduction from the receptor, such that, e.g. a Delta3 activity is induced. Alternatively, a compound of the disclosure can be a compound which inhibits interaction of a Delta3 protein with a toporythmic protein, e.g., Notch. One compound of the disclosure which interacts with a Delta protein, which is either an agonist or an antagonist, or other protein interacting with Delta3. Another compound is a soluble toporythmic protein or other protein interacting with Delta3.

Accordingly, a soluble toporythmic protein can be stimulatory form of a toporythmic protein or an inhibitory form of a toporythmic, depending on whether the particular toporythmic protein stimulates or inhibits a Delta3 activity.

Similarly, a soluble Delta3 protein, e.g., Delta3-Ig, can be used to modulate an activity of a toporythmic protein, e.g., Notch. For example, a soluble Delta3 protein can be a stimulatory form of a Delta3 protein, i.e., a Delta3 protein which is capable of stimulating an activity of a toporythmic protein. In one embodiment, such a protein acts in essentially the same manner as wild-type Delta3. In another embodiment, a soluble Delta3 protein is an inhibitory form of a Delta3 protein, i.e., a Delta3 protein which is capable of inhibiting an activity of a toporythmic protein. For example, such a Delta3 protein could inhibit the interaction of wild-type Delta3 with the toporythmic protein. In a preferred embodiment, an inhibitory form of a Delta3 protein inhibits the interaction of several proteins which normally interact with a toporythmic protein, by, e.g, binding to a site of the toporythmic protein that is also a binding site to various other proteins, e.g, other Delta proteins. Accordingly, a Delta3 therapeutic can generally affect the interaction of various toporythmic proteins with each other. Similarly, based at least in part on the sequence and structural similarities between Delta proteins, a Delta therapeutic, other than a Delta3 therapeutic, can also be used for modulating the interaction between a Delta3 protein and a Delta3 interacting binding molecule.

The compounds of the disclosure can be identified using various assays depending on the type of compound and activity of the compound that is desired. Set forth below are at least some assays that can be used for identifying Delta3 therapeutics. It is within the skill of the art to design additional assays for identifying Delta therapeutics, e.g., Delta3 therapeutics.

By making available purified and recombinant Delta3 polypeptides, the present invention facilitates the development of assays which can be used to screen for drugs, including Delta3 variants, which are either agonists or antagonists of the normal cellular function of the subject Delta3 polypeptides, or of their role in the pathogenesis of cellular differentiation and/or proliferation and disorders related thereto. In one embodiment, the assay evaluates the ability of a compound to modulate binding between a Delta3 polypeptide and a molecule, be it protein or DNA, that interacts either upstream or downstream of the Delta/Notch signaling pathway. A variety of assay formats will suffice and, in light of the present inventions, will be comprehended by a skilled artisan.

### 4.7.1 Cell-free assays

Cell free assays can be used to identify compounds which interact with a Delta3 protein. Such assays are available for testing compounds which are proteins, e.g., toporythmic proteins or variants thereof; as well as for testing compounds which are peptidomimetics, small molecules or nucleic acids. The specific assay used for testing these compounds may vary with the type of compound.

In one embodiment, a compound that interacts with a Delta3 protein is identified by screening, e.g., a library of compounds, for binding to a recombinant or purified Delta3 protein or at least a portion thereof. Such assays can involve labeling one or the two components and measuring the extent of their interaction, by, e.g., determining the level of the one or two labels. In these assays, it may be preferable to attach the Delta3 protein to a solid phase surface. Methods for achieving this are further described infra. In one embodiment, the library of compounds is a library of small molecules. In another embodiment, the library of compounds is a library of Delta3 variants, which can be produced according to methods described infra.

Identification of a compound which inhibits an interaction between a Delta3 protein and a toporythmic protein can also be performed by screening compounds using aggregation assays, as described, e.g., in Fehon et al. (1990) Cell 61:523-534.

The disclosure describes methods for identifying compounds which inhibit the interaction of a Delta3 protein with a molecule, e.g., a toporythmic protein or a protein interacting with the cytoplasmic domain of a Delta3 protein. Such methods, which are preferably used in high throughput assays can be peformed as follows.

In many drug screening programs which test libraries of compounds and natural extracts, high throughput assays are desirable in order to maximize the number of compounds surveyed in a given period of time. Assays which are performed in cell-free systems, such as may be derived with purified or semi-purified proteins, are often preferred as "primary" screens in that they can be generated to permit rapid development and relatively easy detection of an alteration in a molecular target which is mediated by a test compound. Moreover, the effects of cellular toxicity and/or bioavailability of the test compound can be generally ignored in the *in vitro* system, the assay instead being focused primarily on the effect of the drug on the molecular target as may be manifest in an alteration of binding affinity with upstream or downstream elements. Accordingly, in an exemplary screening assay, the compound of interest is contacted with proteins which may function upstream (including both activators and repressors of its activity) or to proteins or nucleic acids which may function downstream of the Delta3 polypeptide, whether they are positively or negatively regulated by it. For example, a protein functioning upstream of a Delta3 polypeptide can be a compound interacting with the extracellular portion of the Delta3 molecule. A protein functioning downstream of a Delta3 polypeptide can be a protein interacting with the cytoplasmic domain of Delta3 and, e.g, transducing a signal to the nucleus. To the mixture of the compound and the upstream or downstream element is then added a composition containing a Delta3 polypeptide. Detection and quantification of complexes of Delta3 with it's upstream or downstream elements provide a means for determining a compound's efficacy at inhibiting (or potentiating) complex formation between Delta3 and the Delta-binding elements. The efficacy of the compound can be assessed by generating dose response curves from data obtained using various concentrations of the test compound. Moreover, a control assay can also be performed to provide a baseline for comparison. In the control assay, isolated and purified Delta3 polypeptide is added to a composition containing the Delta-binding element, and the formation of a complex is quantitated in the absence of the test compound.

Complex formation between the Delta3 polypeptide and a Delta3 binding element may be detected by a variety of techniques. Modulation of the formation of complexes can be quantitated using, for example, detectably labeled proteins such as radiolabeled, fluorescently labeled, or enzymatically labeled Delta3 polypeptides, by immunoassay, or by chromatographic detection.

Typically, it will be desirable to immobilize either Delta3 or its binding protein to facilitate separation of complexes from uncomplexed forms of one or both of the proteins, as well as to accommodate automation of the assay. Binding of Delta3 to an upstream or downstream element, in the presence and absence of a candidate agent, can be accomplished in any vessel suitable for containing the reactants. Examples include microtitre plates, test tubes, and micro-centrifuge tubes. A fusion protein can be provided which adds a domain that allows the protein to be bound to a matrix. For example, glutathione-S-transferase/Delta3 (GST/Delta) fusion proteins can be adsorbed onto glutathione sepharose beads (Sigma Chemical, St. Louis, MO) or glutathione derivatized microtitre plates, which are then combined with the cell lysates, e.g. an ³⁵S-labeled, and the test compound, and the mixture incubated under conditions conducive to complex formation, e.g. at physiological conditions for salt and pH, though slightly more stringent conditions may be desired. Following incubation, the beads are washed to remove any unbound label, and the matrix immobilized and radiolabel determined directly (e.g. beads placed in scintilant), or in the supernatant after the complexes are subsequently dissociated. Alternatively, the complexes can be dissociated from the matrix, separated by SDS-PAGE, and the level of Delta-binding protein found in the bead fraction quantitated from the gel using standard electrophoretic techniques such as described in the appended examples.

Other techniques for immobilizing proteins on matrices are also available for use in the subject assay. For instance, either Delta3 or its cognate binding protein can be immobilized utilizing conjugation of biotin and streptavidin. For instance, biotinylated Delta3 molecules can be prepared from biotin-NHS (N-hydroxysuccinimide) using techniques well known in the art (e.g., biotinylation kit, Pierce Chemicals, Rockford, IL), and immobilized in the wells of streptavidin-coated 96 well plates (Pierce Chemical). Alternatively, antibodies reactive with Delta3 but which do not interfere with binding of upstream or downstream elements can be derivatized to the wells of the plate, and Delta3 trapped in the wells by antibody conjugation. As above, preparations of a Delta-binding protein and a test compound are incubated in the Delta-presenting wells of the plate, and the amount of complex trapped in the well can be quantitated. Exemplary methods for detecting such complexes, in addition to those described above for the GST-immobilized complexes, include immunodetection of complexes using antibodies reactive with the Delta3 binding element, or which are reactive with Delta3 protein and compete with the binding element; as well as enzyme-linked assays which rely on detecting an enzymatic activity associated with the binding element, either intrinsic or extrinsic activity. In the instance of the latter, the enzyme can be chemically conjugated or provided as a fusion protein with the Delta-BP. To illustrate, the Delta-BP can be chemically cross-linked or genetically fused with horseradish peroxidase, and the amount of polypeptide trapped in the complex can be assessed with a chromogenic substrate of the enzyme, e.g. 3,3'-diamino-benzadine terahydrochloride or 4-chloro-1-napthol. Likewise, a fusion protein comprising the polypeptide and glutathione-S-transferase can be provided, and complex formation quantitated by detecting the GST activity using 1-chloro-2,4-dinitrobenzene (Habig et al (1974) J Biol Chem 249:7130).

For processes which rely on immunodetection for quantitating one of the proteins trapped in the complex, antibodies against the protein, such as anti-Delta3 antibodies, can be used. Alternatively, the protein to be detected in the complex can be "epitope tagged" in the form of a fusion protein which includes, in addition to the Delta3 sequence, a second polypeptide for which antibodies are readily available (e.g. from commercial sources). For instance, the GST fusion proteins described above can also be used for quantification of binding using antibodies against the GST moiety. Other useful epitope tags include myc-epitopes (e.g., see Ellison et al. (1991) J Biol Chem 266:21150-21157) which includes a 10-residue sequence from c-myc, as well as the pFLAG system (International Biotechnologies, Inc.) or the pEZZ-protein A system (Pharamacia, NJ).

### 4.7.2. Cell based assays

In addition to cell-free assays, such as described above, the readily available source of Delta3 proteins provided by the present invention also facilitates the generation of cell-based assays for identifying small molecule agonists/antagonists and the like. For example, cells which are sensitive to bFGF/VEGF or matrigel can be caused to overexpress a recombinant Delta3 protein in the presence and absence of a test agent of interest, with the assay scoring for modulation in Deha3 responses by the target cell mediated by the test agent. As with the cell-free assays, agents which produce a statistically significant change in Delta-dependent responses (either inhibition or potentiation) can be identified. In an illustrative embodiment, the expression or activity of a Delta3 is modulated in embryos or cells and the effects of compounds of interest on the readout of interest (such as tissue differentiation, proliferation, tumorigenesis) are measured. For example, the expression of genes which are up- or down-regulated in response to a Delta-dependent signal cascade can be assayed. In preferred embodiments, the regulatory regions of such genes, e.g., the 5' flanking promoter and enhancer regions, are operably linked to a detectable marker (such as luciferase) which encodes a gene product that can be readily detected.

Exemplary cell lines may include endothelial cells such as MVEC's and bovine aortic endothelial cells (BAEC's); as well as generic mammalian cell lines such as HeLa cells and COS cells, e.g., COS-7 (ATCC#CRL-1651).

A test compound that modifies a Delta3 activity can be identified by incubating a cell having a Delta3 protein with the test compound and measuring signal transduction from the Delta3 protein. Comparison of the signal transduction in the cells incubated with or without the test compound will reveal whether the test compound is a Delta3 therapeutic. Similarly, a test compound that modifies a Delta3 activity can be identified by incubating a cell having a Delta3 ligand with the test compound, e.g., a Delta3 derived compound, and measuring signal transduction from the Delta3 ligand. Comparison of the signal transduction in the cells incubated with or without the test compound will reveal whether the test compound is a Delta3 therapeutic.

In the event that the Delta3 proteins themselves, or in complexes with other proteins, are capable of binding DNA and/or modifying transcription of a gene, a transcriptional based assay could be used, for example, in which a Delta3 responsive regulatory sequence is operably linked to a detectable marker gene, e.g., a luciferase gene. Similarly, Delta3 therapeutics could also be identified by using an assay in which expression of genes that are modulated upon binding of a Delta3 protein to a Delta3 ligand on a cell is monitored. Genes that are responsive to interaction with a Delta3 protein or Delta3 ligand can be identified according to methods known in the art, e.g., differential hybridization or differential display.

A silicon-based device, called a microphysiometer, can be used to detect and measure the response of cells having a Delta3 protein to test compounds to identify Delta3 therapeutics. This instrument measures the rate at which cells acidify their environment, which is indicative of cellular growth and/or differentiation (McConnel et al. (1992) Science 257:1906).

Monitoring the influence of compounds on cells may be applied not only in basic drug screening, but also in clinical trials. In such clinical trials, the expression of a panel of genes may be used as a "read out" of a particular drug's therapeutic effect.

The subject Delta3 polypeptides can be used to generate a "two hybrid" assay (see, for example, U.S. Patent No. 5,283,317; Zervos et al. (1993) Cell 72:223-232; Madura et al. (1993) J Biol Chem 268:12046-12054; Bartel et al. (1993) Biotechniques 14:920-924; Iwabuchi et al. (1993) Oncogene 8:1693-1696; and Brent WO94/10300) for isolating coding sequences for other cellular proteins which bind to or interact with Delta3 ("Delta-binding proteins" or "Delta-bp"), such as Notch, and the like. Briefly, the two hybrid assay relies on reconstituting *in vivo* a functional transcriptional activator protein from two separate fusion proteins. In particular, the method makes use of chimeric genes which express hybrid proteins. To illustrate, a first hybrid gene comprises the coding sequence for a DNA-binding domain of a transcriptional activator fused in frame to the coding sequence for a Delta3 polypeptide. The second hybrid protein encodes a transcriptional activation domain fused in frame to a sample gene from a cDNA library. If the bait and sample hybrid proteins are able to interact, e.g., form a Delta-dependent complex, they bring into close proximity the two domains of the transcriptional activator. This proximity is sufficient to cause transcription of a reporter gene which is operably linked to a transcriptional regulatory site responsive to the transcriptional activator, and expression of the reporter gene can be detected and used to score for the interaction of the Delta3 and sample proteins. This system can be used to identify compounds which modify, e.g., inhibit the interaction between a Delta3 protein and another protein, by adding 1 test compound to a cell containing the above-described plasmids. The effect of the test compound on the reporter gene expression and then measured to determine the effect of the test compound on the interaction.

Also described are arrays for identifying compounds that can induce apoptosis of cells through a Delta3 protein. Apoptotic arrays are known in the act and are described, e.g in Grimm et al (1996) Proc. Natl. Acad. Sci. USA 93:10923.

### 5. EXAMPLES

### 5.1 Isolation of a full length cDNA encoding hDelta3

Human microvascular endothelial cells (HMVEC catalog #CGC2543; Clonetics, San Diego, CA.) were separated into four samples of cells which were treated as follows. The first sample was untreated. The second sample was treated with human TGF-β 1(hTGF-β 1) (10ng/ml) (Upstate Biotechnology, Lake Placid, N.Y., Catalog No. 01-134). The third sample was treated with bFGF (10ng/ml)/VEGF (23ng/ml) (Upstate Biotechnology, Lake Placid, N.Y., Catalog No. 01-134, Catalog Nos. 01-106 and out-185, respectively). The fourth sample was differentiated on Matrigel (Collaborative Biomedical Products, Becton Dickinson Labware, Bedford, MA). Cells were treated as indicated for 24 hours, the 4 samples were pooled, and RNA was extracted from the pooled cells using a QLAGEN RNeasy kit The resulting cDNA library was subjected to high throughput random sequencing. This allowed identification of a cDNA fragment comprising the following 171 nucleotide long sequence:

Comparison of the nucleotide sequence of this partial cDNA with the sequences in GenBank using the BLAST program (Altschul et al. (1990) J. Mol. Biol. 215:403) revealed that the nucleotide sequence encoded a protein fragment having a significant homology to Delta proteins. In fact, the amino acid sequence had significant homology with a chicken Delta1 protein (GenBank Accession No. U26590), a *Xenopus* Delta1 protein (GenBank Accession No. IA2229), a rat Delta 1 protein (GenBank Accession No. U78889), a Xenopus Delta2 protein (GenBank Accession No. U70843) as well as Notch proteins.

A full length cDNA of about 3.2 kb was then isolated by screening a human microvascular endothelial cell (HMVEC) cDNA library using the partial cDNA (SEQ ID NO. 21). This nucleic acid was deposited at the American Type Culture Collection (ATCC) on March 5, 1997, and has been assigned ATCC Accession No. 98348. The nucleotide sequence of the cDNA isolated is shown in Figure 1 and has SEQ ID No. 1.

A nucleic acid sequence comparison of SEQ ID No. 1 against EST sequence databases using the BLAST program (Altschul et al. (1990) J. Mol. Biol. 215:403) indicated that 5 ESTs have a homology to portions of SEQ ID No. 1. These are all located 3' of the nucleotide sequence encoding the transmembrane domain, i.e., downstream of nucleotide 1996 of SEQ ID No. 1. Three of these ESTs (having accession Nos. T33770, T33811, and T07963) have a nucleotide sequence starting at about nucleotide 2044 of SEQ ID No. 1. However, the nucleotide sequence of the three EST is significantly different from the nucleotide sequence of hDelta3 in about the first 50 nucleotides 3' of nucleotide 2044 of SEQ ID No. 1. Two ESTs (having Accession Nos. R32717 and T07962) are located further downstream of the three ESTs.

The nucleic acid having SEQ ID No. 1 encodes a protein of 685 amino acids having SEQ ID No. 2. A comparison of the amino acid sequence of SEQ ID No. 2 with sequences in GenBank using BLASTP. (Altschul et al. (1990) J. Mol. Biol. 215:403) reveals that this protein has a certain homology to previously described Delta proteins. Figure 2 shows an alignment of the human Delta3 protein having SEQ ID No. 2 with the amino acid sequence of mouse Delta1 protein (Accession No. X80903), rat DeltaI protein (Accession No. U78889), chicken Deltal protein (Accession No. U26590), two *Xenopus* DeltaI proteins (Accession Nos. L42229 and U70843) and *Drosophila* Delta1 protein (Accession No. AA142228). The sequence comparison indicates that human Delta3 protein has the general structure of a Delta3 protein. In particular, human Delta3 protein has a signal peptide corresponding to about amino acid 1 to about amino acid 17 of SEQ ID No. 2, a DSL motif corresponding to the sequence from about amino acid 173 to about amino acid 217, a first EGF-like repeat corresponding to the sequence from about amino acid 222 to about amino acid 250, a second EGF-like repeat corresponding to the sequence from about amino acid 253 to about amino acid 281, a third EGF-like repeat corresponding to the sequence from about amino acid 288 to about amino acid 321, a fourth EGF-like repeat corresponding to the sequence from about amino acid 328 to about amino acid 359, a fifth EGF-like repeat corresponding to the sequence from about amino acid 366 to about amino acid 399, a sixth EGF-like repeat corresponding to the sequence from about amino acid 411 to about amino acid 437, a seventh EGF-like repeat corresponding to the sequence from about amino acid 444 to about amino acid 475, an eight EGF-like repeat corresponding to the sequence from about amino acid 484 to about amino acid 517, a transmembrane domain corresponding to the sequence from about amino acid 530 to about amino acid 553, and a cytoplasmic domain corresponding to the sequence from about amino acid 554 to about amino acid 685 of SEQ ID No. 2.

An amino acid and nucleotide sequence comparison between the members of the Delta1 and Delta3 protein family and human Delta3 on one hand and between the members of the Delta1 family reveals that the homology between the Delta3 family members is stronger than the homology between human Delta3 and any of the Delta 1 family members. For example, although hDelta3 is only approximately 58% similar to the *Drosophila* Delta1 protein; approximately 70% similar to the mouse Delta1 protein; approximately 70% similar to the rat Delta1 protein; approximately 68% similar to the chick Deltal protein; and approximately 68% similar to the *Xenopus* Delta1 proteins; the drosophila, mouse, rat, chick and *Xenopus* Delta1 proteins are very similar to each other (e.g. the mouse and rat are about 96% similar). Published PCT application WO97/01571 discloses a partial nucleotide and amino acid sequence of a protein having significant homology to Delta1 family members, indicating that it is likely to be a human Delta1 protein. The homology between the partial amino acid sequence of human Delta1 and the amino acid sequence of human Delta3 is indicated in Table I and shows that the proteins are encoded by different genes. All these amino acid and nucleotide sequence comparisons indicate that human Delta3 is an additional species of Delta proteins, sharing some sequence and structure homology with the Delta1 proteins.

### 5.2 Tissue Expression of the hDelta3 gene

This Example describes the tissue distribution of Delta3 protein, as determined by Northern blot hybridization with a 1.6 kb fragment of human Delta3 cDNA corresponding to the extreme 3' end of SEQ ID No. 1.

Northern blot hybridizations with the various RNA samples were performed under standard conditions and washed under stringent conditions, i.e., in 0.2 x SSC at 65°C. In each sample, the probe hybridized to a single RNA of about 3.5 kb. The results of hybridization of the probe to various mRNA samples are described below.

Hybridization of a Clontech Fetal Multiple Tissue Northern (MTN) blot (Clontech, LaJolla, CA) containing RNA from fetal brain, lung, liver, and kidney indicated the presence of Delta3 RNA in each of these fetal tissues. Expression was significantly higher in fetal lung and kidney than in fetal brain and liver. Hybridization of a Clontech human Multiple Tissue Northern I (MTNI) and Multiple Tissue Northern II (MTNII) blots (Clontech, LaJolla, CA) containing RNA from adult heart, brain, placenta, lung, liver, skeletal muscle, kidney, pancreas, spleen , thymus, prostate, testis, ovary, small intestine, mucosal lining of the colon, and peripheral blood leukocytes with the human 1.6 kb Delta3 probe indicated expression in heart, placenta, lung, skeletal muscle, kidney, pancreas, spleen, thymus, prostate, testis, ovary, small intestine and colon. Expression was particularly strong in adult heart, placenta, lung, and skeletal muscle. Expression was also found in adult brain, liver and testis. However, no significant amount of hDelta3 mRNA was detected in adult peripheral blood leukocytes.

Further, Northern blot hybridization of total mRNA from HMVEC cells treated with TGF-β1 at 10 ng/ml for 24 hours, bFGF at 10 ng/ml/ VEGF at 25 ng/ml for 24 hours, or untreated for 24 hours indicated that Delta3 expression was induced upon induction with bFGFNEGF. Accordingly, expression of Delta3 is up regulated in HMV endothelial cells in response to certain growth factors.

Hybridization of a "cancer" Northern blot containing RNA from HL-60, HeLa, K562, MoLT4, Raji, SW480, A549, and G361 cells, revealed that Delta3 is expressed at high levels in the colorectal carcinoma cell line SW480. Thus, Delta3 expression is high in at least certain tumor cells.

Thus, this Example shows that the Delta3 gene is expressed in numerous tissues, but that it is non detectable in certain tissues, e.g., peripheral blood leukocytes and adult heart tissue (at least when using Northern blot hybridization), that it is expressed at relatively high levels in at least some tumorcells, e.g, colon carcinoma cells, and that its expression can be up-regulated in response to some growth factors, e.g, bFGF and VEGF.

### 5.3 Chromosomal localization of the hDelta3 gene

A Southern blot containing DNA from a panel of a human/hamster monochromosomal somatic cell hybrids was probed with an hDelta1cDNA probe. The results obtained clearly indicates that the human Delta3 gene resides on chromosome 15.

### 5.4 Increased expression of hDelta3 in differentiating endothelial cells

This Example shows that the expression of the hDelta3 gene increases in differentiating endothelial cells relative to non differentiating endothelial cells.

HMVEC cells were separated into 5 cultures and treated as follows: (1) cells were Induced to quiescence by growth in basal endothelial growth medium (EGM) (Clontech) which contains 10% fetal calf serum (FCS); (2) cells were grown in complete endothelial growth medium (EGM-MV) (Ctontech, Catalog No. CC-3125) which contains 10% FCS and growth factors; (3) cells were stimulated to proliferate by culture in EGM-MV in the presence of bFGF at 10 ng/ml and VEGF at 25 ng/ml; (4) cells were stimulated to proliferate by culture in EGM-MV in the presence of TGF-β1 at 10 ng/ml; and (5) cells were stimulated to differentiate by culture in EGM-MV on Matrigel. After 24 hours of culture, the cells were harvested, the RNA was extracted and submitted to Northern blot analysis. Hybridization was performed with the 1.6 kb hDelta3 probe described above. The results indicate that among the culture conditions tested, quiescent cells express the lowest amount of hDelta3 (at a barely detectable level). Cells which are proliferating express a higher level of hDelta3. Interestingly, the mRNA level of hDelta3 was strongly increased in cells induced to differentiate by plating on Matrigel.

Thus, this Example clearly demonstrates that hDelta3 expression is strongly increased in cells induced to differentiate and also in cells induced to proliferate.

### 5.5 hDelta3 is located in a chromosomal region associated with ACCPN

The location of hDelta3 on human chromosome 15 was determined using radiation hybrid (RH) mapping.

A sequence tagged site (STS) was generated from the 3' untranslated region of the gene using a forward primer having the nucleotide sequence GTTTACATTGCATCCTGGAT (SEQ ID NO. 21) and a reverse primer having the nucleotide sequence CTCTTCTGTTCCTCTGGTTG (SEQ ID NO. 22). The STS was used to screen the Genebridge 4 (Gyapay et al. (1996) Human Molecular Genetics 5:339) and the Standford G3 (Stewart et al. (1997) Genome Res. 7: 422) radiation hybrid panels. These panels were derived by fusion of irradiated human donor cells with rodent recipient cells (reference) and can be used for positioning STS markers within existing framework maps, ordering markers in the region of interest as well as establishing the distance between markers.

RH mapping was performed by PCR under the following conditions: 25 ng DNA/20µl reaction, 0,5 µ M of each primer, 0.2mM of each nucleotide, 1.5mM MgCl₂, 1 x buffer as provided by the manufacturer of the enzyme, 35 cycles at 94°C, 55°C, 72°C for 30 seconds each.

The results of the RH mapping indicated that hDelta3 maps to 15q12-15 close to framework marker D15S1244 on the Stanford G3 panel and close to framework marker D 15 S 144 on the Genebridge 4 panel with a LOD score >3. Searching of the OMIM database (Online Mendelian Inheritence in Man; http://www.ncbi.nlm.nih.gob/Omim/searchom-im.html) indicated that this region has previously been genetically linked to a neurological disorder called Agenesis of the Corpus Callosum with Peripheral Neuropathy (ACCPN) (Casaubon et al. (1996) Am. J. Hum. Genet. 58:28).

### 5.6 Identification of Delta-therapeutics

This Example describes a simple assay for isolating Delta therapeutics, (e.g., agonist or antagonist of a Delta bioactivity), e.g, Delta3 therapeutics. Based at least in part on the results described in the previous Examples, Delta therapeutics can be used for treating various diseases, including neurological diseases, and/or hyper- or hypoproliferative diseases, and diseases or conditions associated with defects in vasculature. In addition, based at least in part on the similarity of amino acid sequence and structure between the various Delta proteins, Delta3 therapeutics can be used to treat diseases or conditions associated with an aberrant Delta3 activity or an aberrant Delta activity other than a Delta3 activity. Similarly, Delta3 therapeutics as well as Delta therapeutics other than Delta3 therapeutics can be used to treat diseases or conditions associated with an aberrant Delta3 activity. The assay set forth below is applicable to Delta proteins other than Delta3 proteins.

A Delta3 therapeutic can be identified by using an *in vitro* assay, in which the interaction between a Delta3 protein and a Delta3 binding protein, e .g, a Notch protein, is determined in the presence and in the absence of a test compound. A soluble binding fragment of a Delta3 protein can be prepared by expression of the extracellular portion of human Delta3, e.g., about amino acids 1-529 of SEQ ID No. 2, in E. coli according to methods known in the art. Alternatively, the Delta3 protein fragment can be about amino acid 173 to about amino acid 517 of SEQ ID NO. 2. Similarly, a Delta3 binding fragment of a Delta3 binding protein (i.e., Delta3 binding partner) can be produced recom-binantly. A Delta3 binding protein can be a Notch protein and can be identified, e.g., by determining whether the protein is capable of binding to a Delta3 protein. A nucleic acid encoding a Notch protein can be obtained, e.g, by PCR amplifying a portion of a Notch gene encoding at least an EGF-like domain, using primers having a nucleotide sequence derived from the nucleotide sequence of a Notch gene present in GenBank or disclosed in PCT Application No. PCT/US92/03651 or PCT/US93/09338.

Test compounds can then be tested to determine whether they inhibit the interaction between the Delta3 and the Delta3 binding protein by using an ELISA type assay. Accordingly, one of the recombinantly produced Delta3 protein and the Delta3 binding protein, e.g., Notch protein, is attached to a solid phase surface and the other protein is labeled, e.g., such as by tagging the protein with an epitope, for which an antibody Is available (e.g., FLAG epitope, available from International Biotechnologies, Inc.). For example, the Delta3 protein can be linked to the wells of a microtiter (96 well) plate by overnight incubation of the protein at a concentration of 10 µg/ml in PBS. After blocking unoccupied sites on the plate with a BSA solution, various amounts of test compounds and the recombinantly produced Delta3 binding protein are added to the wells in a buffer suitable for a specific interaction between the proteins. After an incubation time of several hours, the wells are rinsed with buffer, and the amount of Delta3 binding protein attached to the wells is determined. The amount of bound protein can be determined by incubating the wells with an anti-tag, e.g, anti-myc, antibody, which can then be detected by enzyme immunoassay. The amount of bound protein is then determined by determining the optical density using an ELISA reader. A lower amount ofDelta3 binding protein in a well that contained a test compound relative to a well that did not contain a test compound is indicative that the test compound inhibits the interaction between Delta3 and a Delta3 binding protein.

A Delta3 therapeutic can also be identified by using a reporter assay in which the level of expression of a reporter construct under the control of a Delta3 promoter is measured in the presence or absence of a test compound. A Delta3 promoter can be isolated by screening a genomic library with a Delta3 cDNA which preferably contains the 5' end of the cDNA A portion of the Delta3 promoter, typically from about 50 to about 500 base pairs long is then cloned upstream of a reporter gene, e.g., a luciferase gene, in a plasmid. This reporter construct is then transfected into cells, e.g., neural cells or endothelial cells. Transfected cells are then be distributed Into wells of a multiwell plate and various concentrations of test compounds are added to the wells. After several hours incubation, the level of expression of the reporter construct is determined according to methods known in the art. A difference in the level of expression of the reporter construct in transfected cells incubated with the test compound relative to transfected cells incubated without the test compound will indicate that the test compound is capable of modulating the expression of the Delta3 gene and is thus a Delta3 therapeutic.

### Deposit of Microorganisms

A nucleic acid encoding a full length human Delta protein is contained in a plasmid which was deposited with the American Type Culture Collection (ATCC) on March 5,1997 and has been assigned ATCC accession number98348.

## Claims

1. An isolated nucleic acid molecule selected from the group consisting of:
a) a nucleic acid molecule whose nucleotide sequence is at least 95% identical to the nucleotide sequence of SEQ ID NO:1 or SEQ ID NO:3, the cDNA insert of the plasmid deposited with the ATCC as Accession Number 98348, or a complement thereof;
b) a nucleic acid molecule which encodes a polypeptide which has the amino acid sequence of SEQ ID NO:2 or the amino acid sequence encoded by the cDNA insert of the plasmid deposited with the ATCC as Accession Number 98348.

2. The isolated nucleic acid of claim 1 further comprising a label.

3. A vector comprising an isolated nucleic acid of claim 1.

4. A host cell comprising a vector of claim 3.

5. An isolated polypeptide whose amino acid sequence is at least 95% identical to the amino acid sequence of SEQ ID NO:2.

6. An antibody or antibody fragment that is specifically reactive with the polypeptide of claim 5.

7. The antibody or antibody fragment of claim 6, which is selected from the group consisting of:
(a) polyclonal antibodies;
(b) monoclonal antibodies;
(c) F(ab) fragments;
(d) F(ab')2 fragments.

8. The antibody of any of claims 6 or 7 which is conjugated to a detectable substance or to a cytotoxic drug.

9. The antibody of claim 8 wherein the detectable substance is selected from the group consisting of:
(a) enzymes;
(b) fluorescent materials;
(c) chemiluminescent materials;
(d) bioluminescent materials.

## Patentansprüche

1. Isoliertes Nukleinsäuremolekül, ausgewählt aus der Gruppe bestehend aus:
a) einem Nukleinsäuremolekül, dessen Nukleotid-sequenz zu wenigstens 95% mit der Nukleotidsequenz der SEQ ID NO:1 oder SEQ ID NO:3, der cDNA-Insertion des bei der ATCC als Accession Number 98348 hinterlegten Plasmids, oder einem Komplement davon identisch ist;
b) einem Nukleinsäuremolekül, das für ein Polypeptid codiert, welches die Aminosäuresequenz der SEQ ID NO:2 oder die von der cDNA-Insertion des bei der ATCC als Accession Number 98348 hinterlegten Plasmids codierte Aminosäuresequenz aufweist.

2. Isolierte Nukleinsäure nach Anspruch 1, die ferner eine Markierung umfaßt.

3. Vektor, umfassend eine isolierte Nukleinsäure nach Anspruch 1.

4. Wirtszelle, umfassend einen Vektor nach Anspruch 3.

5. Isoliertes Polypeptid, dessen Aminosäuresequenz zu wenigstens 95% mit der Aminosäuresequenz der SEQ ID NO:2 identisch ist

6. Antikörper oder Antikörperfragment, der bzw. das spezifisch mit dem Polypeptid nach Anspruch 5 reagiert.

7. Antikörper oder Antikörperfragment nach Anspruch 6, der bzw. das ausgewählt ist aus der Gruppe bestehend aus:
(a) polyklonalen Antikörpern;
(b) monoklonalen Antikörpern;
(c) F(ab)-Fragmenten;
(d) F(ab')2-Fragmenten.

8. Antikörper nach Anspruch 6 oder 7, der an eine nachweisbare Substanz oder an einen zytotoxischen Arzneistoff konjugiert ist.

9. Antikörper nach Anspruch 8, wobei die nachweisbare Substanz ausgewählt ist aus der Gruppe bestehend aus:
(a) Enzymen;
(b) fluoreszierenden Stoffen;
(c) chemilumineszierenden Stoffen;
(d) biolumineszierenden Stoffen.

## Revendications

1. Molécule d'acide nucléique isolé choisie dans le groupe constitué par :
a) une molécule d'acide nucléique, dont la séquence nucléotidique est identique à au moins 95 % à la séquence nucléotidique de la SEQ ID n°1 ou de la SEQ ID n°3, de l'insert d'ADNc du plasmide déposé à l'ATCC sous le numéro d'accession 98348, ou d'un complément desdites séquences ;
b) une molécule d'acide nucléique qui code un polypeptide ayant la séquence d'acides aminés de la SEQ ID n°2 ou la séquence d'acides aminés codée par l'insert d'ADNc du plasmide déposé à l'ATCC sous le numéro d'accession 98348.

2. Acide nucléique isolé selon la revendication 1, comprenant en outre un marqueur.

3. Vecteur comprenant un acide nucléique isolé selon la revendication 1.

4. Cellule hôte comprenant un vecteur selon la revendication 3.

5. Polypeptide isolé, dont la séquence d'acides aminés est identique à au moins 95 % à la séquence d'acides aminés de la SEQ ID n°2.

6. Anticorps ou fragment d'anticorps qui réagit spécifiquement avec le polypeptide selon la revendication 5.

7. Anticorps ou fragment d'anticorps selon la revendication 6, qui est choisi dans le groupe constitué par :
(a) les anticorps polyclonaux ;
(b) les anticorps monoclonaux ;
(c) les fragments F(ab) ;
(d) les fragments F(ab')2.

8. Anticorps selon l'une quelconque des revendications 6 ou 7, qui est conjugué à une substance détectable ou à un médicament cytotoxique.

9. Anticorps selon la revendication 8, dans lequel la substance détectable est choisie dans le groupe constitué par :
(a) les enzymes ;
(b) les substances fluorescentes ;
(c) les substances chimioluminescentes ;
(d) les substances bioluminescentes.
